# EUROPEAN PATENT SPECIFICATION

(11) **EP 1 740 568 B1**
(45) Date of publication and mention of the grant of the patent: **26.11.2008**
(21) Application number: 05707417.1
(22) Date of filing: 10.02.2005
(51) Int. Cl.: C07D 333/54, C07D 333/56, C07D 417/12, C07D 307/79, C07D 307/80, A61K 31/343, A61K 31/381, A61K 31/427, A61P 3/06

(54) **BENZOFURAN AND BENZOTHIOPHENE DERIVATIVES USEFUL FOR THE TREATMENT OF CARDIOVASCULAR DISEASE.**
BENZOFURAN- UND BENZOTHIOPHENDERIVATE, DIE SICH FÜR DIE BEHANDLUNG VON HERZKREISLAUFERKRANKUNGEN EIGNEN
DERIVES DE BENZOFURAN ET BENZOTHIOPHENE QUI CONVIENNENT POUR LE TRAITEMENT DES MALADIES CARDIOVASCULAIRES

(30) Priority: 12.02.2004 GB 0403148
(43) Date of publication of application: 10.01.2007
(73) Proprietor: SMITHKLINE BEECHAM CORPORATION, Philadelphia, PA 19101 (US)
(72) Inventor: HARLING, John, David, GlaxoSmithKline, Harlow, Essex CM19 5AW (GB); LAMBETH, Paul, Francis, c/o GlaxoSmithKline, Stevenage, Hertfordshire SG1 2NY (GB); LIVERMORE, David, George, GlaxoSmithKline, Harlow, Essex CM19 5AW (GB)
(74) Representative: Learoyd, Stephanie Anne
(86) International application number: PCT/EP2005/001540
(87) International publication number: WO 2005/077926

(56) References cited:
- EP-A- 1 284 265
- WO-A-97/28149
- WO-A-20/04037775
- US-A- 5 859 051

## Description

The present invention relates to certain novel compounds. In particular, the present invention relates to compounds that activate human peroxisome proliferator activated receptors ("hPPARs"). The present invention also relates to methods for preparing the compounds; their use in medicine, pharmaceutical compositions containing them an their use in the manufacture of a medicament for the prevention or treatment of PPAR mediated diseases or conditions.

Several independent risk factors have been associated with cardiovascular disease. These include hypertension, increased fibrinogen levels, high levels of triglycerides, elevated LDL cholesterol, elevated total cholesterol, and low levels of HDL cholesterol. HMG CoA reductase inhibitors ("statins") are useful for treating conditions characterized by high LDL-c levels. It has been shown that lowering LDL-c is not sufficient for reducing the risk of cardiovascular disease in some patients, particularly those with normal LDL-c levels. This population pool is identified by the independent risk factor of low HDL-c. The increased risk of cardiovascular disease associated with low HDL-c levels has not yet been totally successfully addressed by drug therapy (Bisgaier, C. L.; Pape, M. E. Curr. Pharm. Des. 1998, 4, 53-70).

Syndrome X (including metabolic syndrome) is loosely defined as a collection of abnormalities including hyperinsulemia, obesity, elevated levels of the following:- triglycerides, uric acid, fibrinogen, small dense LDL particles, and plasminogen activator inhibitor 1 (PAI-1), and decreased levels of HDL-c.

NIDDM is described as insulin resistance, which in turn causes anomalous glucose output and a decrease in glucose uptake, by skeletal muscle. These factors eventually lead to impaired glucose tolerance (IGT) and hyperinsulinemia.

Peroxisome Proliferator Activated Receptors (PPARs) are orphan receptors belonging to the steroid/retinoid receptor superfamily of ligand-activated transcription factors. See, for example Willson T.M. and Wahli, W., Curr. Opin. Chem. Biol., 1, 235-241 (1997) and Willson T.M. et. al., J. Med. Chem., 43, 527-549 (2000). The binding of agonist ligands to the receptor results in changes in the expression level of mRNA's encoded by PPAR target genes.

Three mammalian Peroxisome Proliferator-Activated Receptors have been isolated and termed PPAR-alpha, PPAR-gamma, and PPAR-delta (also known as NUC1 or PPAR-beta). These PPARs regulate expression of target genes by binding to DNA sequence elements, termed PPAR response elements (PPRE). To date, PPRE's have been identified in the enhancers of a number of genes encoding proteins that regulate lipid metabolism suggesting that PPARs play a pivotal role in the adipogenic signalling cascade and lipid homeostasis (H. Keller and W. Wahli, Trends Endocrinol. Metab 291-296, 4 (1993)).

It has been reported that the thiazolidinedione class of drugs are potent and selective activators of PPAR-gamma and bind directly to the PPAR-gamma receptor (J. M. Lehmann et. al., J. Biol. Chem. 12953-12956, 270 (1995)), providing evidence that PPAR-gamma is a possible target for the therapeutic actions of the thiazolidinediones.

Activators of the nuclear receptor PPARγ, for example rosiglitazone, have been shown in the clinic to enhance insulin-action, reduce serum glucose and have small but significant effects on reducing serum triglyceride levels in patients with Type 2 diabetes. See, for example, D. E. Kelly et al., Curr. Opin. Endocrino/. Diabetes, 90-96, 5 (2), (1998); M. D. Johnson et al., Ann. Pharmacother., 337 348, 32 (3), (1997); and M. Leutenegger et al., Curr. Ther. Res., 403-416, 58 (7), (1997).

The mechanism for this triglyceride lowering effect appears to be predominantly increased clearance of very low density lipoproteins (VLDL) through induction of lipoprotein lipase (LPL) gene expression. See, for example, B. Staels et al., Arterioscler. Thromb., Vasc. Biol., 1756-1764, 17 (9), (1997).

Fibrates are a class of drugs which may lower serum triglycerides 20-50%, lower LDLc 10-15%, shift the LDL particle size from the more atherogenic small dense to normal dense LDL, and increase HDLc 10-15%. Experimental evidence indicates that the effects of fibrates on serum lipids are mediated through activation of PPARα. See, for example, B. Staels et al., Curr. Pharm. Des., 1-14, 3 (1), (1997). Activation of PPARα results in transcription of enzymes that increase fatty acid catabolism and decrease de-novo fatty acid synthesis in the liver resulting in decreased triglyceride synthesis and VLDL production/secretion. In addition, PPARα activation decreases production of apoC-III. Reduction in apoC-III, an inhibitor of LPL activity, increases clearance of VLDL. See, for example, J. Auwerx et al., Atherosclerosis, (Shannon, Irel.), S29-S37, 124 (Suppl), (1996).

Certain compounds that activate or otherwise interact with one or more of the PPARs have been implicated in the regulation of triglyceride and cholesterol levels in animal models. See, for example, U.S. Patents 5,847,008 (Doebber et al.) and 5,859,051 (Adams et al.) and PCT publications-WO 97/28149 (Leibowitz et al.), WO99/04815 (Shimokawa et al.) and WO01/00603 (Glaxo Group Ltd). Oliver et al Proc Natl Acad Sci 98, 5306-5311 (2001) reports that raising of serum triglycerides in the obese rhesus monkey following administration of a PPAR delta agonist.

Accordingly the invention provides a compound of formula (I) and pharmaceutically acceptable salts and solvates and C₁₋₆ alkyl esters thereof. 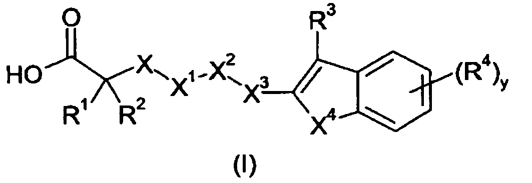 wherein:
R¹ and R independently represent H or C₁₋₃ alkyl;
X represents O, CH₂, or a bond (i.e. is absent);
X¹ represents: 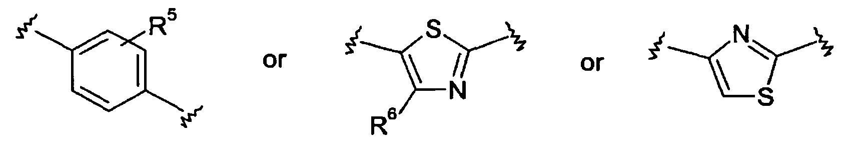
wherein R⁵ represents H, C₁₋₆alkyl. halogen, -OC₁₋₃ alkyl, CF₃;
R⁶ represents C₁₋₃ alkyl or H;
X² is O or S;
X³ is -(CH₂)ₙ - CH(R⁷)- or CH₂-CH = CH-
wherein n is 0,1 or 2; R⁷ represents H or C₁₋₆ alkyl, and the alkyl chain being optionally interrupted by one or more O atoms;
X⁴ is S or O;
R³ represents H or C₁₋₆ alkyl;
R⁴ represents H, CF₃, C₁₋₆ alkyl, halogen, or -OC₁₋₃ alkyl;
y is 0, 1, 2, 3, or 4.

hPPAR mediated diseases or conditions include dyslipidemia including associated diabetic dyslipidemia and mixed dyslipidemia, syndrome X (as defined in this application this embraces metabolic syndrome), heart failure, hypercholesterolemia, cardiovascular disease including atherosclerosis, arteriosclerosis, and hypertriglyceridemia, type II diabetes mellitus, type I diabetes, obesity, insulin resistance, hyperlipidemia, inflammation, epithelial hyperproliferative diseases including eczema and psoriasis and conditions associated with the lung and gut and regulation of appetite and food intake in subjects suffering from disorders such as obesity, anorexia bulimia, and anorexia nervosa, cancer, Alzheimers disease, multiple sclerosis or other cognitive disorders. In particular, the compounds of this invention are useful in the treatment and prevention of diabetes and cardiovascular diseases, obesity and conditions including atherosclerosis, arteriosclerosis, hypertriglyceridemia, and mixed dyslipidaemia.

In another aspect, the present invention provides pharmaceutical compositions comprising a compound of the invention, preferably in association with a pharmaceutically acceptable diluent or carrier.

In another aspect, the present invention provides a compound of the invention for use in therapy, and in particular, in human medicine.

In another aspect, the present invention provides the use of a compound of the invention for the manufacture of a medicament for the treatment of a hPPAR mediated disease or condition.

As used herein, "a compound of the invention" means a compound of formula (I) or a pharmaceutically acceptable salt, or solvate, or hydrolysable ester thereof.

While hydrolyzable esters are included in the scope of this invention, the acids are preferred because the data suggests that while the esters are useful compounds, it may actually be the acids to which they hydrolyse that are the active compounds. Esters that hydrolyse readily can produce the carboxylic acid in the assay conditions or in vivo. Generally the carboxylic acid is active in both the binding and transient transfection assays, while the ester does not usually bind well but is active in the transient transfection assay presumably due to hydrolysis. Preferred hydrolysable esters are C₁₋₆ alkyl esters wherein the alkyl group may be straight chain or branched chain. Methyl or ethyl esters are more preferred.

Preferably each R¹ and R² is independently H or methyl. More preferably R¹ and R² are both H or both methyl. Even more preferably, R¹ and R² are both H.

Preferably X² is O or S

Preferably X³ is -(CH₂)ₙ - CH(R⁷)- wherein R⁷ represents C₁₋₄ alkyl, (methyloxy)ethyl, propyloxy, or H. Preferably n is 1 or 2, more preferably 2.

Preferably R³ is CH₃ or H.

Preferably y is 1 or 0, more preferably 1.

Preferably R⁴ is CH₃, CF₃, OMe, or halogen.

A particularly preferred group of compounds are compounds of Formula (Ia): 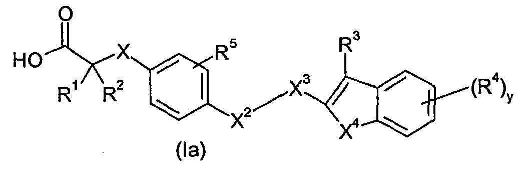 wherein X represents O, CH₂, or a bond (ie is absent), R¹, R², R³, R⁴, R⁵, X² and X³ and y are as defined for Formula (I).

Preferably in Formula (Ia), R⁵ is halogen, H₁, C₁₋₆ alkyl or CF₃. More preferably R⁵ is methyl, ethyl or CF₃.

In the compound of Formula (Ia) preferably each R¹ and R² is independently H or methyl. More preferably R¹ and R² are both H or both methyl. Even more preferably, R¹ and R² are both H.

Preferably in Formula (Ia), X² is O or S

Preferably in Formula (Ia), X³ is -(CH₂)ₙ - CH(R⁷)- wherein R⁷ represents C₁₋₄ alkyl, (methyloxy)ethyl, propyloxy, or H. Preferably n is 1 or 2, more preferably 2.

Preferably in Formula (Ia), R³ is CH₃ or H.

Preferably in Formula (Ia), y is 1 or 0, more preferably 1.

Preferably in Formula (Ia), R⁴ is CH₃, CF₃, OMe, or halogen.

A second preferred group of compounds are of Formula (Ib): 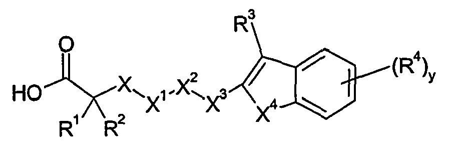 wherein X¹ represents: 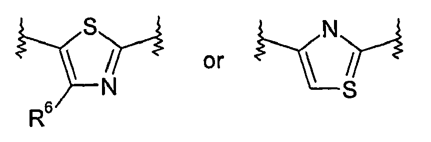 wherein X is a bond (ie) is absent), X², X³, X⁴, R¹, R², R³, R⁴ and R⁶ are as defined for Formula (I).

Preferably, R⁶ is H or methyl;

Preferably in Formula (Ib) each R¹ and R² is independently H or methyl. More preferably R¹ and R² are both H or both methyl. Even more preferably, R¹ and R² are both H.

Preferably in Formula (Ib) X² is O or S

Preferably in Formula (Ib) X³ is -(CH₂)ₙ - CH(R⁷)- wherein R⁷ represents C₁₋₄ alkyl, (methyloxy)ethyl, propyloxy, or H. Preferably n is 1 or 2, more preferably 2.

Preferably in Formula (Ib) R³ is CH₃ or H.

Preferably in Formula (Ib) y is 1 or 0, more preferably 1.

Preferably in Formula (Ib) R⁴ is CH₃, CF₃, OMe, or halogen.

Preferred compounds of the invention are:
[(4-{[3-(5-Bromo-3-methyl-1-benzothien-2-yl)propyl]thio}-2-methylphenyl)oxy]acetic acid
[(4-{[3-(6-Bromo-3-methyl-1-benzothien-2-yl)propyl]thio}-2-methylphenyl)oxy]acetic acid
{[2-Methyl-4-({3-[3-methyl-5-(trifluoromethyl)-1-benzothien-2-yl]propyl}thio)phenyl]oxy}acetic acid
{[2-Ethyl-4-({3-[3-methyl-5-(trifluoromethyl)-1-benzothien-2-yl]propyl}thio)phenyl]oxy}acetic acid
{[4-({3-[3-Ethyl-6-(trifluoromethyl)-1-benzothien-2-yl]propyl}thio)-2-methylphenyl]oxy}acetic acid
{[2-Ethyl-4-({3-[3-ethyl-6-(trifluoromethyl)-1-benzothien-2-yl]propyl}thio)phenyl]oxy}acetic acid
{[2-Methyl-4-({3-[6-(trifluoromethyl)-1-benzothien-2-yl]propyl}thio)phenyl]oxy}acetic acid
{[2-Ethyl-4-({3-[6-(trifluoromethyl)-1-benzothien-2-yl]propyl}thio)phenyl]oxy}acetic acid
{[2-(Trifluoromethyl)-4-({3-[6-(trifluoromethyl)-1-benzothien-2-yl]propyl}thio)phenyl]oxy}acetic acid
{[2-Ethyl-4-({3-[3-methyl-6-(trifluoromethyl)-1-benzothien-2-yl]propyl}thio)phenyl]oxy}acetic acid
2-Methyl-2-{[2-methyl-4-({3-[3-methyl-6-(trifluoromethyl)-1-benzothien-2-yl]propyl}thio)phenyl]oxy}propanoic acid
{[4-({3-[3-Methyl-6-(trifluoromethyl)-1-benzothien-2-yl]propyl}thio)-2-(trifluoromethyl)phenyl]oxy}acetic acid
{[2-Methyl-4-({(2E)-3-[3-methyl-5-(trifluoromethyl)-1-benzothien-2-yl]-2-propen-1-yl}thio)phenyl]oxy}acetic acid
2-[(4-{[3-(6-Bromo-3-methyl-1-benzothien-2-yl)propyl]oxy}-2-methylphenyl)oxy]-2-methylpropanoic acid
2-Methyl-2-{[2-methyl-4-({3-[3-methyl-5-(trifluoromethyl)-1-benzothien-2-yl]propyl}oxy)phenyl]oxy}propanoic acid
2-Methyl-2-{[2-methyl-4-({3-[3-methyl-6-(trifluoromethyl)-1-benzothien-2-yl]propyl}oxy)phenyl]oxy}propanoic acid
{[2-Methyl-4-({3-[6-(trifluoromethyl)-1-benzothien-2-yl]heptyl}thio)phenyl]oxy}acetic acid
{[2-(Trifluoromethyl)-4-({3-[6-(trifluoromethyl)-1-benzothien-2-yl]heptyl}thio)phenyl]oxy}acetic acid
{[2-Methyl-4-({3-[5-(trifluoromethyl)-1-benzothien-2-yl]heptyl}thio)phenyl]oxy}acetic acid
{[2-Methyl-4-({3-[5-(trifluoromethyl)-1-benzothien-2-yl]heptyl}oxy)phenyl]oxy}acetic acid
{[2-Ethyl-4-({3-[5-(trifluoromethyl)-1-benzothien-2-yl]heptyl}oxy)phenyl]oxy}acetic acid
{[2-Methyl-4-({3-[3-methyl-6-(trifluoromethyl)-1-benzothien-2-yl]hexyl}thio)phenyl]oxy}acetic acid
[4-Methyl-2-({3-[3-methyl-6-(trifluoromethyl)-1-benzothien-2-yl]hexyl}thio)-1,3-thiazol-5-yl]acetic acid
{[2-Methyl-4-({3-[3-methyl-6-(trifluoromethyl)-1-benzothien-2-yl]hexyl}oxy)phenyl]oxy}acetic acid
{[2-Ethyl-4-({3-[3-methyl-6-(trifluoromethyl)-1-benzothien-2-yl]hexyl}oxy)phenyl]oxy}acetic acid
{[2-Methyl-4-({3-[3-methyl-6-(trifluorom ethyl)-1-benzothien-2-yl]heptyl}thio)phenyl]oxy}acetic acid
[4-Methyl-2-({3-[3-methyl-6-(trifluoromethyl)-1-benzothien-2-yl]heptyl}thio)-1,3-thiazol-5-yl]acetic acid
{[2-Methyl-4-({3-[3-methyl-6-(trifluoromethyl)-1-benzothien-2-yl]heptyl}oxy)phenyl]oxy}acetic acid
{[2-Ethyl-4-({3-[3-methyl-6-(trifluoromethyl)-1-benzothien-2-yl]heptyl}oxy)phenyl]oxy}acetic acid
[4-({3-[3-Methyl-6-(trifluoromethyl)-1-benzothien-2-yl]heptyl}oxy)phenyl]acetic acid.
3-[4-({3-[3-Methyl-6-(trifluoromethyl)-1-benzothien-2-yl]heptyl}oxy)phenyl]propanoic acid
[3-Chloro-4-({3-[3-methyl-6-(trifluoromethyl)-1-benzothien-2-yl]heptyl}oxy)phenyl]acetic acid
[3-(Methyloxy)-4-({3-[3-methyl-6-(trifluoromethyl)-1-benzothien-2-yl]heptyl}oxy)phenyl]acetic acid
3-[3-(Methyloxy)-4-({3-[3-methyl-6-(trifluoromethyl)-1-benzothien-2-yl]heptyl}oxy)phenyl]propanoic acid
[2-({3-[3-Methyl-6-(trifluoromethyl)-1-benzothien-2-yl]heptyl}thio)-1,3-thiazol-4-yl]acetic acid
[(4-{[3-(6-Fluoro-1-benzothien-2-yl)heptyl]oxy}-2-methylphenyl)oxy]acetic acid
[(4-{[3-(6-Fluoro-3-methyl-1-benzothien-2-yl)heptyl]oxy}-2-methylphenyl)oxy]acetic acid
{[4-({3-[3-Ethyl-5-(trifluoromethyl)-1-benzothien-2-yl]heptyl}oxy)-2-methylphenyl]oxy}acetic acid
[(4-{[3-(5-Fluoro-3-methyl-1-benzothien-2-yl)heptyl]oxy}-2-methylphenyl)oxy]acetic acid
[(4-{[3-(6-Fluoro-3-methyl-1-benzothien-2-yl)heptyl]thio}-2-methylphenyl)oxy]acetic acid
{[2-Methyl-4-({3-[3-methyl-5-(trifluoromethyl)-1-benzothien-2-yl]heptyl}thio)phenyl]oxy}acetic acid
(2-{[3-(3,6-Dimethyl-1-benzofuran-2-yl)heptyl]thio}-4-methyl-1,3-thiazol-5-yl)acetic acid
[(4-{[3-(3,6-Dimethyl-1-benzofuran-2-yl)heptyl]oxy}-2-methylphenyl)oxy]acetic acid
[(4-{[3-(3,6-Dimethyl-1-benzofuran-2-yl)heptyl]thio}-2-methylphenyl)oxy]acetic acid
[4-Methyl-2-({3-[3-methyl-6-(trifluoromethyl)-1-benzofuran-2-yl]heptyl}thio)-1,3-thiazol-5-yl]acetic acid
{[2-Methyl-4-({3-[3-methyl-6-(trifluoromethyl)-1-benzofuran-2-yl]heptyl}oxy)phenyl]oxy}acetic acid
{[2-Methyl-4-({3-[3-methyl-6-(trifluoromethyl)-1-benzofuran-2-yl]heptyl}thio)phenyl]oxy}acetic acid
[4-Methyl-2-({5-(methyloxy)-3-[3-methyl-6-(trifluoromethyl)-1-benzothien-2-yl]pentyl}thio)-1,3-thiazol-5-yl]acetic acid
{[2-Methyl-4-({5-(methyloxy)-3-[3-methyl-6-(trifluoromethyl)-1-benzothien-2-yl]pentyl}thio)phenyl]oxy}acetic acid
{[2-Methyl-4-({5-(methyloxy)-3-[3-methyl-6-(trifluoromethyl)-1-benzothien-2-yl]pentyl}oxy)phenyl]oxy}acetic acid
{[2-Ethyl-4-({5-(methyloxy)-3-[3-methyl-6-(trifluoromethyl)-1-benzothien-2-yl]pentyl}oxy)phenyl]oxy}acetic acid
[(2-Methyl-4-{[3-[3-methyl-6-(trifluoromethyl)-1-benzothien-2-yl]-3-(propyloxy)propyl]thio}phenyl)oxy]acetic acid
[(2-Methyl-4-{[3-[3-methyl-6-(trifluoromethyl)-1-benzothien-2-yl]-3-(propyloxy)propyl]oxy}phenyl)oxy]acetic acid
[2-({3-[3-Methyl-6-(methyloxy)-1-benzothien-2-yl]heptyl}thio)-1,3-thiazol-4-yl]acetic acid
{[2-Methyl-4-({3-[3-methyl-6-(methyloxy)-1-benzothien-2-yl]heptyl}oxy)phenyl]oxy}acetic acid
[4-({3-[3-Methyl-6-(methyloxy)-1-benzothien-2-yl]heptyl}oxy)-3-(methyloxy)phenyl]acetic acid

While the preferred groups for each variable have generally been listed above separately for each variable, preferred compounds of this invention include those in which several or each variable in Formula (I) is selected from the preferred, more preferred, or most preferred groups for each variable. Therefore, this invention is intended to include all combinations of preferred and most preferred groups.

Those skilled in the art will recognize that stereocenters exist in compounds of formula (I). Accordingly, the present invention includes all possible stereoisomers and geometric isomers of formula (I) and includes not only racemic compounds but this invention is also intended to cover each of these isomers in their racemic, enriched, or purified forms. When a compound of formula (I) is desired as a single enantiomer, it may be obtained either by resolution of the final product or by stereospecific synthesis using an optically active catalyst or a catalytic system with optically active ligands or isomerically pure starting material or any convenient intermediate. Resolution of the final product, an intermediate or a starting material may be effected by any suitable method known in the art. See, for example, Stereochemistry of Carbon Compounds by E. L. Eliel (Mcgraw Hill, 1962) and Tables of Resolving Agents by S. H. Wilen. Additionally, in situations where tautomers of the compounds of formula (I) are possible, the present invention is intended to include all tautomeric forms of the compounds. In some of these chiral compounds the activities at the various PPAR receptors varies between the S and R isomers. Which of these isomers is preferred depends on the particular desired utility of the compound. In other words, even with the same compound, it is possible that the S isomer will be preferred for some uses, while the R isomer will be preferred for others.

The hPPAR agonists of formula (I) may be agonists of only one type ("selective agonists"), agonists for two PPAR subtypes ("dual agonists"), or agonists for all three subtypes ("pan agonists"). As used herein, by "agonist", or "activating compound", or "activator", or the like, is meant those compounds which have a pKᵢ of at least 6.0 preferably at least 7.0 to the relevant PPAR, for example hPPARδ, in the binding assay described below, and which achieve at least 50% activation of the relevant PPAR relative to the appropriate indicated positive control in the transfection assay described below at concentrations of 10⁻⁵ M or less. More preferably, the agonists of this invention achieve 50% activation of at least one human PPAR in the relevant transfection assay at concentrations of 10⁻⁶ M or less. Preferably, the compounds of formula (I) are hPPAR agonists. More preferably the compounds are hPPARδ agonists. More preferably they are selective PPARδ agonists.

It will also be appreciated by those skilled in the art that the compounds of the present invention may also be utilised in the form of a pharmaceutically acceptable salt or solvate thereof. The physiologically acceptable salts of the compounds of formula (I) include conventional salts formed from pharmaceutically acceptable inorganic or organic acids or bases as well as quaternary ammonium acid addition salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic, naphthalene-2-sulfonic, benzenesulfonic hydroxynaphthoic, hydroiodic, malic, steroic, tannic and the like. Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts. Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of formula (I) are within the scope of the invention. References hereinafter to a compound according to the invention include both compounds of formula (I) and their pharmaceutically acceptable salts and solvates.

The compounds of the invention and their pharmaceutically acceptable derivatives are conveniently administered in the form of pharmaceutical compositions. Such compositions may conveniently be presented for use in conventional manner in admixture with one or more physiologically acceptable carriers or excipients.

While it is possible that compounds of the present invention may be therapeutically administered as the raw chemical, it is preferable to present the active ingredient as a pharmaceutical composition. The carrier(s) must be "acceptable" in the sense of being compatible with the other ingredients of the composition and not deleterious to the recipient thereof.

Accordingly, the present invention further provides for a pharmaceutical composition comprising a compound of formula (I) or a pharmaceutically acceptable salt or solvate thereof together with one or more pharmaceutically acceptable carriers therefore and, optionally, other therapeutic and/or prophylactic ingredients.

The compositions include those suitable for oral, parenteral (including subcutaneous e.g. by injection or by depot tablet, intradermal, intrathecal, intramuscular e.g. by depot and intravenous), rectal and topical (including dermal, buccal and sublingual) administration although the most suitable route may depend upon for example the condition and disorder of the recipient. The compositions may conveniently be presented in unit dosage form and may be prepared by any of the methods well known in the art of pharmacy. All methods include the step of bringing into association the compounds ("active ingredient") with the carrier, which constitutes one or more accessory ingredients. In general the compositions are prepared by uniformly and intimately bringing into association the active ingredient with liquid carriers or finely divided solid carriers or both and then, if necessary, shaping the product into the desired composition.

Compositions suitable for oral administration may be presented as discrete units such as each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a other conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art.

Alternatively, the compounds of the present invention may be incorporated into oral liquid preparations such as aqueous or oily suspensions, solutions, emulsions, syrups or elixirs, for example. Moreover, compositions containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

Compositions for parenteral administration include aqueous and non-aqueous sterile injection solutions which may contain anti-oxidants, buffers, bacteriostats and solutes which render the composition isotonic with the blood of the intended recipient; and aqueous and non-aqueous sterile suspensions which may include suspending agents and thickening agents.

The compositions may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Compositions for rectal administration may be presented as a suppository with the usual

Compositions for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

The compounds may also be formulated as depot preparations. Such long acting compositions may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

In addition to the ingredients particularly mentioned above, the compositions may include other agents conventional in the art having regard to the type of composition in question, for example those suitable for oral administration may include flavouring agents.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day. The compositions according to the invention may contain between 0.1-99% of the active ingredient, conveniently from 30-95% for tablets and capsules and 3-50% for liquid preparations.

The compound of formula (I) for use in the instant invention may be used in combination with other therapeutic agents for example, statins and/or other lipid lowering drugs for example MTP inhibitors and LDLR upregulators. The compounds of the invention may also be used in combination with antidiabetic agents, e.g. metformin, sulfonylureas and/or PPAR gamma, PPAR alpha or PPAR alpha/gamma agonists (for example thiazolidinediones such as e.g. Pioglitazone and Rosiglitazone). The compounds may also be used in combination with antihypertensive agents such as angiotensin antagonists e.g. telmisartan, calcium channel antagonists e.g. lacidipine and ACE inhibitors e.g. enalapril. The invention thus provides in a further aspect the use of a combination comprising a compound of formula (I) with a further therapeutic agent in the treatment of a hPPAR mediated disease.

When the compounds of formula (I) are used in combination with other therapeutic agents, the compounds may be administered either sequentially or simultaneously by any convenient route.

The combinations referred to above may conveniently be presented for use in the form of a pharmaceutical composition and thus pharmaceutical compositions comprising a combination comprise a further aspect of the invention. The individual components of such combinations may be administered either sequentially or simultaneously in separate or combined pharmaceutical compositions.

When combined in the same composition it will be appreciated that the two compounds must be stable and compatible with each other and the other components of the composition and may be formulated for administration. When formulated separately they may be provided in any convenient composition, conveniently in such a manner as are known for such compounds in the art.

When a compound of formula (I) is used in combination with a second therapeutic agent active against the same hPPAR mediated disease, the dose of each compound may differ from that when the compound is used alone. Appropriate doses will be readily appreciated by those skilled in the art.

Compounds of this invention may be conveniently prepared by a general process wherein a moiety like (A) is coupled to an alcohol (B) using the Mitsunobu protocol (O. Mitsunobu, 1981, Synthesis p1) or by alklylaton of (A) using a suitable non nucleophilic base such as K₂CO₃, Cs₂CO₃ or NaH, with an alkyl halide (C).

Note this synthesis is preferably carried out with the acid group protected by R to give intermediate (D). Preferably R is C₁₋₆alkyl which can be hydrolysed to give an acid of formula (I), or if readily hydrolyzable, the resulting ester can be administered. The groups R¹-R⁷ and X¹ of intermediate (D) can be further modified by standard chemistry.

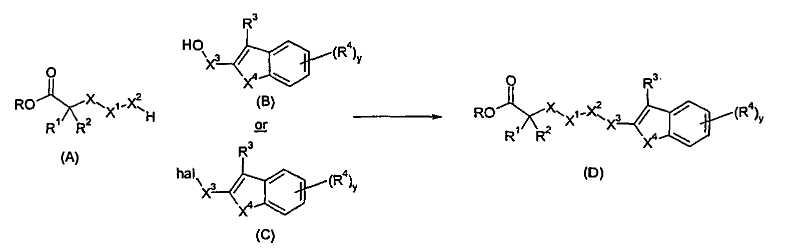

Intermediates of formulae (A), (B), (C) and (D) are commercially available or may be synthesised as outlined in the schemes below. Alcohol (B) can be converted to alkyl halide (C) using standard halogenation conditions.

For example, when X² is O or S, the following synthetic schemes may be followed.

### Scheme 1

Mitsunobu followed by hydrolysis

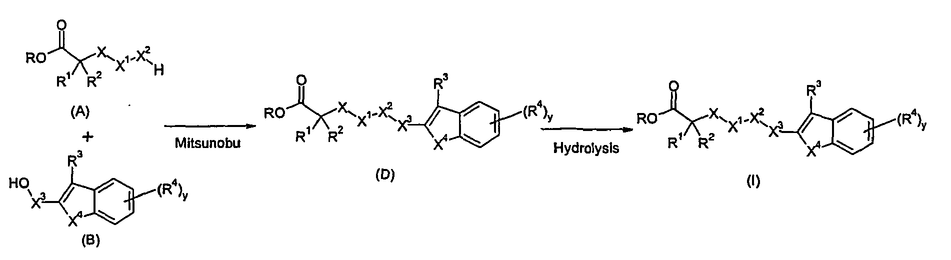

### Scheme 2

Alkylation followed by hydrolysis

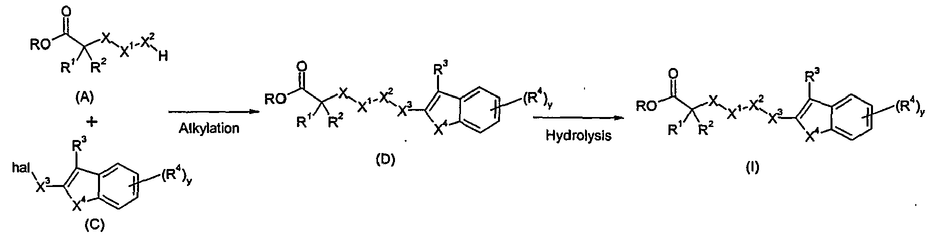

### Scheme 3

The following synthetic scheme may be followed to prepare intermediates (B) and (C)
where X³ is -(CH₂)ₙCH(R⁷)- and n = 2, R⁷ is C₁₋₆alkyl.

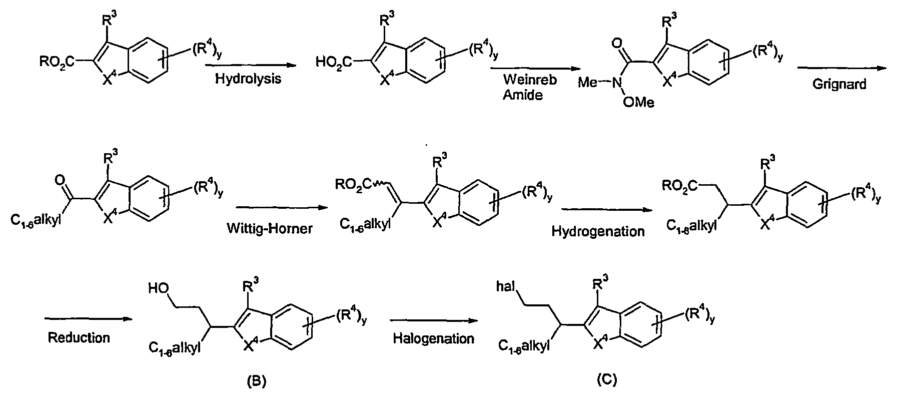

### Scheme 4

The following synthetic scheme may be followed to prepare intermediates (B) and (C)
where X³ is -(CH₂)ₙCH(R⁷)- and n = 2, R⁷ is -(CH₂)₂OMe.

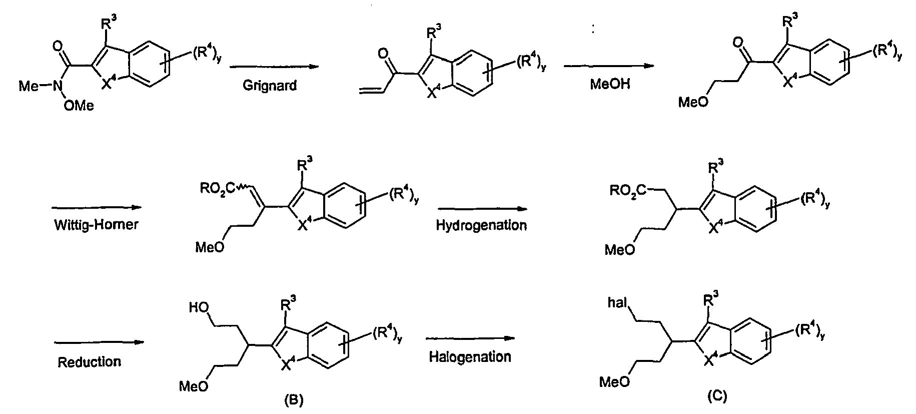

### Scheme 5

The following synthetic scheme may be followed to prepare intermediates (B) and (C)
where X³ is -(CH₂)ₙCH(R⁷)- and n = 2, R⁷ is -O(CH₂)₂CH₃.

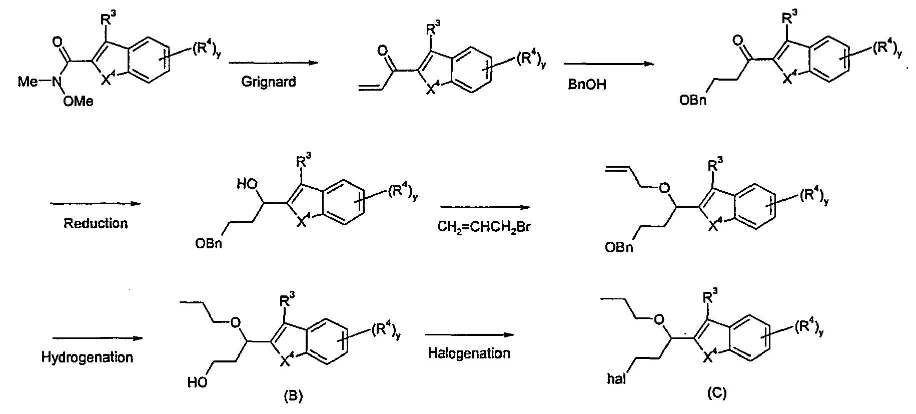

### Scheme 6

The following synthetic scheme may be followed to prepare intermediates (B) and (C)
where X³ is -(CH₂)ₙCH(R⁷)- and n = 2, R⁷ is H.

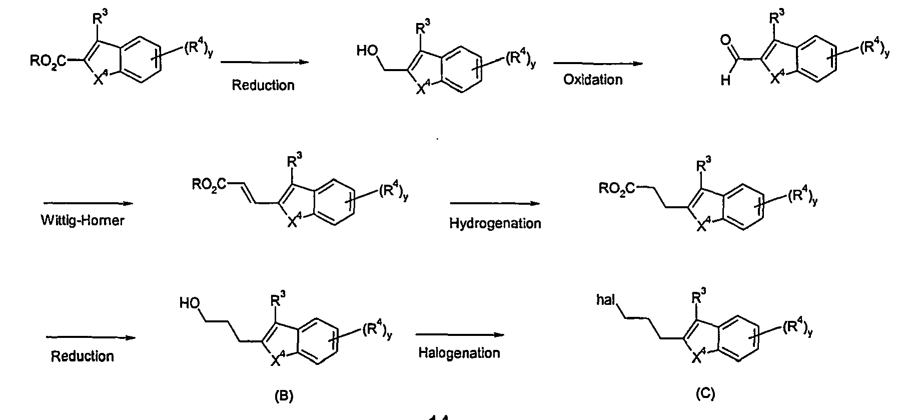

### Scheme 7

The following synthetic scheme may be followed to prepare intermediates (B) and (C)
where X³ is -CH₂CH=CH-.

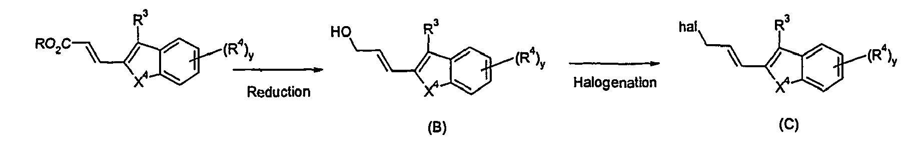

### Scheme 8

The following synthetic scheme may be followed to prepare intermediates used in Schemes 3 and 6 where X⁴ is S.

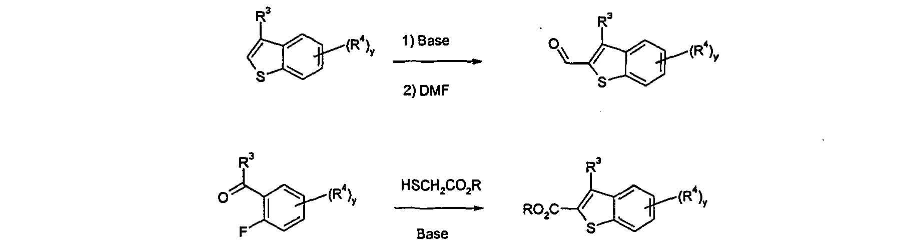

### Scheme 9

The following synthetic scheme may be followed to prepare intermediates used in Schemes 3 and 6 where X⁴ is O.

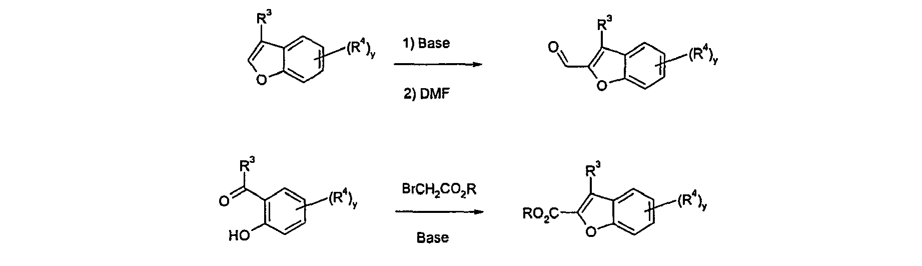

The following illustrates Intermediates and Examples of Formula 1 which should not be construed as constituting a limitation thereto.

### General purification and analytical methods

LC/MS refers to analysis by analytical HPLC which was conducted on a Supelcosil LCABZ+PLUS column (3µm, 3.3cm x 4.6mm ID) eluting with 0.1% HCO₂H and 0.01 M ammonium acetate in water (solvent A), and 95% acetonitrile and 0.05% HCO₂H in water (solvent B), using the following elution gradient 0-0.7 minutes 0%B, 0.7-4.2 minutes 0→100%B, 4.2-5.3 minutes 100%B, 5.3-5.5 minutes 100→%B at a flow rate of 3 ml/minute. The mass spectra (MS) were recorded on a Fisons VG Platform mass spectrometer using electrospray positive ionisation [(ES+ve to give [M+H]⁺ and [M+NH₄]⁺ molecular ions] or electrospray negative ionisation [(ES-ve to give [M-H]⁻ molecular ion] modes.

¹H NMR spectra were recorded using a Bruker DPX 400MHz spectrometer.

Biotage^{™} chromatography refers to purification carried out using equipment sold by Dyax Corporation (either the Flash 40i or Flash 150i) and cartridges pre-packed with KP-Sil^{™} silica.

Mass directed auto-prep HPLC refers to the method where the material was purified by high performance liquid chromatography on a HPLCABZ+ 5µm column (5cm x 10mm i.d.) with 0.1% HCO₂H in water and 95% MeCN, 5% water (0.5% HCO₂H) utilising the following gradient elution conditions: 0-1.0 minutes 5%B, 1.0-8.0 minutes 5→30%B, 8.0-8.9 minutes 30%B, 8.9-9.0 minutes 30→95%B, 9.0-9.9 minutes 95%B, 9.9-10 minutes 95→0%B at a flow rate of 8ml/minute. The Gilson 202-fraction collector was triggered by a VG Platform Mass Spectrometer on detecting the mass of interest.

Hydrophobic frits refers to filtration tubes sold by Whatman.

SPE (solid phase extraction) refers to the use of cartridges sold by International Sorbent Technology Ltd. SCX is a benzene sulfonic acid stationary phase.

TLC (thin layer chromatography) refers to the use of TLC plates sold by Merck coated with silica gel 60 F₂₅₄.

### Abbreviations :

- TLC :: thin layer chromatography
- DMSO-d₆: deuterated dimethylsulfoxide
- CDCl₃ :: deuterated chloroform
- MeOH-d₄ :: deuterated methanol
- DCM :: dichloromethane
- DMSO:: dimethylsulfoxide
- DMF :: N,N-dimethylformamide
- EtOAc :: ethyl acetate
- MeCN :: acetonitrile
- MeOH :: methanol
- Rₜ :: retention time
- THF :: tetrahydrofuran
- br :: broad
- s :: singlet
- d :: doublet
- dd :: doublet of doublets
- t :: triplet
- q :: quartet
- m :: multiplet
- rt :: room temperature

### Intermediate 1 : 5-Bromo-3-methyl-1-benzothiophene-2-carbaldehyde

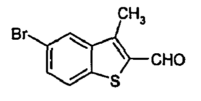

To a solution of 5-bromo-3-methylbenzothiophene (0.77 g, 3.4 mmol) in anhydrous THF (10mL) stirring under nitrogen at -70°C was added lithium bis(trimethylsilyl)amide (1.7 mL 2M solution, 3.4 mmol) and the reaction was stirred for 2 hours. DMF (1 mL) was added, the reaction was allowed to come to room temperature and stirred under nitrogen for 2 hours. The reaction was partitioned between DCM and water and the aqueous layer was extracted twice with DCM (20 mL). The combined extracts were washed with water (20 mL), dried and the solvent removed *in vacuo.* Purification by SPE (Silica, 20 g) eluting with cyclohexane: EtOAc (100:1) gave the title compound as a colourless oil (0.55 g). ¹H NMR (400MHz; CDCl₃) δ: 2.76 (3H, s), 7.6 (1 H, dd, J 8, 2 Hz), 7.74 (1 H, d, J 9 Hz), 8.04 (1 H, d, J 2 Hz), 10.7 (1 H, s).

### Intermediate 2 : 6-Bromo-3-methyl-1-benzothiophene-2-carbaldehyde

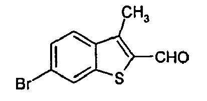

Prepared in a similar manner to Intermediate 1 from 6-bromo-3-methylbenzothiophene (0.886 g, 3.9 mmol). Purification by Biotage^{™} (Silica 90 g) eluting with cyclohexane: EtOAc (20:1) gave the title compound as a yellow solid (0.58 g). ¹H NMR (400MHz; CDCl₃) δ: 2.77 (3H, s), 7.55 (1 H, dd, J 8, 2Hz), 7.74 (1 H, d, J 9 Hz), 8.04 (1 H, d, J 2 Hz), 10.3 (1 H, s).

### Intermediate 3 : Methyl 3-methyl-5-(trifluoromethyl)-1-benzothiophene-2-carboxylate

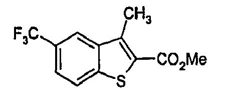

To a suspension of sodium hydride (0.31 g of a 60% suspension in oil, 7.8 mmol) in a mixture of anhydrous THF (2 mL) and anhydrous DMSO (8 mL) stirring under nitrogen was added methyl thioglycolate (0.53 g, 5 mmol) and the reaction was stirred until the evolution of gas had ceased. Stirred for a further 15 minutes before the addition of 2-fluoro-5-trifluoromethylacetophenone (1.03 g, 5 mmol) in anhydrous DMSO (2 mL) in one portion. This resulted in an exothermic reaction and considerable evolution of gas. The reaction was allowed to come to room temperature and stirred under nitrogen for 1 hour. The mixture was poured into water (100 mL) and extracted with EtOAc (3x 20 mL). The combined extracts were washed with water (20 mL), brine (20 mL), dried and the solvent removed *in vacuo*. Purification by SPE (Silica 20 g) eluting with cyclohexane: EtOAc (50:1) gave the title compound as a white solid (0.8 g).¹H NMR (400MHz; CDCl₃) δ: 2.82 (3H, s), 3.96 (3H, s) 7.68 (1H, dd, J 9, 2 Hz), 7.94 (1 H, d, J 8 Hz), 8.11 (1 H, s).

### Intermediate 4 : Methyl 3-ethyl-6-(trifluoromethyl)-1-benzothiophene-2-carboxylate

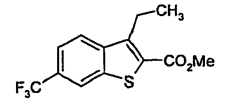

Prepared in a similar manner to Intermediate 3 from 2-fluoro-6-trifluoromethylpropriophenone (1.03 g, 4.68 mmol). Purification by SPE (Silica 20 g) eluting with cyclohexane: EtOAc (50:1) gave the title compound as a white solid (0.86 g).¹H NMR (400MHz; CDCl₃) δ: 1.29 (3H, t, J 7.5 Hz), 3.35 (2H, q, J 7 Hz), 3.96 (3H, s), 7.64 (1 H, dd, J 9, 2 Hz), 7.97 (1 H, d, J 9 Hz), 8.13 (1 H, s).

### Intermediate 5 : Methyl 6-(trifluoromethyl)-1-benzothiophene-2-carboxylate

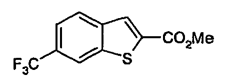

Prepared in a similar manner to Intermediate 3 from 2-fluoro-6-trifluoromethylbenzaldehyde (4.8 g, 25 mmol). Purification by Biotage^{™} (Silica 90 g) eluting with cyclohexane: EtOAc (20:1) gave the title compound as a yellow solid (3.47 g).¹H NMR (400MHz; CDCl₃) δ: 3.98 (3H, s), 7.63 (1H, dd, J 9, 2 Hz), 7.98 (1H, d J 9 Hz), 8.11 (1H, s), 8.17 (1 H, m).

### Intermediate 6 : 6-(Trifluoromethyl)-1-benzothiophene-2-carboxylic acid

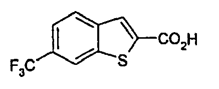

A solution of the methyl 6-(trifluoromethyl)-1-benzothiophene-2-carboxylate (2 g, 7.7 mmol) in MeOH (150 mL) was stirred at reflux with sodium hydroxide (17 mL 2M solution, 34 mmol) for 2 hours. The solvent was removed *in vacuo,* the residues were dissolved in water (100 mL) and the pH of the solution adjusted to 1 with 2M HCl. Filtration and drying gave the title compound as a yellow solid (1.77 g).¹H NMR (400MHz; DMSO-d₆) δ: 7.77 (1 H, dd, J 8, 2 Hz), 8.23 (1 H, d, J 8 Hz), 8.25 (1 H, s), 8.6 (1 H, s), 13.8 (1 H, br).

### Intermediate 7 : N-Methyl-N-(methyloxy)-6-(trifluoromethyl)-1-benzothiophene-2-carboxamide

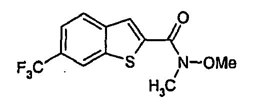

To a suspension of 6-(trifluoromethyl)-1-benzothiophene-2-carboxylic acid (1.76 g, 7.15 mmol) in DCM (150 mL) stirring under nitrogen was added a solution of CDI (1.5 g, 9.3 mmol) in DCM (10 mL). The mixture was stirred at room temperature for 1 hour resulting in a yellow solution. N,O-Dimethyl-hydroxylamine, liberated from the hydrochloride salt (1.4 g, 14.3 mmol),in DCM (30 mL) was added dropwise and the reaction was stirred for 20 hours. The resulting solution was washed with saturated sodium bicarbonate (20 mL) and brine (20 mL). Drying and removal of solvent *in vacuo* gave the title compound as a brown solid (1.97 g).¹H NMR (400MHz; CDCl₃) δ: 3.44 (3H, s), 3.84 (3H, s), 7.62 (1 H, dd, J 8, 2 Hz), 7.99 (1 H, d, J 8 Hz), 8.05 (1 H, s), 8.25 (1 H, s).

### Intermediate 8 : 1-[6-(Trifluoromethyl)-1-benzothien-2-yl]-1-pentanone

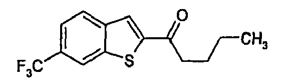

To a solution of *N*-methyl-*N*-(methyloxy)-6-(trifluoromethyl)-1-benzothiophene-2-carboxamide (1.97 g, 6.8 mmol), stirring under nitrogen at -70°C was added n-butylmagnesium chloride (4.76 mL of a 20% solution, 8.16 mmol). The reaction was allowed to come to room temperature and stirred for 24 hours. It was diluted with saturated ammonium chloride solution (100 mL) and extracted with DCM (3x 50 mL). The combined extracts were washed with water (20 mL), dried and the solvent removed *in vacuo* to give a yellow solid which was purified by SPE (Silica 20 g). Elution with cyclohexane:EtOAc (100:1) gave the title compound as a white solid (0.88 g).¹H NMR (400MHz; CDCl₃) δ: 0.97 (3H, t, J 7 Hz), 1.45 (2H, m), 1.78 (2H, m), 3.13 (2H, t, J 7.5 Hz), 7.63 (1 H, dd, J 8, 2 Hz), 7.99 (1 H, s), 8.0 (1 H, d, J 8 Hz), 8.17 (1 H, m).

### Intermediate 9 ; [3-Methyl-5-(trifluoromethyl)-1-benzothien-2-yl]methanol

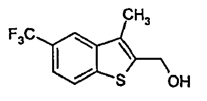

To a solution of the methyl 3-methyl-5-(trifluoromethyl)-1-benzothiophene-2-carboxylate (2.3 g, 8.4 mmol) in anhydrous THF (20 mL) stirring under nitrogen at 0°C was added diisobutylaluminium hydride (25.5 mL M solution, 25.5 mmol) dropwise. The reaction was stirred at 0°C for 2 hours and excess diisobutylaluminium hydride was destroyed by the cautious addition of water, before the mixture was partitioned between 0.5M HCl and EtOAc. The aqueous layer was extracted with EtOAc (2x50 mL), the combined extracts were washed with water (20 mL), brine (20 mL), dried and the solvent removed *in vacuo*. This gave the title compound as an oil which solidified on standing (2 g).¹H NMR (400MHz; CDCl₃) δ: 1.88 (1 H, br, exchanges with D₂O), 2.42 (3H, s), 4.95 (2H, m), 7.56 (1 H, dd, J 8, 2 Hz), 7.88-7.95 (2H, m).

### Intermediate 10 : [3-Ethyl-6-(trifluoromethyl)-1-benzothien-2-yl]methanol

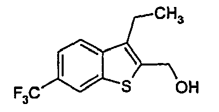

Prepared in a similar manner to Intermediate 9 from methyl 3-ethyl-6-(trifluoromethyl)-1-benzothiophene-2-carboxylate (2.91 g), used without any further purification. ¹H NMR (400MHz; CDCl₃) δ: 1.42 (3H, t, J 8 Hz), 1.95 (1 H, br, exchanges with D₂O), 2.9 (2H, q, J 7 Hz), 4.95 (2H, s), 7.6 (1 H, dd, J 9, 2 Hz), 7.79 (1 H, d, J 9 Hz) 8.1 (1 H, s).

### Intermediate 11 : [6-(Trifluoromethyl)-1-benzothien-2-yl]methanol

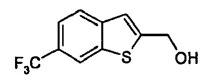

Prepared in a similar manner to Intermediate 9 from methyl 6-(trifluoromethyl)-1-benzothiophene-2-carboxylate (3.95 g) used without any further purification. ¹H NMR (400MHz; CDCl₃) δ: 2.13 (1 H, br, exchanges with D₂O) 4.97 (2H, s), 7.26 (1 H, s), 7.27 (1 H, dd, J 9, 2 Hz), 7.81 (1 H, d, J 8 Hz), 8.1 (1H, s).

### Intermediate 12 : 3-Methyl-5-(trifluoromethyl)-1-benzothiophene-2-carbaldehyde

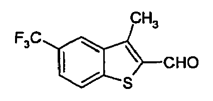

A solution of [3-methyl-5-(trifluoromethyl)-1-benzothien-2-yl]methanol (2 g, 8.13 mmol) in chloroform (50 mL) was stirred with manganese(IV)oxide (14 g, 165 mmol) for 6 hours. The reaction was filtered, the residues were washed with chloroform (3x50 mL) and the solvent was removed *in vacuo* to give a solid. This was purified by Biotage^{™} (Silica 90 g) eluting with cyclohexane: EtOAc (20:1) to give the title compound as a white solid (1.6 g).¹H NMR (400MHz; CDCl₃) δ: 2.85 (3H, s), 7.73 (1 H, dd, J 9, 2 Hz), 7.99 (1 H, d, J 9 Hz), 8.16 (1 H, s), 10.3 (1 H, s).

### Intermediate 13 : 3-Ethyl-6-(trifluoromethyl)-1-benzothiophene-2-carbaldehyde

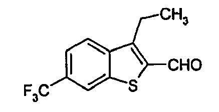

Prepared in a similar manner to Intermediate 12 from [3-ethyl-6-(trifluoromethyl)-1-benzothien-2-yl]methanol (2.1 g, 8 mmol). Purification by SPE (Silica, 50 g) eluting with cyclohexane: EtOAc (50:1) gave the title compound as a yellow solid (1.21 g). ¹H NMR (400MHz; CDCl₃) δ: 1.42 (3H, t, J 7.5 Hz), 3.32 (2H, q, J 8 Hz), 7.66 (1 H, dd, J 9, 2 Hz), 8.03 (1H, d, J 9 Hz), 8.18 (1 H, s), 10.35 (1 H, s).

### Intermediate 14 : 6-(Trifluoromethyl)-1-benzothiophene-2-carbaldehyde

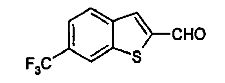

Prepared in a similar manner to Intermediate 12 from [6-(trifluoromethyl)-1-benzothien-2-yl]methanol. Purification by Biotage^{™} (Silica, 90 g) eluting with cyclohexane: EtOAc (30:1) gave the title compound as a pink solid (1.98 g).¹H NMR (400MHz; CDCl₃) δ: 7.67 (1 H, dd, J 9, 2 Hz), 8.07 (1H, d, J 9 Hz), 8.09 (1H, s), 8.21 (1 H, m) 10.35 (1 H, s).

### Intermediate 15 : 1,1-Dimethylethyl (2E)-3-(5-bromo-3-methyl-1-benzothien-2-yl)-2-propenoate

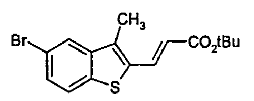

To a solution of t-butyldiethylphosphonoacetate (0.34 g, 1.35 mmol) in anhydrous THF (10 mL) stirring under nitrogen at 0°C was added sodium hydride (0.06 g of a 60% suspension in oil, 1.35 mmol) and the reaction was stirred until the evolution of hydrogen had ceased. A solution of 5-bromo-3-methyl-1-benzothiophene-2-carbaldehyde (0.3 g, 1.17 mmol) in anhydrous THF (20 mL) was added dropwise and the mixture was stirred at 0°C for 2 hours. The reaction was poured into water (200 mL) and extracted with EtOAc (3x 50 mL). The combined extracts were washed with water (50 mL), brine (50 mL) and dried. Removal of solvent *in vacuo* gave an oil which was purified by SPE (Silica 20 g). Elution with cyclohexane:EtOAc (50:1) gave the title compound as a yellow solid (0.34 g).¹H NMR (400MHz; CDCl₃) δ: 1.55 (9H, s), 2.46 (3H,s), 6,32 (1 H, d, J 16 Hz), 7.46 (1 H, dd, J 9, 2 Hz), 7.62 (1 H, d, J 8 Hz), 7.83 (1 H, d, J 2 Hz), 7.92 (1 H, d, J 16 Hz).

### Intermediate 16 : Ethyl (2E)-3-(6-bromo-3-methyl-1-benzothien-2-yl)-2-propenoate

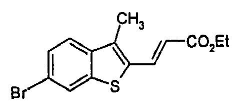

To a solution of t-triethylphosphonoacetate (0.56 g, 2.5 mmol) in anhydrous THF (10 mL) stirring under nitrogen at 0°C was added sodium hydride (0.1 g of a 60% suspension in oil, 2.5 mmol) and the reaction was stirred until the evolution of hydrogen had ceased. A solution of 6-bromo-3-methyl-1-benzothiophene-2-carbaldehyde (0.58 g, 2.27 mmol) in anhydrous THF (20 mL) was added dropwise and the mixture was stirred at 0°C for 2 hours. The reaction was poured into water (200 mL) and extracted with EtOAc (3x 50 mL). The combined extracts were washed with water (50 mL), brine (50 mL) and dried. Removal of solvent *in vacuo* gave a solid which was purified by SPE (Silica 20 g). Elution with cyclohexane:EtOAc (20:1) gave the title compound as a yellow solid (0.68 g). ¹H NMR (400MHz; CDCl₃) δ: 1 .35 (3H, t, J 7 Hz), 2.49 (3H,s), 4.27 (2H, q, J 7 Hz), 6,27 (1 H, d, J 16 Hz), 7.47 (1 H, dd, J 8, 2 Hz), 7.55 (1 H, d, J 9 Hz), 7.92 (1 H, d, J 2 Hz), 7.99 (1 H, d, J 16 Hz).

### Intermediate 17 : Ethyl (2E)-3-[3-methyl-5-(trifluoromethyl)-1-benzothien-2-yl]-2-propenoate

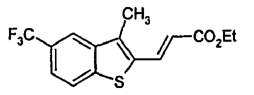

Prepared in a similar manner to Intermediate 16 from 3-methyl-5-trifluoromethyl-1-benzothiophene-2-carbaldehyde (0.95 g, 4 mmol). Purification by SPE (Silica 20 g) eluting with cyclohexane: EtOAc (20:1) gave the title compound as a yellow solid (1.98 g). ¹H NMR (400MHz; CDCl₃) δ: 1.35 (3H, t, J 7 Hz), 2.56 (3H,s), 4.29 (2H, q, J 7 Hz), 6,32 (1H, d, J 16 Hz), 7.6 (1 H, dd, J 8, 2 Hz), 7.87 (1H, d, J 8Hz), 7.95 (1H, m), 8.02 (1H, d, J 16 Hz).

### Intermediate 18 : Ethyl (2E)-3-[3-ethyl-6-(trifluoromethyl)-1-benzothien-2-yl]-2-propenoate

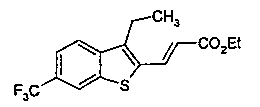

Prepared in a similar manner to Intermediate 16 from 3-ethyl-6-(trifluoromethyl)-1-benzothiophene-2-carbaldehyde (0.98 g, 4 mmol). Purification by SPE (Silica 20 g) eluting with cyclohexane: EtOAc (50:1) gave the title compound as a white solid (1.12 g).¹H NMR (400MHz; CDCl₃) δ: 1.27 (3H, t, J 8 Hz), 1.35 (3H, t, J 7 Hz), 3.04 (2H, q, J 7 Hz), 4.3 (2H, q, J 7 Hz), 6,36 (1 H, d, J 16 Hz), 7.59 (1 H, dd, J 9, 2 Hz), 7.83 (1 H, d, J 8 Hz), 8.01 (1 H, d, J 16 Hz), 8.07 (1 H, s).

### Intermediate 19 : Ethyl (2E)-3-16-(trifluoromethyl)-1-benzothien-2-yl]-2-propenoate

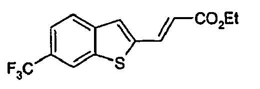

Prepared in a similar manner to Intermediate 16 from 6-(trifluoromethyl)-1-benzothiophene-2-carbaldehyde (0.5 g, 17 mmol). Purification by SPE (Silica 20 g) eluting with cyclohexane: EtOAc (50:1) gave the title compound as a white solid (0.63 g).¹H NMR (400MHz; CDCl₃) δ: 1.35 (3H, t, J 7 Hz), 4.29 (2H, q, J 7 Hz), 6,37 (1 H, d, J 16 Hz), 7.51 (1 H, s) 7.59 (1 H, dd, J 9, 2 Hz), 7.85 (1H, d, J 8 Hz), 7.87 (1 H, d, J 16 Hz), 8.08 (1 H, s).

### Intermediate 20 : Ethyl (2E)-3-[6-(trifluoromethyl)-1-benzothien-2-yl]-2-heptenoate

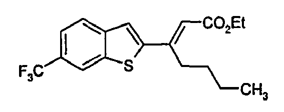

Prepared in a similar manner to Intermediate 16 from 1-[6-(trifluoromethyl)-1-benzothien 2-yl]-1-pentanone (1.08 g, 3.8 mmol). Purification by SPE (Silica 50 g) eluting with cyclohexane: EtOAc (100:1) gave the title compound as a yellow oil (1.023 g).¹H NMR (400MHz; CDCl₃) δ: 0.96 (3H, t, J 7 Hz), 1.34 (3H, t, J 7 Hz), 1.46 (2H, m), 1.6 (2H, m), 3.13 (2H, m), 4.23 (2H, q, J 8 Hz), 6 31 (1 H, s), 7.56 (1 H, s), 7.57 (1 H, d, J 9 Hz), 7.85 (1 H, d, J 9 Hz), 8.07 (1 H, s).

### Intermediate 21 : 1,1-Dimethylethyl 3-(5-bromo-3-methyl-1-benzothien-2-yl)propanoate

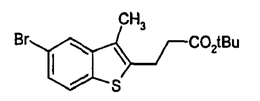

A mixture of 1,1-dimethylethyl (2*E*)-3-(5-bromo-3-methyl-1-benzothien-2-yl)-2-propenoate (0.32 g, 0.9 mmol) and tosylhydrazide on resin (1 g) was stirred and heated at 100°C under nitrogen in toluene (20 mL) for 24 hours. The resin was filtered off, washed with toluene (20 mL) and the solvent removed *in vacuo* to give an oil, which was purified by SPE (Silica 10 g). Elution with cyclohexane:EtOAc (50:1) gave the title compound as an oil which crystallised on standing (0.21 g). ¹H NMR (400MHz; CDCl₃) δ: 1.44 (9H, s), 2.30 (3H, s), 2.60 (2H, t, J 8 Hz), 3.15 (2H, t, J 8 Hz), 7.37 (1H, dd, J 9,2 Hz), 7.6 (1 H, d, J 8 Hz), 7.73 (1 H, d, 2 Hz).

### Intermediate 22 : Ethyl 3-(6-bromo-3-methyl-1-benzothien-2-yl)propanoate

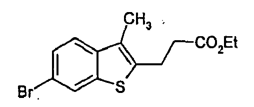

Prepared in a similar manner to Intermediate 21 from ethyl (2*E*)-3-(6-bromo-3-methyl-1-benzothien-2-yl)-2-propenoate (0.68 g, 2.1 mmol). Purification by SPE (Silica 20 g) eluting with cyclohexane: EtOAc (50:1) gave the title compound as an oil (0.57 g). ¹H NMR (400MHz; CDCl₃) δ: 1.25 (3H, t, J 7 Hz), 2.32 (3H, s), 2.67 (2H, t, J 4 Hz), 3.18 (2H, t, J 8 Hz), 4.15 (2H, q, J 7 Hz), 7.45 (2H, m), 7.88 (1 H, m).

### Intermediate 23 : Ethyl 3-[3-methyl-5-(trifluoromethyl)-1-benzothien-2-yl]propanoate

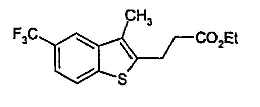

Ethyl (2*E*)-3-[3-methyl-5-(trifluoromethyl)-1-benzothien-2-yl]-2-propenoate (0.85 g, 2.7 mmol) in ethanol (30 mL) was hydrogenated over 10% palladium on carbon (0.16 g). The reaction was filtered through Celite and the residues washed with ethanol (30 mL). The solvent was removed *in vacuo* to give an oil which was purified by SPE (Silica 20 g). Elution with cyclohexane:EtOAc (20:1) gave the title compound as an oil (0.7 g). (400MHz; CDCl₃) δ: 1.25 (3H, t, J 7 Hz), 2.37 (3H, s), 2.7 (2H, t, 7.5 Hz), 3.23 (2H, t, J 7.5 Hz), 4.15 (2H, q, J 7 Hz), 7,5 (1 H, dd, J 8,2 Hz), 7.5 (1 H, d, J 9 Hz), 7.97 (1 H, m).

### Intermediate 24 : Ethyl 3-[3-ethyl-6-(trifluoromethyl)-1-benzothien-2-yl]propanoate

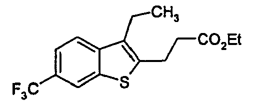

Prepared in a similar fashion to Intermediate 23 from ethyl (2E)-3-[3-ethyl-6-(trifluoromethyl)-1-benzothien-2-yl]-2-propenoate (1.12 g, 3.4 mmol). Purification by SPE (Silica 20 g) eluting with cyclohexane: EtOAc (50:1) gave the title compound as an oil (1.0 g).¹H NMR (400MHz; CDCl₃) δ: 1.24 (3H, t, J 8 Hz), 1.27 (3H, t, J 7Hz), 2.73 (2H, t, J 8 Hz), 2.85 (2H, q, J 7.5 Hz), 3.24 (2H, t, J 7.5 Hz), 4.16 (2H, q, J 6 Hz), 7.57 (1H, dd, J 8, 2 Hz), 7.73 (1H, d, J 8 Hz), 8.03 (1 H, m).

### Intermediate 25 : Ethyl 3-[6-(trifluoromethyl)-1-benzothien-2-yl]propanoate

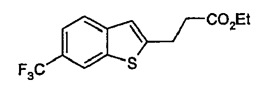

Prepared in a similar fashion to Intermediate 23 from ethyl (2E)-3-[(6-trifluoromethyl)-1-benzothen-2-yl]-2-propenoate (0.62 g, 2.07 mmol). Purification by SPE (Silica 20 g) eluting with cyclohexane: EtOAc (50:1) gave the title compound as an oil (1.0 g). ¹H NMR (400MHz; CDCl₃) δ: 1.27 (3H, t, J 7 Hz), 2.77 (2H, t, J 8Hz), 3.27 (2H, t, J 7 Hz), 4.16 (2H, q, J 6 Hz), 7.12 (1H, m), 7.53 (1 H, dd, J 8, 2 Hz), 7.74 (1 H, d, J 8 Hz), 8.03 (1 H, m).

### Intermediate 26 : Ethyl 3-[6-(trifluoromethyl)-1-benzothien-2-yl]heptanoate

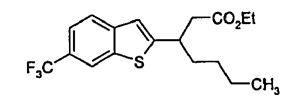

Prepared in a similar fashion to Intermediate 23 from ethyl (2*E*)-3-[6-(trifluoromethyl)-1-benzothien-2-yl]-2-heptenoate (1.11 g, 3.12 mmol). Purification by SPE (Silica 50 g) eluting with cyclohexane: EtOAc (100:1) gave the title compound as an oil (1.06 g). ¹H NMR (400MHz; CDCl₃) δ: 0.85 (3H, t, J 7 Hz), 1.17 (3H, t, J 7 Hz), 1.3-1.4 (4H, m), 1.73 (2H, m), 2.69 (2H, m), 3.54 (1 H, m), 4.08 (2H, dq, J 7, 2 Hz), 7.13 (1 H, s), 7.54 (1 H, dd, J 8, 2 Hz), 7.76 (1 H, d, J 8 Hz), 8.04 (1 H, m).

### Intermediate 27 : 3-(5-Bromo-3-methyl-1-benzothien-2-yl)-1-propanol

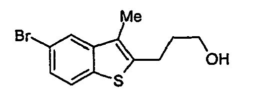

To a solution of the 1,1-dimethylethyl 3-(5-bromo-3-methyl-1-benzothien-2-yl)propanoate (0.21 g, 0.59 mmol) in anhydrous THF (10 mL) stirring under nitrogen at 0°C was added diisobutylaluminium hydride (1.8 mL 1M solution,1.8 mmol) dropwise. The reaction was stirred at 0°C for 2 hours and excess diisobutylaluminium hydride was destroyed by the cautious addition of water, before the mixture was partitioned between 0.5M HCl and EtOAc. The aqueous was extracted with EtOAc (2x30 mL) and the combined extracts were washed with water (20 mL), brine (20 mL), dried and the solvent removed *in vacuo*. This gave the title compound as an oil (0.16 g), which was used without further purification. ¹H NMR (400MHz; CDCl₃) δ: 1.96 (2H, m), 2.3 (3H, s), 2.97 (2H, t, J 7.5 Hz), 3.72 (2H, t, J 6Hz), 7.36 (1 H, dd, J 8, 2 Hz), 7.6 (1 H, d, J 8 Hz), 7.74 (1 H, d, J 2 Hz).

### Intermediate 28 : 3-(6-Bromo-3-methyl-1-benzothien-2-yl)-1-propanol

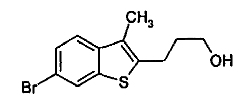

Prepared in a similar fashion to Intermediate 27 from ethyl 3-(6-bromo-3-methyl-1-benzothien-2-yl)propanoate (0.57 g, 1.74 mmol). Material (0.47 g) used without any further purification. ¹H NMR (400MHz; CDCl₃) δ: 1.95 (2H, m), 2.3 (3H, s) 2.96 (2H, t, J 7.5 Hz), 3.72 (2H, t, J 6 Hz), 7.45 (2H, m), 7.88 (1 H, m).

### Intermediate 29 : 3-[3-Methyl-5-(trifluoromethyl)-1-benzothien-2-yl]-1-propanol

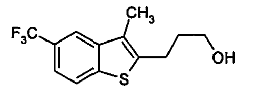

Prepared in a similar fashion to Intermediate 27 from ethyl 3-[3-methyl-5-(trifluoromethyl)-1-benzothien-2-yl]propanoate (0.7 g, 2.22 mmol). The product (0.63 g) was used without any further purification. ¹H NMR (400MHz; CDCl₃) δ: 1.98 (2H, m), 2.37 (3H, s) 3.0 (2H, t, J 7.5 Hz), 3.73 (2H, t, J 6 Hz), 7.49 (1 H, dd, J 8,2 Hz), 7.5 (2H, m).

### Intermediate 30 ; 3-[3-Ethyl-6-(trifluoromethyl)-1-benzothien-2-yl]-1-propanol

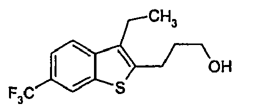

Prepared in a similar fashion to Intermediate 27 from ethyl 3-[3-ethyl-6-(trifluoromethyl)-1-benzothien-2-yl]propanoate (1.08 g, 3.3 mmol). The product (0.91 g) was used without any further purification. ¹H NMR (400MHz; CDCl₃) δ:1.23 (3H, t, J 7.5 Hz), 1.36 (1H, br), 2.0 (2H, m), 2.85 (2H, q, J 8 Hz), 3.02 (2H, t, J 7.5 Hz), 3.75 (2H, t, J 6 Hz), 7.57 (1 H, dd, J 8, 2 Hz) 7.73 (1 H, d, J 8 Hz), 8.04 (1H, m).

### Intermediate 31 : 3-[6-(Trifluoromethyl)-1-benzothien-2-yl]-1-propanol

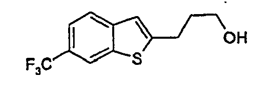

Prepared in a similar fashion to Intermediate 27 from ethyl 3-[6-(trifluoromethyl)-1-benzothien-2-yl)propanoate (0.56 g, 1.85 mmol). Product (0.48 g) was used without any further purification. ¹H NMR (400MHz; CDCl₃) δ: 1.57 (1H, br), 2.04 (2H, m), 3.07 (2H, t, J 7.5 Hz), 3.75 (2H, t, J 6 Hz), 7.1 (1 H, s) 7.54 (1H, dd, J 8, 2 Hz), 7.74 (1 H, d, J 8 Hz), 8.04 (1H, m).

### Intermediate 32 : (2E)-3-[3-Methyl-5-(trifluoromethyl)-1-benzothien-2-yl]-2-propen-1-ol

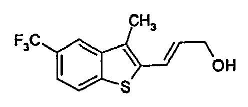

Prepared in a similar fashion to Intermediate 27 from ethyl (2*E*)-3-[3-ethyl-6-(trifluoromethyl)-1-benzothien-2-yl]-2-propenoate (0.2 g, 0.63 mmol). The product (0.185 g) used without any further purification. ¹H NMR (400MHz; CDCl₃) δ: 2.43 (3H, s), 4.39 (2H, d, J 5 Hz), 6.29 (1 H, dt, J 16, 5 Hz), 7.0 (1H, m), 7.52 (1H, d, J 8 Hz), 7.83 (1H, d, J 9 Hz), 7.87 (1 H, s).

### Intermediate 33 : 3-[6-(Trifluoromethyl)-1-benzothien-2-yl]-1-heptanol

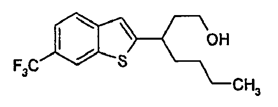

Prepared in a similar fashion to Intermediate 27 from ethyl 3-[6-(trifluoromethyl)-1-benzothien-2-yl]heptanoate (1.06 g, 3.0 mmol). The product (1.0 g) was used without any further purification. ¹H NMR (400MHz; CDCl₃) δ: 0.85 (3H, t, J 7 Hz), 1.2-1.4 (4H, m), 1.6 (1 H, br), 1.6-1.8 (2H, m), 1.82-1.92 (1 H, m), 2.0-2.1 (1H, m), 3.19 (1 H, m), 3.55 (1 H, m), 3.66 (1 H, m), 7.1 (1 H, s), 7.54 (1 H, d, J 8 Hz), 7.75 (1 H, d, 8Hz), 8.04 (1 H, m).

### Intermediate 34 : 5-Bromo-2-(3-bromopropyl)-3-methyl-1-benzothiophene

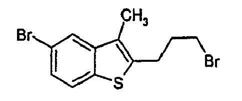

A mixture of the 3-(5-bromo-3-methyl-1-benzothien-2-yl)-1-propanol (0.16 g, 0.56 mmol), carbon tetrabromide (0.33 g, 0.8 mmol) and triphenylphosphine (0.21 g, 0.8 mmol) in DCM (5 mL) was stirred at room temperature for 4 hours. The solution was concentrated and applied to an SPE (Silica 20 g) column. Elution with cyclohexane:EtOAc (100:1) gave the title compound as an oil (0.2 g). ¹H NMR (400MHz; CDCl₃) δ: 2.25 (2H, m), 2.33 (3H, s), 3.05 (2H, t, J 7 Hz), 3.45 (2H, t, J 6 Hz), 7.38 (1 H, dd, J 8, 2 Hz), 7.62 (1 H, d, J 8 Hz), 7.75 (1 H, d, J 2 Hz).

### Intermediate 35 : 6-Bromo-2-(3-bromopropyl)-3-methyl-1-benzothiophene

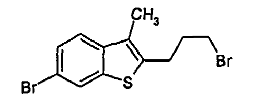

Prepared in a similar fashion to Intermediate 34 from 3-(6-bromo-3-methyl-1-benzothien-2-yl)-1-propanol (0.57 g, 1.74 mmol). Purification by SPE (Silica 20 g) eluting with cyclohexane: EtOAc (100:1) gave the title compound as an oil (0.62 g). ¹H NMR (400MHz; CDCl₃) δ: 2.23 (2H, m), 2.33 (3H, s), 3.04 (2H, t, J 7 Hz), 3.45 (2H, t, J 6 Hz), 7.45 (2H, m), 7.88 (1H, m).

### Intermediate 36 : 2-(3-Bromopropyl)-3-methyl-5-(trifluoromethyl)-1-benzothiophene

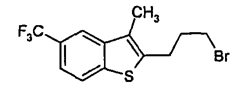

Prepared in a similar fashion to Intermediate 34 from 3-[3-methyl-5-(trifluoromethyl)-1-benzothien-2-yl]-1-propanol (0.62 g, 2.26 mmol). Purification by SPE (Silica 20 g) eluting with cyclohexane: EtOAc (100:1) gave the title compound as an oil (0.66 g). ¹H NMR (400MHz; CDCl₃) δ: 2.26 (2H, m), 2.4 (3H, s), 3.09 (2H, t, J 7 Hz), 3.46 (2H, t, J 6 Hz), 7.52 (1H, dd, J 8, 2 Hz), 7.86 (2H, m).

### Intermediate 37 : 2-(3-Bromopropyl)-3-ethyl-6-(trifluoromethyl)-1-benzothiophene

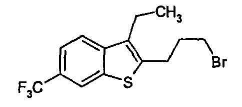

Prepared in a similar fashion to Intermediate 34 from 3-[3-ethyl-6-(trifluoromethyl)-1-benzothien-2-yl]-1-propanol (0.91 g, 3.15 mmol). Purification by SPE (Silica 50 g) eluting with cyclohexane: EtOAc (100:1) gave the title compound as an oil (1.05 g). ¹H NMR (400MHz; CDCl₃) δ: 1.24 (3H, t, J 8 Hz), 2.27 (2H, m), 2.87 (2H, q, J 7 Hz), 3.1 (2H, t, J 7.5 Hz),), 3.48 (2H, t, J 6 Hz), 7.57 (1 H, dd, J 8, 2 Hz), 7.74 (1 H, d, J 8 Hz), 8.05 (1 H, m).

### Intermediate 38 : 2-(3-Bromopropyl)-6-(trifluoromethyl)-1-benzothiophene

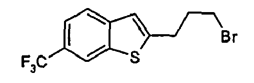

Prepared in a similar fashion to Intermediate 34 from 3-[6-(trifluoromethyl)-1-benzothien-2-yl)-1-propanol (0.47 g, 1.81 mmol). Purification by SPE (Silica 20 g) eluting with cyclohexane: EtOAc (50:1) gave the title compound as an oil (0.59 g).¹H NMR (400MHz; CDCl₃) δ: 2.3 (2H, m), 3.14 (2H, t J 7 Hz), 3.47 (2H, t, J 6 Hz), 7.13 (1 H, s), 7.55 (1 H, dd, J 8, 2 Hz), 7.77 (1 H, d, J 8 Hz), 8.05 (1 H, m).

### Intermediate 39 : 2-[(1E)-3-Bromo-1-propen-1-yl]-3-methyl-5-(trifluoromethyl)-1-benzothiophene

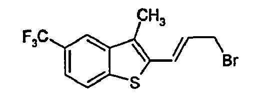

A solution of the (2*E*)-3-[3-methyl-5-(trifluoromethyl)-1-benzothien-2-yl]-2-propen-1-ol (0.09 g, 0.33 mmol) in DCM (20 mL) was stirred with carbon tetrabromide (0.13 g, 0.4 mmol) and triphenylphosphine on resin (0.8 g 1.2 mmol) for 24 hours. The resin was filtered off, washed with DCM (20 mL) and the solvent removed *in vacuo.* This gave the title compound (0.07 g) as a yellow oil which solidified. It was used without further purification. ¹H NMR (400MHz; CDCl₃) δ: 2.44 (3H, s), 4.19 (2H, d, J 8 Hz), 6.32 (1 H, dt, J 15, 8 Hz), 7.0 (1 H, d, J 15 Hz), 7.54 (1 H, m), 7.83 (1 H, d, J 8 Hz), 7.88 (1 H, m).

### Intermediate 40 : 2-[1-(2-Bromoethyl)pentyl]-6-(trifluoromethyl)-1-benzothiophene

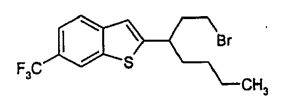

Prepared in a similar fashion to Intermediate 34 from 3-[6-(trifluoromethyl)-1-benzothien-2-yl]-1-heptanol (1.0 g, 3.15 mmol). Purification by SPE (Silica 50 g) eluting with cyclohexane: gave the title compound as an oil (1.3 g). ¹H NMR (400MHz; CDCl₃) δ: 0.85 (3H, t, J 7 Hz), 1.2-1.4 (4H, m), 1.6 (1 H, br), 1.6-1.8 (1 H, m), 1.82-1.92 (1 H, m), 2.0-2.1 (1 H, m), 3.19 (1 H, m), 3.26 (1 H, m), 3.41 (1H, m), 7.15 (1 H, s), 7.55 (1 H, dd, J 8, 2 Hz), 7.78 (1 H, d, J 8 Hz), 8.05 (1 H, m).

### Intermediate 41 : Ethyl [(4-{[3-(5-bromo-3-methyl-1-benzothien-2-yl)propyl]thio}-2-methylphenyl)oxy]acetate

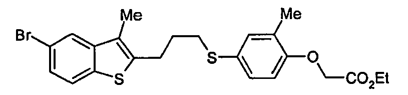

A mixture of the 5-bromo-2-(3-bromopropyl)-3-methyl-1-benzothiophene (0.2 g, 0.57 mmol), ethyl [(4-mercapto-2-methylphenyl)oxy]acetate (0.14 g, 0.6 mmol) and potassium carbonate (0.3 g, 2.2 mmol) was stirred at room temperature for 2 hours. The reaction was poured into water (80 mL) and extracted with EtOAc (3x 20 mL). The combined extracts were washed with water (20 mL), brine (20 mL) and dried. Removal of solvent *in vacuo* gave an oil which was purified by SPE (Silica 20 g). Elution with cyclohexane:EtOAc (100:1) gave the title compound as an oil (0.2 g). ¹H NMR (400MHz; CDCl₃) δ: 1.3 (3H, t, J 7Hz), 1.95 (2H, m), 2.25 (3H, s), 2.26 (3H, s), 2.86, (2H, t, J 7 Hz), 2.99 (2H, t, J 7.5 Hz), 4.26 (2H, q, J 7 Hz), 4.62 (2H, s), 6.63 (1H, d, J 8 Hz), 7.16 (1H, dd, J 8, 2 Hz), 7.2 (1H, m), 7.36 (1H, dd, J 8,2 Hz), 7.6 (1H, d,J8Hz), 7.72 (1H,d,J2Hz).

### Intermediate 42 : Ethyl [(4-{[3-(6-bromo-3-methyl-1-benzothien-2-yl)propyl]thio}-2-methylphenyl)oxy]acetate

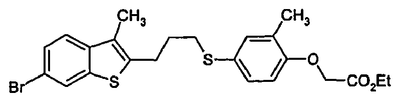

Prepared in a similar fashion to Intermediate 41 from 6-bromo-2-(3-bromopropyl)-3-methyl-1-benzothiophene (0.1 g, 0.28 mmol). Purification by SPE (Silica 20 g) eluting with cyclohexane: EtOAc (100:1) gave the title compound as an oil (0.1 g). ¹H NMR (400MHz; CDCl₃) δ: 1.28 (3H, t, J 7 Hz), 1.95 (2H, m), 2.25 (3H, s), 2.28 (3H, s), 2.86, (2H, t, J 6.5 Hz), 2.97 (2H, t, J 7 Hz), 4.26 (2H, q, J 7 Hz), 4.62 (2H, s), 6.62 (1H, d, J 8Hz), 7.16 (1 H, dd, J 8, 2 Hz), 7.2 (1 H, m), 7.44 (2H, m), 7.87 (1 H, m).

### Intermediate 43 : Ethyl {[2-methyl-4-({3-[3-methyl-5-(trifluoromethyl)-1-benzothien-2-yl]propyl}thio)phenyl]oxy}acetate

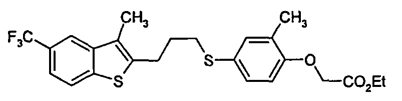

Prepared in a similar fashion to Intermediate 41 from 2-(3-bromopropyl)-3-methyl-5-(trifluoromethyl)-1-benzothiophene (0.1 g, 0.29 mmol). Purification by SPE (Silica 20 g) eluting with cyclohexane: EtOAc (50:1) gave the title compound as an oil (0.14 g). ¹H NMR (400MHz; CDCl₃) δ: 1.29 (3H, t, J 8 Hz), 1.97 (2H, m), 2.25 (3H, s), 2.35 (3H, s), 2.67, (2H, t, J 7 Hz), 3.03 (2H, t, J 7 Hz), 4.25 (2H, q, J 7 Hz), 4.62 (2H, s), 6.63 (1 H, d, J 8 Hz), 7.17 (1 H, dd, J 8, 2 Hz), 7.21 (1 H, m), 7.5 (1 H, dd, J, 8, 2 Hz), 7.83 (2H, m).

### Intermediate 44 : Ethyl {[2-ethyl-4-({3-[3-methyl-5-(trifluoromethyl)-1-benzothien-2-yl]propyl}thio)phenyl]oxy}acetate

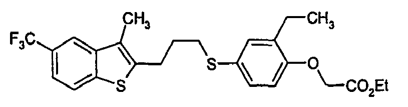

Prepared in a similar fashion to Intermediate 41 2-(3-bromopropyl)-3-methyl-5-(trifluoromethyl)-1-benzothiophene (0.1 g, 0.29 mmol). Purification by SPE (Silica 20 g) eluting with cyclohexane: EtOAc (50:1) gave the title compound as an oil (0.14 g). ¹H NMR (400MHz; CDCl₃) δ: 1.2 (3H, t, J 8 Hz) 1.28 (3H, t, J 8Hz), 1.97 (2H, m), 2.34 (3H, s), 2.67, (2H, q, J 7 Hz), 2.88 (2H, t, J 8 Hz), 3.03 (2H, t, J 8 Hz), 4.26 (2H, q, J 7 Hz), 4.62 (2H, s), 6.63 (1H, d, J 8 Hz), 7.17 (1 H, dd, J 8, 2Hz), 7.23 (1 H, m), 7.49 (1 H, dd, J 8, 2Hz), 7.83 (2H, m).

### Intermediate 45 : Ethyl {[4-({3-[3-ethyl-6-(trifluoromethyl)-1-benzothien-2-yl]propyl}thio)-2-methylphenyl]oxy}acetate

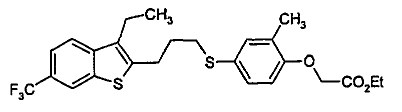

Prepared in a similar fashion to Intermediate to Intermediate 41 from 2-(3-bromopropyl)-3-ethyl-6-(trifluoromethyl)-1-benzothiophene (0.1 g, 0.28 mmol). Purification by SPE (Silica 20 g) eluting with cyclohexane: EtOAc (50:1) gave the title compound as an oil (0.13 g). ¹H NMR (400MHz; CDCl₃) δ: 1.2 (3H, t, J 7 Hz), 1.29 (3H, t, J 7.5 Hz), 1.98 (2H, m), 2.25 (3H, s), 2.82 (2H, q, J 8 Hz), 2.88 (2H, t, J 7 Hz), 3.03 (2H, t, J 8 Hz), 4.26 (2H, q, J 7 Hz), 4.63 (2H, s), 6.63 (1H, d, J 8 Hz), 7.18 (1 H, dd, J 8, 2 Hz), 7.22 (1 H, m), 7.55 (1 H, dd, J 8, 2Hz), 7.72 (1 H, d, J 8 Hz), 8.04 (1 H, s).

### Intermediate 46 : Ethyl {[2-ethyl-4-({3-[3-ethyl-6-(trifluoromethyl)-1-benzothien-2-yl]propyl}thio)phenyl]oxy}acetate

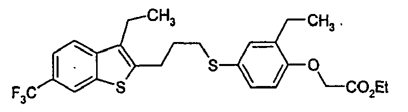

Prepared in a similar fashion to Intermediate 41 from 2-(3-bromopropyl)-3-ethyl-6-(trifluoromethyl)-1-benzothiophene (0.1 g, 0.28 mmol). Purification by SPE (Silica 20 g) eluting with cyclohexane: EtOAc (50:1) gave the title compound as an oil (0.13 g). ¹H NMR (400MHz; CDCl₃) δ: 1.19 (6H, m), 1.27 (3H, t, J 7.5 Hz), 1.98 (2H, m), 2.67 (2H, q, J 8 Hz), 2 .82 (2H, q, J 8 Hz), 2.89 (2H, t, J 7 Hz), 3.04 (2H, t, J 8 Hz), 4.25 (2H, q, J 7 Hz), 4.63 (2H, s), 6.64 (1 H, d, J 8 Hz), 7.18 (1 H, dd, J 8, 2 Hz), 7.23 (1 H, m), 7.55 (1 H, dd, J 8, 2 Hz), 7.71 (1 H, d, J 8 Hz), 8.04 (1 H, s).

### Intermediate 47 : Ethyl {[2-methyl-4-({3-[6-(trifluoromethyl)-1-benzothien-2-yl]propyl}thio)phenyl]oxy}acetate

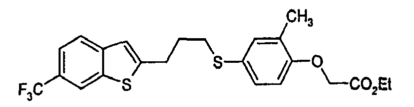

Prepared in a similar fashion to Intermediate 41 from 2-(3-bromopropyl)-6-(trifluoromethyl)-1-benzothiophene (0.144 g, 0.45 mmol). Purification by SPE (Silica 20 g) eluting with cyclohexane: EtOAc (50:1) gave the title compound as an oil (0.13 g). ¹H NMR (400MHz; CDCl₃) δ: 1.28 (3H, t, J 7 Hz), 2.03 (2H, m), 2.25 (3H, s), 2.87 (2H, t, J 7 Hz), 3.06 (2H, t, 7 Hz), 4.25 (2H, q, J 7 Hz), 4.62 (2H, s), 6.62 (1 H d, 8 Hz), 7.05 (1 H, s), 7.18 (1H, dd, J 8, 2 Hz), 7.21 (1 H, m), 7.53 (1 H, dd, J 8, 2 Hz), 7.73 (1 H, d, J 8 Hz), 8.02 (1 H, s).

### Intermediate 48 : Ethyl {[2-ethyl-4-({3-[6-(trifluoromethyl)-1-benzothien-2-yl]propyl}thio)phenyl]oxy}acetate

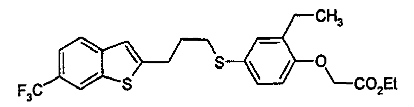

Prepared in a similar fashion to Intermediate 41 from 2-(3-bromopropyl)-6-(trifluoromethyl)-1-benzothiophene (0.144 g, 0.45 mmol). Purification by SPE (Silica 20 g) eluting with cyclohexane: EtOAc (19:1) gave the title compound as an oil (0.126 g). ¹H NMR (400MHz; CDCl₃) δ: 1.2 (3H, t, J 8 Hz), 1.28 (3H, t, J 7 Hz), 2.03 (2H, m), 2.67 (2H, q, J 7 Hz), 2.88 (2H, t, J 7 Hz), 3.07 (2H, t, J 8 Hz), 4.25 (2H, q, J 7 Hz), 4.63 (2H, s), 6.64 (1 H, d, J 8 Hz), 7.05 (1 H, s) 7.18 (1 H, dd, J 8, 2 Hz), 7.23 (1H, m), 7.53 (1 H, dd, J 8, 2 Hz), 7.73 (1 H, d, J 8 Hz), 8.02 (1 H, m).

### Intermediate 49 : Ethyl {[2-(trifluoromethyl)-4-({3-[6-(trifluoromethyl)-1-benzothien-2-yl]propyl}thio)phenyl]oxy}acetate

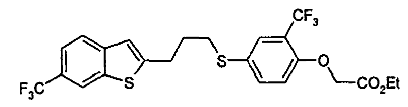

Prepared in a similar fashion to Intermediate 41 from 2-(3-bromopropyl)-6-(trifluoromethyl)-1-benzothiophene (0.145 g, 0.45 mmol). Purification by SPE (Silica 20 g) eluting with cyclohexane: EtOAc (50:1) gave the title compound as an oil (0.14 g). ¹H NMR (400MHz; CDCl₃) δ: 1.27 (3H, t, J 7 Hz), 2.03 (2H, m), 2.92 (2H, t, J 7 Hz), 3.08 (2H, t, 7 Hz), 4.26 (2H, q, J 7 Hz), 4.7 (2H, s), 6.81 (1 H d, 8 Hz), 7.07 (1 H, s), 7.48 (1 H, dd, J 8, 2 Hz), 7.54 (1 H, dd, J 8, 2 Hz), 7.63 (1 H, d J 2 Hz) 7.74 (1 H, d, J 8 Hz), 8.04 (1 H, m).

### Intermediate 50 : Ethyl {[2-ethyl-4-({3-[3-methyl-6-(trifluoromethyl)-1-benzothien-2-yl]propyl}thio)phenyl]oxy}acetate

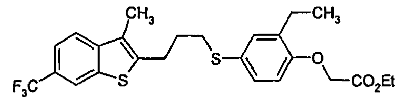

Prepared in a similar fashion to Intermediate 41 from 2-(3-bromopropyl)-3-methyl-6-(trifluoromethyl)-1-benzothiophene (0.2 g, 0.59 mmol). Purification by SPE (Silica 20 g) eluting with cyclohexane: EtOAc (19:1) gave the title compound as an oil (0.21 g).¹H NMR (400MHz; CDCl₃) δ: 1.2 (3H, t, J 8 Hz), 1.28 (3H, t, J 7Hz), 1.97 (2H, m), 2.33 (3H, s), 2.67 (2H, q, J 7 Hz), 2.88 (2H, t, J 7 Hz), 3.04 (2H, t, J 8 Hz), 4.26 (2H, q, J 7 Hz), 4.63 (2H, s), 6.64 (1 H, d, J 8 Hz), 7.18 (1H, dd, J 8, 2 Hz), 7.23 (1 H, m), 7.56 (1 H, dd, J 8, 2 Hz), 7.68 (1 H, d, J 8 Hz), 8.02 (1H, m).

### Intermediate 51 : Ethyl 2-methyl-2-{[2-methyl-4-({3-[3-methyl-6-(trifluoromethyl)-1-benzothien-2-yl]propyl}thio)phenyl]oxy}propanoate

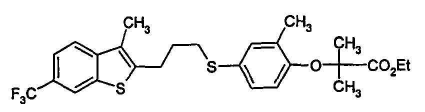

Prepared in a similar fashion to Intermediate 41 from 2-(3-bromopropyl)-3-methyl-6-(trifluoromethyl)-1-benzothiophene (0.2 g, 0.59 mmol). Purification by SPE (Silica 20 g) eluting with cyclohexane: EtOAc (50:1) gave the title compound as an oil (0.24 g). ¹H NMR (400MHz; CDCl₃) δ: 1.24 (3H, t, J 7 Hz), 1,57 (6H, s), 1.96 (2H, m), 2.18 (3H, s), 2.33 (3H, s), 2.87 (2H, t, J 7 Hz), 3.03 (2H, t, 7 Hz), 4.23 (2H, q, J 7 Hz), 6.57 (1 H d, 8 Hz), 7.08 (1 H, dd, J 8, 2 Hz), 7.18 (1H, m), 7.56 (1 H, dd, J 8, 2 Hz), 7.68 (1 H, d, J 8 Hz), 8.02 (1 H, s).

### Intermediate 52 : Ethyl {[4-({3-[3-methyl-6-(trifluoromethyl)-1-benzothien-2-yl]propyl}thio)-2-(trifluoromethyl)phenyl]oxy}acetate

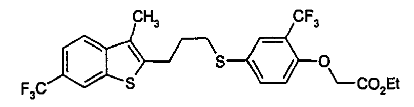

Prepared in a similar fashion to Intermediate 41 from 2-(3-bromopropyl)-3-methyl-6-(trifluoromethyl)-1-benzothiophene (0.15 g, 0.44 mmol). Purification by SPE (Silica 20 g) eluting with cyclohexane: EtOAc (50:1) gave the title compound as an oil (0.21 g). ¹H NMR (400MHz; CDCl₃) δ: 1.28 (3H, t, J 8 Hz), 1.98 (2H, m), 2.34 (3H, s), 2.90 (2H, t, J 7 Hz), 3.04 (2H, t, J 8 Hz), 4.26 (2H, q, J 7 Hz), 4.7 (2H, s), 6.8 (1 H, d, J 9 Hz), 7.48 (1 H, dd, J 8, 2 Hz), 7.57 (1 H, dd, J 8, 2 Hz), 7.63 (1 H, d, J 2 Hz), 7.68 (1 H, d, J 8 Hz), 8.03 (1 H, m).

### Intermediate 53 : Ethyl {[2-methyl-4-({(2E)-3-[3-methyl-5-(trifluoromethyl)-1-benzothien-2-yl]-2-propen-1-yl}thio)phenyl]oxy}acetate

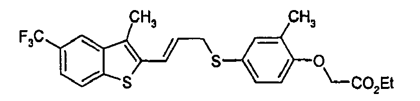

Prepared in a similar fashion to Intermediate 41 from 2-[(1*E*)-3-bromo-1-propen-1-yl]-3-methyl-5-(trifluoromethyl)-1-benzothiophene (0.065 g, 0.19 mmol). Purification by SPE (Silica 10 g) eluting with cyclohexane: EtOAc (50:1) gave the title compound as a yellow oil which solidified. (0.036 g). ¹H NMR (400MHz; CDCl₃) δ: 1.27 (3H, t, J 8 Hz), 2.24 (3H, s), 2.32 (3H, s) 3.62 (2H, d, J 7 Hz), 4.23 (2H, q, J 7 Hz), 4.6 (2H, s), 6.15 (1 H, dt, J 15, 7 Hz), 6.56 (1 H, d, 15 Hz), 6.61 (1H, d, J 9 Hz), 7.22 (1H, dd, J 8, 2 Hz), 7.27 (1 H, m), 5.51 (1H, m), 7.82 (2H, m).

### Intermediate 54 : Ethyl {[2-methyl-4-({3-[6-(trifluoromethyl)-1-benzothien-2-yl]heptyl}thio)phenyl]oxy}acetate

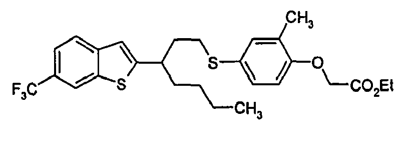

Prepared in a similar fashion to Intermediate 41 from 2-[1-(2-bromoethyl)pentyl]-6-(trifluoromethyl)-1-benzothiophene (0.14 g, 0.3 mmol. Purification by SPE (Silica 10 g) eluting with cyclohexane: EtOAc (100:1) gave the title compound as an oil (0.09 g). ¹H NMR (400MHz; CDCl₃) δ 0.84 (3H, t, J 7 Hz), 1.12-1.37 (4H, m), 1.29 (3H, t, J 7 Hz), 1.55-1.74 (2H, m), 1.87-2.03 (2H, m), 2.22 (3H, s), 2.66 (1 H, m), 2.8 (1H, m), 3.15 (1H, m), 4.26 (2H, q, J 7 Hz), 4.61 (2H, s), 6.6 (1H, d, J 8 Hz), 7.12 (1H, dd, J 8, 2 Hz), 7.15 (1H, m), 7.54 (1H, d, J 8 Hz), 7.75 (1 H, d, J 8 Hz); 8.05 (1 H, m).

### Intermediate 55 : Ethyl {[2-(trifluoromethyl)-4-({3-[6-(trifluoromethyl)-1-benzothien-2-yl]heptyl}thio)phenyl]oxy}acetate

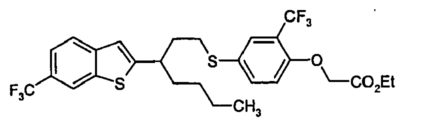

Prepared in a similar fashion to Intermediate 41 from 2-[1-(2-bromoethyl)pentyl]-6-(trifluoromethyl)-1-benzothiophene (0.14 g, 0.3 mmol). Purification by SPE (Silica 10 g) eluting with cyclohexane: EtOAc (100:1) gave the title compound as an oil (0.08 g). ¹H NMR (400MHz; CDCl₃) δ: 0.84 (3H, t, J 7 Hz), 1.14-1.36 (4H, m), 1.27 (3H, t, J 7 Hz), 1.58-1.75 (2H, m), 1.85-2.05 (2H, m), 2.69 (1 H, m), 2.83 (1 H, m), 3.15 (1 H, m), 4.26 (2H, q, J 7 Hz), 4.7 (2H, s), 6.78 (1 H, d, J 8 Hz), 7.07 (1 H, s), 7.42 (1 H, dd, J 8, 2 Hz), 7.54 (1 H, dd, J 8, 2 Hz), 7.57 (1H, d J 8 Hz), 7.76 (1 H, d, J 8 Hz), 8.05 (1 H, m).

### Intermediate 56 : Ethyl 2-[(4-{[3-(6-bromo-3-methyl-1-benzothien-2-yl)propyl]oxy}-2-methylphenyl)oxy]-2-methylpropanoate

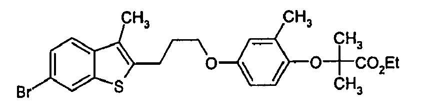

A mixture of the 6-bromo-2-(3-bromopropyl)-3-methyl-1-benzothiophene (0.1 g 0.28 mmol), ethyl 2-[(4-hydroxy-2-methylphenyl)oxy]-2-methylpropanoate (0.07 g, 0.28 mmol) and cesium carbonate (0.12 g, 0.37 mmol) in anhydrous MeCN (3 mL) was stirred at room temperature for 24 hours. The reaction was poured into water (50 mL) and extracted with EtOAc (3x 20 mL). The combined extracts were washed with water (20 mL), brine (20 mL) and dried. Removal of solvent *in vacuo* gave an oil which was purified by SPE (Silica 20 g). Elution with cyclohexane:EtOAc (100:1) gave the title compound as an oil (0.08 g). ¹H NMR (400MHz; CDCl₃) δ: 1.28 (3H, t, J 7 Hz), 1.53 (6H, s), 2.12 (2H, m), 2.2 (3H, s), 2.26 (3H, s), 3.04 (2H, t, J 7 Hz), 3.91 (2H, t, J 6 Hz), 4.25 (2H, q, J 7 Hz), 6.56 (1 H, dd, J 8, 2 Hz), 6.65 (2H, m) 7.44 (2H, m), 7.88 (1 H, m).

### Intermediate 57 : Ethyl 2-methyl-2-{[2-methyl-4-({3-[3-methyl-5-(trifluoromethyl)-1-benzothien-2-yl]propyl}oxy)phenyl]oxy}propanoate

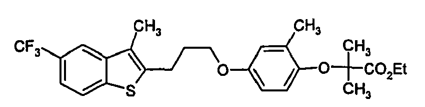

Prepared in a similar fashion to Intermediate 56 from 2-(3-bromopropyl)-3-methyl-5-(trifluoromethyl)-1-benzothiophene (0.1 g, 0.29mmol). Purification by SPE (Silica 20 g) eluting with cyclohexane: EtOAc (50:1) gave the title compound as an oil (0.11 g). ¹H NMR (400MHz; CDCl₃) δ: 1.28 (3H, t, J 8 Hz), 1.54 (3H, s), 1.57 (3H, s), 2.15 (2H, m), 2.21 (3H, s), 2.33 (3H, s), 3.1 (2H, t, J 7 Hz), 3.93 (2H, t, J 7 Hz), 4.25 (2H, q, J 7 Hz), 6.57 (1 H, dd, J 8, 2 Hz), 6.66 (1 H, d, J 8 Hz), 6.7(1 H, d, J 3 Hz), 7.5 (1 H, dd, J, 8, 2 Hz), 7.84 (2H, m).

### Intermediate 58 : Ethyl 2-methyl-2-{[2-methyl-4-({3-[3-methyl-6-(trifluoromethyl)-1-benzothien-2-yl]propyl}oxy)phenyl]oxy}propanoate

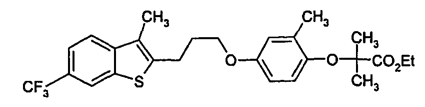

Prepared in a similar fashion to Intermediate 56 from 2-(3-bromopropyl)-3-methyl-6-(trifluoromethyl)-1-benzothiophene (0.2 g, 0.59 mmol). Purification by SPE (Silica 20 g) eluting with cyclohexane: EtOAc (20:1) gave the title compound as an oil (0.16 g). ¹H NMR (400MHz; CDCl₃) δ: 1.28 (3H, t, J 8 Hz), 1.54 (3H, s), 1.57 (3H, s), 2.15 (2H, m), 2.20 (3H, s), 2.32 (3H, s), 3.1 (2H, t, J 7 Hz), 3.92 (2H, t, J 7Hz), 4.25 (2H, q, J 7Hz), 6.57 (1 H, dd, J 8, 2 Hz), 6.67 (1H, d, J 8 Hz), 6.7(1 H, d, J 3 Hz), 7.57 (1 H, dd, J, 8, 2 Hz), 7.67 (1 H, d, J 8 Hz), 8.03 (1 H, m).

### Intermediate 59 : 3-Methyl-5-(trifluoromethyl)-1-benzothiophene-2-carboxylic acid

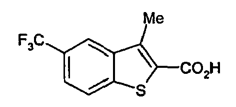

A solution of methyl 3-methyl-5-(trifluoromethyl)-1-benzothiophene-2-carboxylate (2.35 g, 8.6 mmol) in MeOH (150 mL) was treated with 2M aqueous sodium hydroxide (17 mL, 34 mmol) and stirred under reflux for 2 h. The mixture was cooled and evaporated to dryness. The residue was diluted with water (100 mL), acidified (2M hydrochloric acid) and filtered. The solid was washed with water and dried *in vacuo* at 60°C to afford the title compound (2.15 g). LC/MS: m/z 259 [M-H]⁻, Rₜ 3.91 min.

### Intermediate 60 : N,3-dimethyl-N-(methyloxy)-5-(trifluoromethyl)-1-benzothiophene-2-carboxamide

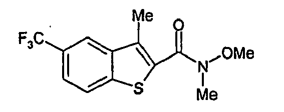

A suspension of 3-methyl-5-(trifluoromethyl)-1-benzothiophene-2-carboxylic acid (2.05 g, 7.8 mmol) in DCM (165 mL) was treated with a solution of carbonyldiimidazole (1.64 g, 10 mmol) in DCM (10 mL) and stirred at 20°C under nitrogen for 1.5 h. A solution of N,O-bismethylhydroxylamine, generated from the hydrochloride salt (1.5 g, 15.6 mmol) by partitioning between aqueous sodium carbonate solution and DCM (20 mL), was added portionwise and the mixture was stirred at 20°C for 4 days. The DCM solution was then washed with aqueous sodium bicarbonate solution (100 mL), water (100 mL), and brine (100 mL), dried over MgSO₄ and evaporated to yield an orange oil. This was eluted through a 50g SPE silica cartridge with EtOAc: cyclohexane: (1 : 4) to afford the title compound, which was isolated as a solid (1.32 g). LC/MS: m/z 304 [M+H]⁺, Rₜ 3.38 min.

### Intermediate 61 : 1-[3-Methyl-5-(trifluoromethyl)-1-benzothien-2-yl]-1-pentanone

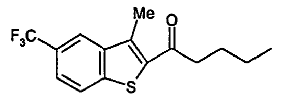

A solution of *N*,3-dimethyl-*N*-(methyloxy)-5-(trifluoromethyl)-1-benzothiophene-2-carboxamide (1.32 g, 4.3 mmol) in anhydrous THF (15 mL) was stirred under nitrogen at -70°C and treated with 20% butyl magnesium chloride in THF/toluene (3 mL, 5 mmol) The mixture was stirred at -70°C for 1 h, and then at 20°C overnight. More 20% butylmagnesium chloride solution (3 mL, 5 mmol) was then added and the mixture stirred for a further 1 h. The mixture was then diluted with aqueous ammonium chloride and extracted with DCM (2 x 50 mL). The combined organic extracts were washed with water, filtered through a phase-separating paper and evaporated to dryness to yield a gummy solid which was eluted through a 20g SPE silica cartridge with EtOAc : cyclohexane (1 : 5) to afford the title compound as a white solid. ¹H NMR (400 MHz; CDCl₃) δ: 0.97 (3H, t, J 7 Hz), 1.44 (2H, m), 1.77 (2H, m), 2.80 (3H, s), 2.95 (2H, t, J 7 Hz). 7.69 (1 H, d J 8 Hz), 7.95 (1 H, d, J 8 Hz), 8.13 (1 H, s).

### Intermediate 62 : Ethyl 3-[3-methyl-5-(trifluoromethyl)-1-benzothien-2-yl]-2-heptenoate

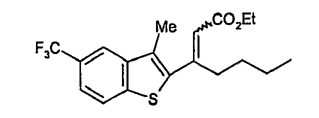

A solution of triethyl phosphonoacetate (0.69 g, 3 mmol) in anhydrous THF (10 mL) was stirred at 0°C under nitrogen and treated with sodium hydride (60% dispersion in oil, 0.12 g, 3 mmol). After 15 min a solution of 1-[3-methyl-5-(trifluoromethyl)-1-benzothien-2-yl]-1-pentanone (0.70 g, 2.3 mmol) in anhydrous THF (6 mL) was added, and the mixture was stirred at 20°C overnight. The mixture was diluted with water (200 mL) and extracted with EtOAc (3 x 100 mL). The organic extracts were combined, washed with brine (100 mL), dried over MgSO₄ and evaporated to dryness. The residue was eluted through a 50g SPE silica cartridge with EtOAc : cyclohexane (1 : 2) to afford a mixture of isomers of the title compound as a colourless oil (0.80g). LC/MS: m/z 371 [M+H]⁺, Rₜ 4.25 min and 4.38 min.

### Intermediate 63 : Ethyl 3-[3-methyl-5-(trifluoromethyl)-1-benzothien-2-yl]heptanoate

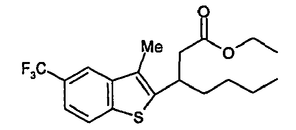

A solution of ethyl 3-[3-methyl-5-(trifluoromethyl)-1-benzothien-2-yl]-2-heptenoate (0.785 g, 2.1 mmol) in EtOAc (40 mL) was added to 10% palladium on carbon (wetted, 0.150 g), and the mixture was stirred under a hydrogen atmosphere at 20°C overnight. The mixture was filtered through a Celite pad, washing through with EtOAc. The filtrate and washings were evaporated to dryness to afford the title compound as a colourless oil (0.80g). ¹H NMR (400 MHz; CDCl₃) δ 0.84 (3H, t, J 7 Hz), 1.15 (3H, t, J 7 Hz), 1.15-1.40 (4H, m), 1.65 (1H, m), 1.76 (1 H, m), 2.40 (3H, s), 2.67 (2H, m). 3.70 (1 H, m), 4.05 (2H, q, J 7 Hz), 7.50 (1 H, d J 8 Hz), 7.86 (1 H, d, J 8 Hz), 7.87 (1 H, s).

### Intermediate 64 : 3-[3-Methyl-5-(trifluoromethyl)-1-benzothien-2-yl]-1-heptanol

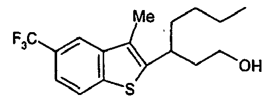

A solution of ethyl 3-[3-methyl-5-(trifluoromethyl)-1-benzothien-2-yl]heptanoate (0.750 g, 2 mmol) in anhydrous THF (10 mL) was stirred at 0°C under nitrogen and treated dropwise with 1.5M diisobutylaluminium hydride in toluene (4 mL). The mixture was stirred at 0°C for 1 h and at 20°C overnight. Sufficient water was carefully added to the mixture to destroy excess reagent, and then diluted with EtOAc (30 mL) and 1M hydrochloric acid (30 mL). The phases were separated and the aqueous layer extracted with EtOAc (2 x 20 mL). The combined organic extracts were washed with water (30 mL) and brine (30 mL), dried over MgSO₄ and evaporated to give an oil. This was eluted through a 50g SPE silica cartridge with EtOAc : cyclohexane (1 : 3) to afford the title compound as a colourless oil (0.31 g). ¹H NMR (400 MHz; CDCl₃) δ 0.84 (3H, t, J 7 Hz), 1.15-1.38 (5H, m), 1.65 (1 H, m), 1.75 (1 H, m), 1.84 (1 H, m), 2.04 (1 H, m), 2.38 (3H, s), 3.37 (1 H, m). 3.49 (1 H, m), 3.63 (1 H, m), 7.50 (1 H, d J 8 Hz), 7.86 (1 H, d, J 8 Hz), 7.87 (1 H, s). **Intermediate 65 : 2-[1-(2-Bromoethyl)pentyl]-3-methyl-5-(trifluoromethyl)-1-benzothiophene**

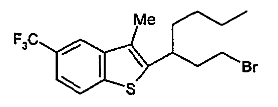

A solution of 3-[3-methyl-5-(trifluoromethyl)-1-benzothien-2-yl]-1-heptanol (304 mg, 0.92 mmol) in DCM (10 mL) was treated with triphenylphosphine (360 mg, 1.37 mmol) and carbon tetrabromide (0.454 g, 1.37 mmol). The mixture was stirred at 20°C under nitrogen for 5 h, and then evaporated to dryness. The residue was eluted through a 20 g SPE silica cartridge with cyclohexane to afford the title compound as a colourless oil (280 mg). ¹H NMR (400 MHz; CDCl₃) δ: 0.84 (3H, t, J 7 Hz), 1.14-1.38 (4H, m), 1.65 (1 H, m), 1.75 (1 H, m), 2.17 (1H, m), 2.2; (1H, m), 2.43 (3H, s), 3.16 (1 H, m). 3.37-3.50 (2H, m), 7.51 (1H, d J 8 Hz), 7.86 (1 H, d, J 8 Hz), 7.89 (1 H, s).

### Intermediate 66 : Ethyl {[2-methyl-4-({3-[3-methyl-5-(trifluoromethyl)-1-benzothien-2-yl]heptyl}thio)phenyl]oxy}acetate

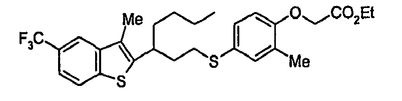

A solution of 2-[1-(2-bromoethyl)pentyl]-3-methyl-5-(trifluoromethyl)-1-benzothiophene (90 mg, 0.23 mmol) in anhydrous MeCN (4 mL) was treated with ethyl [(4-mercapto-2-methylphenyl)oxyJacetate (76 mg, 0.26 mmol) and potassium carbonate (150 mg). The mixture was stirred at 20°C under nitrogen for 4 h, and then diluted with water (40 mL), and extracted with DCM (2 x 25 mL). The organic extracts were combined, washed with water (25 mL) and brine (25 mL), dried over MgSO₄ and evaporated to give a yellow oil. This oil was eluted through a 10g SPE silica cartridge with EtOAc : cyclohexane (1 : 9) to afford the title compound as a gum (55 mg). LC/MS: m/z 539 [M+H]⁺, Rₜ 4.64 min.

### Intermediate 67 : Methyl 3-methyl-6-(trifluoromethyl)-1-benzothiophene-2-carboxylate

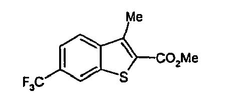

Methyl thioglycollate (2.65 g, 25 mmol) was added to a stirred suspension of sodium hydride (60% dispersion in oil, 1.56 g, 39 mmol) in anhydrous THF (10 mL) and anhydrous DMSO (40 mL). The mixture was stirred for 5 min to allow effervescence to subside, and then added a solution of 2'-fluoro-4'-trifluromethylacetophenone (5 g, 25 mmol) in anhydrous DMSO (10 mL). The mixture, which got hot, was stirred at ambient temperature for 2 h, and then diluted with water (500 mL) and extracted with EtOAc (3 x 200 mL). The combined organic extracts were washed with aqueous sodium bicarbonate (2 x 200 mL), water (200 mL) and brine (200 mL), dried over MgSO₄ and evaporated. The residue was triturated with a little cyclohexane and dried *in vacuo* to afford the title compound as a white solid (4.14 g). ¹H NMR (400 MHz; CDCl₃) δ: 2.81 (3H, s), 3.96 (3H, s), 7.65 (1 H, d, J 9 Hz), 7.94 (1 H, d, J 9 Hz), 8.12 (1 H, s).

### Intermediate 68 : 3-Methyl-6-(trifluoromethyl)-1-benzothiophene-2-carboxylic acid

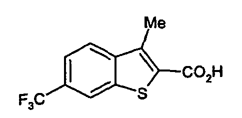

A solution of methyl 3-methyl-6-(trifluoromethyl)-1-benzothiophene-2-carboxylate (4.13 g, 15 mmol) in MeOH (250 mL) was treated with 2M aqueous sodium hydroxide (30 mL, 60 mmol) and stirred under reflux for 2 h. The mixture was cooled and evaporated to dryness. The residue was diluted with water (300 mL), acidified (2M hydrochloric acid) and filtered. The solid was washed with water and dried *in vacuo* at 60°C to afford the title compound (3.8 g). LC/MS: m/z 259 [M-H]⁻, Rₜ 3.88 min.

### Intermediate 69: N,3-Dimethyl-N-(methyloxy)-6-(trifluoromethyl)-1-benzothiophene-2-carboxamide

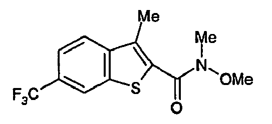

A suspension of 3-methyl-6-(trifluoromethyl)-1-benzothiophene-2-carboxylic acid (3.8 g, 14.6 mmol) in DCM (300 mL) was treated with carbonyldiimidazole (3.0 g, 18 mmol) and stirred at 20°C under nitrogen for 1.5 h. A solution of N,O-bismethylhydroxylamine, generated from the hydrochloride salt (3.0 g, 30 mmol) by partitioning between aqueous sodium carbonate solution (50 mL) and DCM (2 x 50 mL), was added portionwise and the mixture was stirred at 20°C for 3 days. The DCM solution was then washed with aqueous sodium bicarbonate solution (2 x 200 mL), and brine (200 mL), dried by filtration through a hydrophobic frit and evaporated. The residue was eluted through 2 x 50g SPE silica cartridges with EtOAc : cyclohexane (1 : 4) to afford the title compound, which was isolated as a white solid (3.34 g). LC/MS: m/z 304 [M+H]⁺, Rₜ 3.37 min.

### Intermediate 70 : 1-[3-Methyl-6-(trifluoromethyl)-1-benzothien-2-yl]-1-pentanone

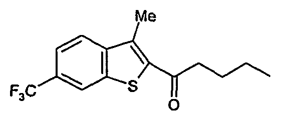

A solution of *N*,3-dimethyl-*N*-(methyloxy)-6-(trifluoromethyl)-1-benzothiophene-2-carboxamide (2.43 g, 8 mmol) in anhydrous THF (40 mL) was stirred under nitrogen at -70°C and treated with 20% butylmagnesium chloride in THF/toluene (10 mL, 16 mmol). The mixture was stirred at -70°C for 1 h, and then at 20°C for 5 days. The mixture was then diluted with aqueous ammonium chloride (300 mL) and extracted with DCM (2 x 150 mL). The combined organic extracts were washed with water (100 mL), filtered through a hydrophobic frit and evaporated to dryness to yield a gummy solid which was eluted through 2 x 50g SPE silica cartridges with EtOAc : cyclohexane (1 : 9) to afford the title compound as a white solid (1.67 g). LC/MS: m/z 301 [M+H]⁺, Rₜ 4.04 min.

### Intermediate 71 : 1-[3-Methyl-6-(trifluoromethyl)-1-benzothien-2-yl]-1-butanone

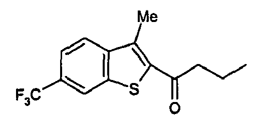

A solution of *N*,3-dimethyl-*N*-(methyloxy)-6-(trifluoromethyl)-1-benzothiophene-2-carboxamide (0.90 g, 3 mmol) in anhydrous THF (10 mL) was stirred under nitrogen at -70°C and treated with 3M propylmagnesium chloride in diethyl ether (2 mL, 6 mmol). The mixture was stirred at -70°C for 1 h, and then at 20°C overnight. More 3M propylmagnesium chloride in diethyl ether (2 mL, 6 mmol) was then added and the mixture was stirred at 20°C overnight. The mixture was then diluted with aqueous ammonium chloride (75 mL) and extracted with DCM (3 x 50 mL). The combined organic extracts were washed with water (50 mL), filtered through a hydrophobic frit and evaporated to dryness to yield a gum which was eluted through a 20g SPE silica cartridge with EtOAc : cyclohexane (1 : 9) to afford the title compound as a white solid (444 mg). LC/MS: m/z 287 [M+H]⁺, Rₜ 3.89 min.

### Intermediate 72 : Ethyl 3-[3-methyl-6-(trifluoromethyl)-1-benzothien-2-yl]-2-heptenoate

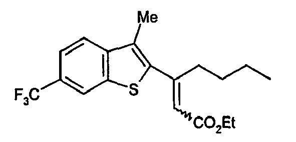

A solution of triethyl phosphonoacetate (1.67 g, 7.3 mmol) in anhydrous THF (25 mL) was stirred at 0°C under nitrogen and treated with sodium hydride (60% dispersion in oil, 0.29 g, 7.3 mmol). After 15 min a solution of 1-[3-methyl-6-(trifluoromethyl)-1-benzothien-2-yl]-1-pentanone (1.67 g, 5.5 mmol) in anhydrous THF (15 mL) was added, and the mixture was stirred at 20°C for 2 days, more sodium hydride (60% dispersion in oil, 0.29 g, 7.3 mmol) was added and stirring continued for a further 3 h. The mixture was diluted with water (250 mL) and extracted with DCM (3 x 100 mL). The organic extracts were combined, washed with water (150 mL), filtered through a hydrophobic frit, and evaporated to dryness. The residue was eluted through a 50g SPE silica cartridge with EtOAc : cyclohexane (1 : 9) to afford a mixture of starting ketone and isomers of the title compound as a yellow oil (1.75 g). This mixture was dissolved in anhydrous THF (20 mL), triethyl phosphonoacetate (0.34 g, 1.5 mmol) added and sodium hydride (60% dispersion in oil, 0.06 g, 1.5 mmol), and stirred at 20°C overnight. The reaction mixture was worked up as above to yield a mixture of isomers of the title compound as a pale yellow oil (1.85 g). LC/MS: m/z 371 [M+H]⁺, Rₜ 4.18 min and 4.35 min.

### Intermediate 73 : Ethyl 3-[3-methyl-6-(trifluoromethyl)-1-benzothien-2-yl]-2-hexenoate

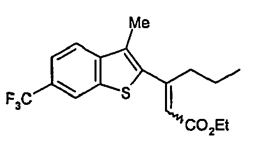

A solution of triethyl phosphonoacetate (576 mg, 2.5 mmol) in anhydrous THF (8 mL) was stirred under nitrogen and treated with sodium hydride (60% dispersion in oil, 100 mg, 2.5 mmol). After 10 min a solution of 1-[3-methyl-6-(trifluoromethyly)-benzothien-2-yl]-1-butanone (440 mg, 1.5 mmol) in anhydrous THF (4 mL) was added, and the mixture was stirred at 20°C overnight. The mixture was diluted with water (100 mL) and extracted with DCM (3 x 50 mL). The organic extracts were combined, washed with water (100 mL), filtered through a hydrophobic frit, and evaporated to dryness. The residue was eluted through a 20g SPE silica cartridge with EtOAc : cyclohexane (1 : 9) to afford a mixture of isomers of the title compound as an oil (550 mg). LC/MS: m/z 357 [M+H]⁺, Rₜ 4.10 min and 4.26 min.

### Intermediate 74 : Ethyl 3-[3-methyl-6-(trifluoromethyl)-1-benzothien-2-yl]heptanoate

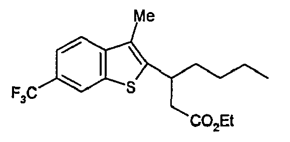

A solution of ethyl 3-[3-methyl-6-(trifluoromethyl)-1-benzothien-2-yl]-2-heptenoate (1.85 g, 5 mmol) in EtOAc (100 mL) was added to 10% palladium on carbon (wetted, 0.50 g), and the mixture was stirred under a hydrogen atmosphere at 20°C overnight. The mixture was filtered through a Celite pad, washing through with EtOAc. The filtrate and washings were evaporated to dryness and eluted through a 50g SPE silica cartridge with EtOAc : cyclohexane (1 : 19) to afford the title compound as an oil (1.62 g). ¹H NMR (400 MHz; CDCl₃) δ: 0.84 (3H, t, J 7 Hz), 1.15 (3H, t, J 7 Hz), 1.12-1.40 (4H, m), 1.65 (1 H, m), 1.76 (1 H, m), 2.40 (3H, s), 2.68 (2H, m). 3.70 (1 H, m), 4.05 (2H, q, J 7 Hz), 7.57 (1 H, d J 8 Hz), 7.71 (1 H, d, J 8 Hz), 8.03 (1 H, s).

### lntermediate 75 : Ethyl 3-[3-methyl-6-(trifluoromethyl)-1-benzothien-2-yl]hexanoate

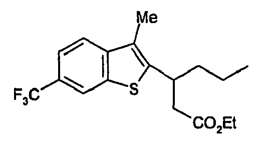

A solution of ethyl 3-[3-methyl-6-(trifluoromethyl)-1-benzothien-2-yl]-2-hexenoate (540 mg, 1.5 mmol) in EtOAc (40 mL) was added to 10% palladium on carbon (wetted, 150 mg), and the mixture was stirred under a hydrogen atmosphere at 20°C for 6 h. The mixture was filtered through a Celite pad, washing through with EtOAc. The filtrate and washings were evaporated to dryness to afford a mixture of the title compound and starting material. This mixture was redissolved in EtOAc (50 mL), added to 10% palladium on carbon (wetted, 150 mg), and stirred under a hydrogen atmosphere at 20°C overnight. The mixture was filtered through a Celite pad washing through with EtOAc. The filtrate and washings were evaporated to dryness and eluted through a 20g SPE silica cartridge with EtOAc : cyclohexane (1 : 19) to afford the title compound as an oil (502 mg). ¹H NMR (400 MHz; CDCl₃) δ: 0.89 (3H, t, J 7 Hz), 1.14 (3H, t, J 7 Hz), 1.25 (2H, m), 1.61-1.79 (2H, m), 2.40 (3H, s), 2.68 (2H, m). 3.72 (1 H, m), 4.05 (2H, q, J 7 Hz), 7.57 (1 H, d J 8 Hz), 7.70 (1 H, d, J 8 Hz), 8.03 (1 H, s).

### Intermediate 76 : 3-[3-Methyl-6-(trifluoromethyl)-1-benzothien-2-yl]-1-heptanol

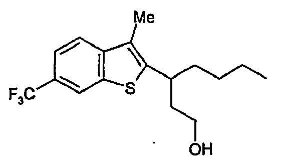

A solution of ethyl 3-[3-methyl-6-(trifluoromethyl)-1-benzothien-2-yl]heptanoate (1.62 g, 4.35 mmol) in anhydrous THF (30 mL) was stirred at 0°C under nitrogen and treated dropwise with 1.5M diisobutylaluminium hydride in toluene (9 mL). The mixture was stirred at 0°C for 1 h and at 20° for 2 h. Sufficient water was carefully added to the mixture to destroy excess reagent, and then diluted with EtOAc (100 mL) and 1 M hydrochloric acid (200 mL). The phases were separated and the aqueous layer extracted with EtOAc (2 x 100 mL). The combined organic extracts were washed with water (150 mL) and brine (150 mL), dried over MgSO₄ and evaporated to give an oil. This was eluted through a 50g SPE silica cartridge with EtOAc : cyclohexane (1 : 4) to afford the title compound as a colourless oil (1.2 g). ¹H NMR (400 MHz; CDCl₃) δ: 0.84 (3H, t, J 7 Hz), 1.15-1.38 (5H, m), 1.65 (1 H, m), 1.75 (1 H, m), 1.84 (1 H, m), 2.04 (1 H, m), 2.38 (3H, s), 3.38 (1 H, m). 3.48 (1 H, m), 3.64 (1 H, m), 7.58 (1 H, d J 8 Hz), 7.70 (1 H, d J 8 Hz), 8.04 (1 H, s).

### Intermediate 77 : 3-[3-Methyl-6-(trifluoromethyl)-1-benzothien-2-yl]-1-hexanol

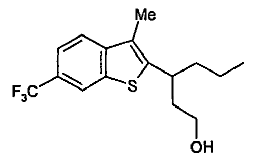

A solution of ethyl 3-[3-methyl-6-(trifluoromethyl)-1-benzothien-2-yl]hexanoate (500 mg, 1.4 mmol) in anhydrous THF (10 mL) was stirred at 0°C under nitrogen and treated dropwise with 1.5M diisobutylaluminium hydride in toluene (3 mL). The mixture was stirred at 0°C for 1 h. Sufficient water was carefully added to the mixture to destroy excess reagent, and then diluted with EtOAc (50 mL) and 1M hydrochloric acid (100 mL). The phases were separated and the aqueous layer extracted with EtOAc (2 x 50 mL). The combined organic extracts were washed with water (75 mL) and brine (75 mL), dried over MgSO₄ and evaporated to give an oil. This was eluted through a 20g SPE silica cartridge with EtOAc : cyclohexane (1 : 4) to afford the title compound as a colourless oil (390 mg). ¹H NMR (400 MHz; CDCl₃) δ: 0.88 (3H, t, J 7 Hz), 1.17-1.32 (3H, m), 1.60-1.90 (3H, m), 2.04 (1 H, m), 2.38 (3H, s), 3.40 (1 H, m). 3.48 (1 H, m), 3.64 (1H, m), 7.57 (1H, d J 8 Hz), 7.70 (1H, d, J 8 Hz), 8.04 (1H, s).

### Intermediate 78 : 2-[1-(2-Bromoethyl)butyl]-3-methyl-6-(trifluoromethyl)-1-benzothiophene

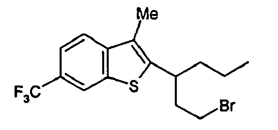

A solution of 3-[3-methyl-6-(trifluoromethyl)-1-benzothien-2-yl]-1-hexanol (390 mg, 1.25 mmol) in DCM (12 mL) was treated with triphenylphosphine (500 mg, 1.9 mmol) and carbon tetrabromide (625 mg, 1.9 mmol). The mixture was stirred at 20°C under nitrogen for 3 h, and then evaporated to dryness. The residue was eluted through a 20 g SPE silica cartridge with cyclohexane to afford the title compound as a colourless oil (328 mg). ¹H NMR (400 MHz; CDCl₃) δ: 0.89 (3H, t, J 7 Hz), 1.20-1.34 (2H, m), 1.60-1.78 (2H, m), 2.12-2.31 (2H, m), 2.42 (3H, s), 3.16 (1 H, m). 3.36-3.53 (2H, m), 7.59 (1H, d J 8 Hz), 7.72 (1H, d, J 8 Hz), 8.03 (1 H, s).

### Intermediate 79 : 2-[1-(2-Bromoethyl)pentyl]-3-methyl-6-(trifluoromethyl)-1-benzothiophene

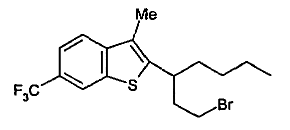

A solution of 3-[3-methyl-6-(trifluoromethyl)-1-benzothien-2-yl]-1-heptanol (413 mg, 1.25 mmol) in DCM (12 mL) was treated with triphenylphosphine (500 mg, 1.9 mmol) and carbon tetrabromide (625 mg, 1.9 mmol). The mixture was stirred at 20°C under nitrogen for 3 h, and then evaporated to dryness. The residue was eluted through a 20 g SPE silica cartridge with cyclohexane to afford the title compound as a colourless oil (365 mg). ¹H NMR (400 MHz; CDCl₃) δ: 0.84 (3H, t, J 7 Hz), 1.13-1.38 (4H, m), 1.60-1.81 (2H, m), 2.12-2.32 (2H, m), 2.43 (3H, s), 3.16 (1 H, m). 3.37-3.51 (2H, m), 7.59 (1 H, d J 8 Hz), 7.72 (1 H, d, J 8 Hz), 8.04 (1 H, s).

### Intermediate 80 : Ethyl {[2-methyl-4-({3-[3-methyl-6-(trifluoromethyl)-1-benzothien-2-yl]hexyl}thio)phenyl]oxy}acetate er

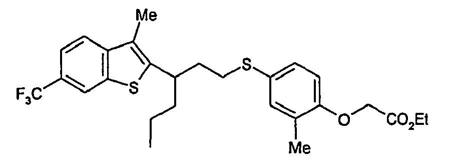

A solution of 2-[1-(2-bromoethyl)butyl]-3-methyl-6-(trifluoromethyl)-1-benzothiophene (80 mg, 0.21 mmol) in anhydrous MeCN (2.5 mL) was treated with cesium carbonate (200 mg) and a solution of ethyl [(4-mercapto-2-methylphenyl)oxy]acetate (75 mg, 0.33 mmol) in anhydrous MeCN (2.5 mL). The mixture was stirred at 20°C under nitrogen overnight, and then evaporated to dryness and eluted through a 10 g SPE silica cartridge with EtOAc : cyclohexane (1 : 20) to afford the title compound as a gum (125 mg). ¹H NMR (400 MHz; CDCl₃) δ: 0.86 (3H t, J 7 Hz), 1.17-1.30 (2H, m), 1.29 (3H, t, J 7 Hz), 1.52-1.72 (2H, m), 1.84-2.03 (2H, m), 2.22 (3H, s), 2.37 (3H, s), 2.63 (1 H, m), 2.79 (1 H, m), 3.38 (1 H, m), 4.27 (2H, q, J 7 Hz), 4.60 (2H, s), 6.59 (1H, d, J 8 Hz), 7.09 (1H, m), 7.13 (1H, m), 7.58 (1H, d, J 8 Hz), 7.70 (1H, d, J 8 Hz), 8.03 (1 H, s).

### Intermediate 81 : Ethyl [4-methyl-2-({3-[3-methyl-6-(trifluoromethyl)-1-benzothien-2-yl]hexyl}thio)-1,3-thiazol-5-yl]acetate

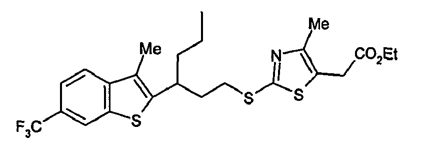

A solution of 2-[1-(2-bromoethyl)butyl]-3-methyl-6-(trifluoromethyl)-1-benzothiophene (80 mg, 0.21 mmol) in anhydrous MeCN (2.5 mL) was treated with cesium carbonate (200 mg) and a solution of ethyl (2-mercapto-4-methyl-1,3-thiazol-5-yl)acetate (75 mg, 0.35 mmol) in anhydrous MeCN (2.5 mL). The mixture was stirred at 20°C under nitrogen overnight, and then evaporated to dryness and eluted through a 10 g SPE silica cartridge with EtOAc : cyclohexane (1 : 20) to afford the title compound as a gum (80 mg). LC/MS: m/z 516 [M+H]⁺, Rₜ 4.38 min.

### Intermediate 82 : Ethyl {[2-methyl-4-({3-[3-methyl-6-(trifluoromethyl)-1-benzothien-2-yl]hexyl}oxy)phenyl]oxy}acetate

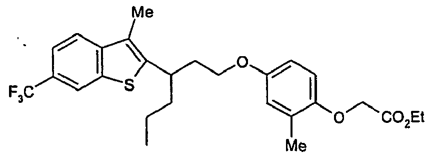

A solution of 2-[1-(2-bromoethyl)butyl]-3-methyl-6-(trifluoromethyl)-1-benzothiophene (80 mg, 0.21 mmol) in anhydrous MeCN (2.5 mL) was treated with cesium carbonate (200 mg) and a solution of ethyl [(4-hydroxy-2-methylphenyl)oxy]acetate (75 mg, 0.36 mmol) in anhydrous MeCN (2.5 mL). The mixture was stirred at 20°C under nitrogen for 5 days, and then evaporated to dryness and eluted through a 10 g SPE silica cartridge with EtOAc : cyclohexane (1 : 20) to afford the title compound as a gum (35 mg). ¹H NMR (400 MHz; CDCl₃) δ: 0.90 (3H, t, J 7 Hz), 1.25-1.30 (2H, m), 1.28 (3H, t, J 7 Hz), 1.62-1.82 (2H, m), 1.94 (1 H, m), 2.24 (1 H, m), 2.23 (3H, s), 2.28 (3H, s), 3.52 (1 H, m), 3.61 (1 H, m), 3.86 (1 H, m), 4.24 (2H, q, J 7 Hz), 4.55 (2H, s), 6.53 (1 H, dd, J 8 Hz, 3 Hz), 6.60 (1 H, d, J 8 Hz), 6.65 (1 H, J 3 Hz), 7.56 (1 H, d, J 8 Hz), 7.65 (1 H, d, J 8 Hz), 8.04 (1 H, s).

### Intermediate 83 : Ethyl {[2-ethyl-4-({3-[3-methyl-6-(trifluoromethyl)-1-benzothien-2-yl]hexyl}oxy)phenyl]oxy}acetate

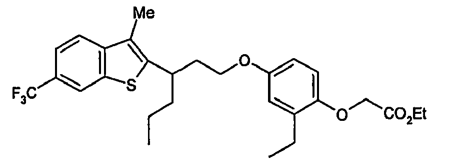

A solution of 2-[1-(2-bromoethyl)butyl]-3-methyl-6-(trifluoromethyl)-1-benzothiophene (80 mg, 0.21 mmol) in anhydrous MeCN (2.5 mL) was treated with cesium carbonate (200 mg) and a solution of ethyl [(2-ethyl-4-hydroxyphenyl)oxy]acetate (75 mg, 0.33 mmol) in anhydrous MeCN (2.5 mL). The mixture was stirred at 20°C under nitrogen for 5 days, and then evaporated to dryness and eluted through a 10 g SPE silica cartridge with EtOAc : cyclohexane (1 : 20) to afford the title compound as a gum (83 mg). ¹H NMR (400 MHz; CDCl₃) δ: 0.90 (3H, t, J 7 Hz), 1.18 (3H, t, J 7 Hz), 1.24-1.32 (2H, m), 1.28 (3H, t, J 7 Hz), 1.62-1.82 (2H, m), 1.95 (1 H, m), 2.25 (1 H, m), 2.28 (3H, s), 2.64 (2H, q, J 7 Hz), 3.53 (1 H, m), 3.63 (1 H, m), 3.87 (1 H, m), 4.24 (2H, q, J 7 Hz), 4.55 (2H, s), 6.53 (1 H, dd, J 8 Hz, 3 Hz), 6.60 (1 H, d, J 8 Hz), 6.68 (1 H, J 3 Hz), 7.56 (1 H, d, J 8 Hz), 7.65 (1H, d, J 8 Hz), 8.04 (1H, s).

### Intermediate 84 : Ethyl {[2-methyl-4-({3-[3-methyl-6-(trifluoromethyl)-1-benzothien-2-yl]heptyl}thio)phenyl]oxy}acetate

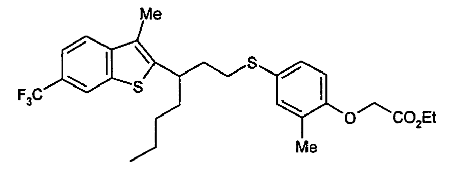

A solution of 2-[1-(2-bromoethyl)pentyl]-3-methyl-6-(trifluoromethyl)-1-benzothiophene (90 mg, 0.23 mmol) in anhydrous MeCN (2.5 mL) was treated with cesium carbonate (200 mg) and a solution of ethyl [(4-mercapto-2-methylphenyl)oxy]acetate (75 mg, 0.33 mmol) in anhydrous MeCN (2.5 mL). The mixture was stirred at 20°C under nitrogen overnight, and then evaporated to dryness and eluted through a 10 g SPE silica cartridge with EtOAc : cyclohexane (1 : 20) to afford the title compound as a gum (95 mg). ¹H NMR (400 MHz; CDCl₃) δ: 0.83 (3H, t, J 7 Hz), 1.10-1.30 (2H, m), 1.29 (3H, t, J 7 Hz), 1.55-1.75 (2H, m), 1.84-2.03 (2H, m), 2.22 (3H, s), 2.37 (3H, s), 2.61 (1 H, m), 2.78 (1 H, m), 3.36 (1 H, m), 4.26 (2H, q, J 7 Hz), 4.60 (2H, s), 6.59 (1H, d, J 8 Hz), 7.09 (1H, dd, J 8 Hz, 2 Hz), 7.13 (1H, d, J 2 Hz), 7.58 (1H, d, J 8 Hz), 7.70 (1 H, d, J 8 Hz), 8.03 (1H, s).

### Intermediate 85 : Ethyl [4-methyl-2-({3-[3-methyl-6-(trifluoromethyl)-1-benzothien-2-yl]heptyl}thio)-1,3-thiazol-5-yl]acetate

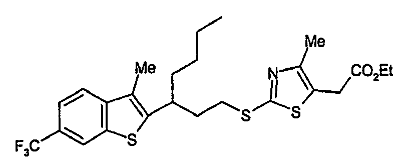

A solution of 2-[1-(2-bromoethyl)pentyl]-3-methyl-6-(trifluoromethyl)-1-benzothiophene (90 mg, 0.23 mmol) in anhydrous MeCN (2.5 mL) was treated with cesium carbonate (200 mg) and a solution of ethyl (2-mercapto-4-methyl-1,3-thiazol-5-yl)acetate (75 mg, 0.35 mmol) in anhydrous MeCN (2.5 mL). The mixture was stirred at 20°C under nitrogen overnight, and then evaporated to dryness and eluted through a 10 g SPE silica cartridge with EtOAc : cyclohexane (1 : 20) to afford the title compound as a gum (80 mg). LC/MS: m/z 530 [M+H]⁺, Rₜ 4.48 min.

### Intermediate 86 : Ethyl {[2-methyl-4-({3-[3-methyl-6-(trifluoromethyl)-1-benzothien-2-yl]heptyl}oxy)phenyl]oxy}acetate

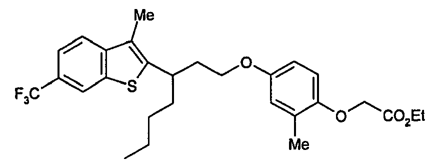

A solution of 2-[1-(2-bromoethyl)pentyl]-3-methyl-6-(trifluoromethyl)-1-benzothiophene (90 mg, 0.23 mmol) in anhydrous MeCN (2.5 mL) was treated with cesium carbonate (200 mg) and a solution of ethyl [(4-hydroxy-2-methylphenyl)oxy]acetate (75 mg, 0.36 mmol) in anhydrous MeCN (2.5 mL). The mixture was stirred at 20°C under nitrogen for 5 days, and then evaporated to dryness and eluted through a 10g SPE silica cartridge with EtOAc : cyclohexane (1 : 20) to afford the title compound as a gum (82 mg). ¹H NMR (400 MHz; CDCl₃) δ: 0.85 (3H, t, J 7 Hz), 1.15-1.35 (4H, m), 1.28 (3H, t, J 7 Hz), 1.62-1.85 (2H, m), 1.94 (1 H, m), 2.26 (1 H, m), 2.23 (3H, s), 2.28 (3H, s), 3.50 (1 H, m), 3.62 (1 H, m), 3.86 (1 H, m), 4.25 (2H, q, J 7 Hz), 4.55 (2H, s), 6.53 (1H, dd, J 8 Hz, 3 Hz), 6.60 (1 H, d, J 8 Hz), 6.65 (1 H, J 3 Hz), 7.56 (1 H, d, J 8 Hz), 7.65 (1 H, d, J 8 Hz), 8.04 (1H, s).

### Intermediate 87 : Ethyl {[2-ethyl-4-({3-[3-methyl-6-(trifluoromethyl)-1-benzothien-2-yl]heptyl}oxy)phenyl]oxy}acetate

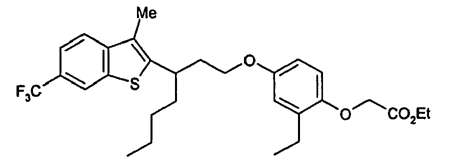

A solution of 2-[1-(2-bromoethyl)pentyl]-3-methyl-6-(trifluoromethyl)-1-benzothiophene (90 mg, 0.23 mmol) in anhydrous MeCN (2.5 mL) was treated with cesium carbonate (200 mg) and a solution of ethyl [(2-ethyl-4-hydroxyphenyl)oxy]acetate (75 mg, 0.33 mmol) in anhydrous MeCN (2.5 mL). The mixture was stirred at 20°C under nitrogen for 5 days, and then evaporated to dryness and eluted through a 10 g SPE silica cartridge with EtOAc : cyclohexane (1 : 20) to afford the title compound as a gum (80 mg). ¹H NMR (400 MHz; CDCl₃) δ: 0.85 (3H, t, J 7 Hz), 1.18 (3H, t, J 7 Hz), 1.24-1.32 (4H, m), 1.28 (3H, t, J 7 Hz), 1.65-1.85 (2H, m), 1.95 (1 H, m), 2.25 (1 H, m), 2.28 (3H, s), 2.64 (2H, q, J 7 Hz), 3.50 (1 H, m), 3.63 (1 H, m), 3.87 (1 H, m), 4.24 (2H, q, J 7 Hz), 4.55 (2H, s), 6.53 (1 H, dd, J 8 Hz, 3 Hz), 6.60 (1 H, d, J 8 Hz), 6.68 (1 H, J 3 Hz), 7.56 (1 H, d, J 8 Hz), 7.66 (1 H, d, J 8 Hz), 8.04 (1 H, s).

### Intermediate 88 : Methyl [4-({3-[3-methyl-6-(trifluoromethyl)-1-benzothien-2-yl]heptyl}oxy)phenyl]acetate

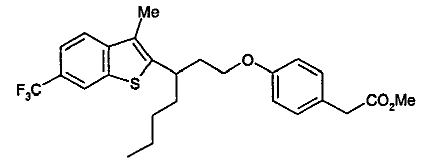

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of methyl (4-hydroxyphenyl)acetate (80 mg, 0.5 mmol) in anhydrous MeCN (2.5 mL). The mixture was stirred at 20°C under nitrogen for 3 days, and then evaporated to dryness and eluted through a 5 g SPE silica cartridge with EtOAc : cyclohexane (1 : 20) to afford the title compound as a gum (45 mg). ¹H NMR (400 MHz; CDCl₃) δ 0.85 (3H, t, J 7 Hz), 1.14-1.38 (4H, m), 1.63-1.86 (2H, m), 1.96 (1 H, m), 2.27 (1 H, m), 2.28 (3H, s), 3.51 (1 H, m), 3.54 (2H, s), 3.67 (1 H, m), 3.68 (3H, s), 3.91 (1 H, m), 6.77 (2H, d, J 8 Hz), 7.14 (2H, d, J 8 Hz), 7.56 (1H, d, J 8 Hz), 7.66 (1H, d, J 8 Hz), 8.04 (1H, s).

### Intermediate 89 : Methyl 3-[4-({3-[3-methyl-6-(trifluoromethyl)-1-benzothien-2-yl]heptyl}oxy)phenyl]propanoate

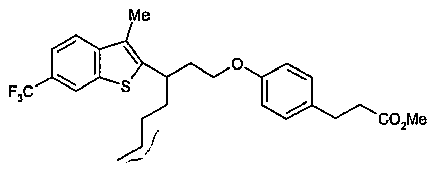

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of methyl 3-(4-hydroxyphenyl)propanoate (90 mg, 0.5 mmol) in anhydrous MeCN (2.5 mL). The mixture was stirred at 20°C under nitrogen for 3 days, and then evaporated to dryness and eluted through a 5 g SPE silica cartridge with EtOAc : cyclohexane (1 : 20) to afford the title compound as a gum (53 mg). ¹H NMR (400 MHz; CDCl₃) δ: 0.85 (3H, t, J 7 Hz), 1.16-1.38 (4H, m), 1.68 (1H, m), 1.81 (1 H, m), 1.96 (1 H, m), 2.27 (1 H, m), 2.27 (3H, s), 2.57 (2H, t, J 7 Hz), 2.86 (2H, t, J 7 Hz), 3.51 (1 H, m), 3.66 (1 H, m), 3.67 (3H, s), 3.89 (1 H, m), 6.73 (2H, d, J 8 Hz), 7.06 (2H, d, J 8 Hz), 7.55 (1 H, d, J 8 Hz), 7.65 (1 H, d, J 8 Hz), 8.04 (1H, s).

### Intermediate 90 : Methyl [3-chloro-4-({3-[3-methyl-6-(trifluoromethyl)-1-benzothien-2-yl]heptyl}oxy)phenyl]acetate

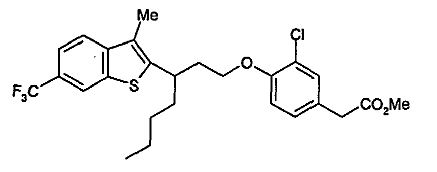

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of methyl (3-chloro-4-hydroxyphenyl)acetate (107 mg, 0.5 mmol) in anhydrous MeCN (2.5 mL). The mixture was stirred at 20°C under nitrogen for 3 days, and then evaporated to dryness and eluted through a 5 g SPE silica cartridge with EtOAc : cyclohexane (1 : 20) to afford the title compound as a gum (65 mg). ¹H NMR (400 MHz; CDCl₃) δ: 0.85 (3H, t, J 7 Hz), 1.16-1.37 (4H, m), 1.69 (1 H, m), 1.83 (1 H, m), 2.00 (1 H, m), 2.28 (3H, s), 2.35 (1H, m), 3.51 (2H, s), 3.61 (1 H, m), 3.69 (3H, s), 3.71 (1H, m), 3.99 (1 H, m), 6.70 (1 H, d, J 8 Hz), 7.02 (1 H, dd, J 8 Hz, 2 Hz), 7.29 (1H, d, J 2 Hz), 7.56 (1H, d, J 8 Hz), 7.66 (1H, d, J 8 Hz), 8.04 (1 H, s).

### Intermediate 91 : Ethyl [3-(methyloxy)-4-({3-[3-methyl-6-(trifluoromethyl)-1-benzothien-2-yl]heptyl}oxy)phenyl]acetate

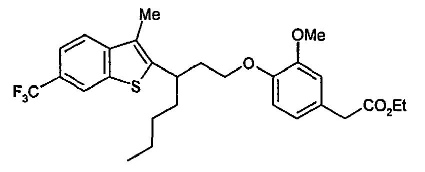

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of ethyl [4-hydroxy-3-(methyloxy)phenyl]acetate (112 mg, 0.5 mmol) in anhydrous MeCN (2.5 mL). The mixture was stirred at 20°C under nitrogen for 3 days, and then evaporated to dryness and eluted through a 5 g SPE silica cartridge with EtOAc : cyclohexane (1 : 20) to afford the title compound as a gum (32 mg). ¹H NMR (400 MHz; CDCl₃) δ: 0.84 (3H, t, J 7 Hz), 1.16-1.34 (4H, m), 1.24 (3H, t, J 7 Hz), 1.68 (1H, m), 1.82 (1 H, m), 2.02 (1H, m), 2.29 (3H, s), 2.35 (1 H, m), 3.52 (2H, s), 3.52 (1 H, m), 3.74 (1 H, m), 3.84 (3H, s), 3.95 (1 H, m), 4.14 (2H, q, J 7 Hz, 6.65 (1 H, d, J 8 Hz), 6.71 (1 H, dd, J 8 Hz, 2 Hz), 6.80 (1 H, d, J 2 Hz), 7.56 (1H, d, J 8 Hz), 7.66 (1H, d, J 8 Hz), 8.04 (1 H, s).

### Intermediate 92 : Ethyl [2-({3-[3-methyl-6-(trifluoromethyl)-1-benzothien-2-yl]heptyl}thio)-1,3-thiazol-4-yl]acetate

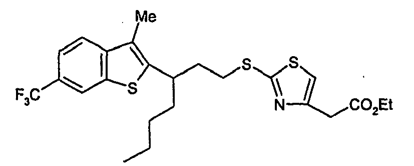

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of ethyl (2-mercapto-1,3-thiazol-4-yl)acetate (101 mg, 0.5 mmol) in anhydrous MeCN (2.5 mL). The mixture was stirred at 20°C under nitrogen for 3 days, and then evaporated to dryness and eluted through a 5 g SPE silica cartridge with EtOAc : cyclohexane (1 : 20) to afford the title compound as a gum (73 mg). LC/MS: m/z 516 [M+H]⁺, Rₜ 4.44 min.

### Intermediate 93 : Methyl 3-[3-(methyloxy)-4-({3-[3-methyl-6-(trifluoromethyl)-1-benzothien-2-yl]heptyl}oxy)phenyl]propanoate

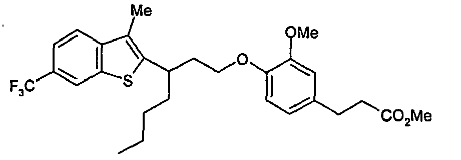

A solution of 2-[1-(2-bromoethyl)pentyl]-3-methyl-6-(trifluoromethyl)-1-benzothiophene (60 mg, 0.15 mmol) in anhydrous MeCN (2.5 mL) was treated with cesium carbonate (150 mg) and a solution of methyl 3-[4-hydroxy-3-(methyloxy)phenyl]propanoate (105 mg, 0.5 mmol) in anhydrous MeCN (2.5 mL). The mixture was stirred at 20°C under nitrogen for 3 days, and then evaporated to dryness and eluted through a 5g SPE silica cartridge with EtOAc : cyclohexane (1 : 20) to afford the title compound as a gum (11 mg). Further title compound was obtained by elution through a 5 g SPE silica cartridge with EtOAc : cyclohexane (1 : 10). The samples were combined to give the title compound as a gum (55 mg). ¹H NMR (400 MHz; CDCl₃) δ: 0.84 (3H, t, J 7 Hz), 1.15-1.35 (4H, m), 1.69 (1 H, m), 1.81 (1 H, m), 2.01 (1 H, m), 2.29 (3H, s), 2.34 (1 H, m), 2.59 (2H, t, J 7 Hz), 2.87 (2H, t, J 7 Hz), 3.51 (1 H, m), 3.66 (3H, s), 3.73 (1H, m), 3.83 (3H, s), 3.93 (1H, m), 6.62 (2H, s), 6.70 (1H, s), 6.80 (1 H, d, J 2 Hz), 7.56 (1 H, d, J 8 Hz), 7.66 (1 H, d, J 8 Hz), 8.04 (1 H, s).

### Intermediates 94 and 95 : Ethyl {[2-methyl-4-({(3R)-3-[3-methyl-6-(trifluoromethyl)-1-benzothien-2-yl]heptyl}oxy)phenyl]oxy}acetate and ethyl {[2-methyl-4-({(3S)-3-[3-methyl-6-(trifluoromethyl)-1-benzothien-2-yl]heptyl}oxy)phenyl]oxy}acetate

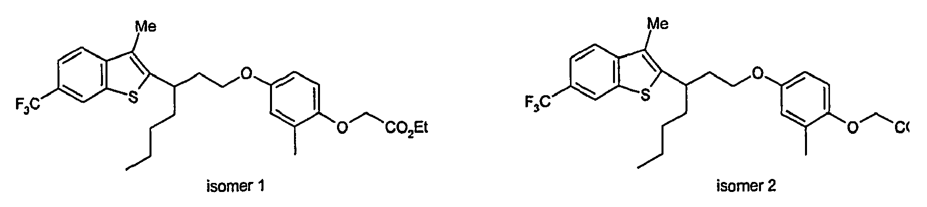

A solution of ethyl {[2-methyl-4-({3-[3-methyl-6-(trifluoromethyl)-1-benzothien-2-yl]heptyl}oxy)phenyl]oxy}acetate (130 mg) was eluted through a chiralpak hplc column AD with 2-propanol : heptane (1 : 19).Products eluting at 4.77 and 5.78 minutes were collected and evaporated to give gums of the title compounds isomer 1 (44 mg) and isomer 2 (45 mg) respectively.
isomer 1 LC/MS: m/z 540 [M+NH₄]⁺, Rₜ 4.52 min.
isomer 2 LC/MS: m/z 540 [M+NH₄]⁺, Rₜ 4.52 min.

### Intermediates 96 and 97: Ethyl [4-methyl-2-({(3R)-3-[3-methyl-6-(trifluoromethyl)-1-benzothien-2-yl]heptyl}thio)-1,3-thiazol-5-yl]acetate and ethyl [4-methyl-2-({(3S)-3-[3-methyl-6-(trifluoromethyl)-1-benzothien-2-yl]heptyl}thio)-1,3-thiazol-5-yl]acetate

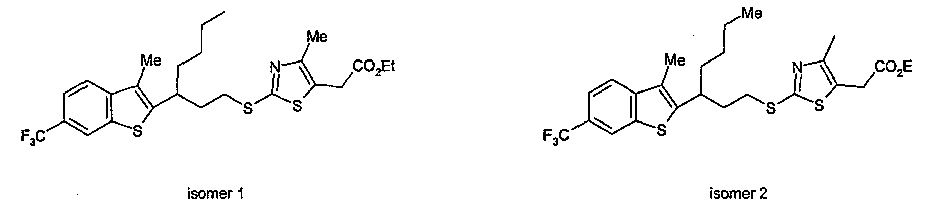

A solution of ethyl [4-methyl-2-({3-[3-methyl-6-(trifluoromethyl)-1-benzothien-2-yl]heptyl}thio)-1,3-thiazol-5-yl]acetate (130 mg) was eluted through a chiralcel hplc column OJ with ethanol : heptane (1 :19). Products eluting at 6.82 and 10.23 minutes were collected and evaporated to give gums of the title compounds isomer 1 (30 mg) and isomer 2 (30 mg) respectively.
isomer 1 LC/MS: m/z 530 [M+H]⁺, Rₜ 4.47 min.
isomer 2 LC/MS: m/z 530 [M+H]⁺, Rₜ 4.47 min.

### Intermediate 98 : 1-[3-Methyl-6-(trifluoromethyl)-1-benzothien-2-yl]-2-propen-1-one

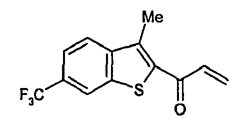

A solution of *N*,3-dimethyl-*N*-(methyloxy)-6-(trifluoromethyl)-1-benzothiophene-2-carboxamide (3.33 g, 11 mmol) in anhydrous THF (100 mL) was treated with 1M vinylmagnesium bromide in THF (33 mL, 33 mmol). The mixture was stirred at 20°C under nitrogen for 2 h. The mixture was then diluted with cold 1 M hydrochloric acid (1 L) and extracted with diethyl ether (3 x 400 mL). The combined organic extracts were washed with 1 M hydrochloric acid (400 mL), water (400 mL) and brine (400 mL), dried over MgSO₄, and evaporated. The residue was eluted through a 90 g Biotage^{™} silica cartridge with EtOAc : cyclohexane (1 : 8) and evaporated to dryness to yield the title compound as a cream solid (2.6 g). LC/MS: m/z 271 [M+H]⁺, Rₜ 3.72 min.

### Intermediate 99 : 3-(Methyloxy)-1-[3-methyl-6-(trifluoromethyl)-1-benzothien-2-yl]-1-propanone

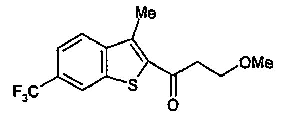

A solution of 1-[3-methyl-6-(trifluoromethyl)-1-benzothien-2-yl]-2-propen-1-one (1.08 g, 4 mmol) in anhydrous DCM (10 mL) and anhydrous MeOH (0. 18 mL, 4.5 mmol) was treated with palladium dichlorobisMeCN (100 mg, 0.4 mmol). The mixture was stirred at 20°C under nitrogen for 7 h. The mixture was then filtered through a pad of Celite and washed with DCM (100 mL). The filtrate and washings were washed with brine (50 mL), filtered through a hydrophobic frit, and evaporated. The residue was eluted through a 90 g Biotage^{™} silica cartridge with EtOAc : cyclohexane (1 : 6) and evaporated to dryness to yield the title compound as a pale yellow solid (850 mg). LC/MS: m/z 303 [M+H]⁺, Rₜ 3.53 min.

### Intermediate 100 : Ethyl 5-(methyloxy)-3-[3-methyl-6-(trifluoromethyl)-1-benzothien-2-yl]-2-pentenoate

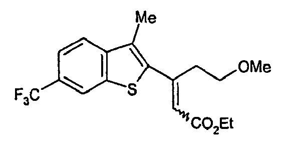

A solution of triethyl phosphonoacetate (1 mL, 1.1 g, 4.75 mmol) in anhydrous THF (15 mL) was stirred under nitrogen and treated with sodium hydride (60% dispersion in oil, 190 mg, 4.75 mmol). After 10 min a solution of 3-(methyloxy)-1-[3-methyl-6-(trifluoromethyl)-1-benzothien-2-yl]-1-propanone (755 mg, 2.5 mmol) in anhydrous THF (15 mL) was added, and the mixture was stirred at 20°C for 4 h. The mixture was diluted with water (200 mL) and extracted with EtOAc (3 x 100 mL). The organic extracts were combined, washed with water (100 mL) and brine (100 mL), dried over MgSO₄, and evaporated. The residue was eluted through a 90 g Biotage^{™} silica cartridge with EtOAc: cyclohexane (1 : 5) to afford a mixture of isomers of the title compound as an oil (590 mg). LC/MS: m/z 373 [M+H]⁺, Rₜ 3.74 min and 3.95 min.

### Intermediate 101 : Ethyl 5-(methyloxy)-3-[3-methyl-6-(trifluoromethyl)-1-benzothien-2-yl]pentanoate

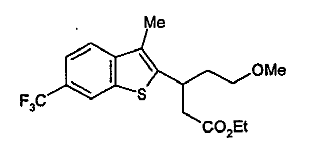

A solution of ethyl 5-(methyloxy)-3-[3-methyl-6-(trifluoromethyl)-1-benzothien-2-yl]-2-pentenoate (560 mg, 1.5 mmol) in EtOAc (30 mL) was added to 10% palladium on carbon (wetted, 150 mg), and the mixture was stirred under a hydrogen atmosphere at 20°C overnight. The mixture was filtered through a Celite pad, washing through with EtOAc. The filtrate and washings were evaporated to dryness and eluted through a 90 g Biotage^{™} silica cartridge with EtOAc : cyclohexane (1 : 5) to afford the title compound as an oil (400 mg). ¹H NMR (400 MHz; CDCl₃) δ: 1.15 (3H, t, J 7 Hz), 1.86 (1 H, m), 2.11 (1 H, m), 2.42 (3H, s), 2.74 (2H, m). 3.17 (1 H, m), 3.33 (1 H, m), 3.28 (3H, s), 3.96 (1 H, m), 4.05 (2H, q, J 7 Hz), 7.59 (1 H, d J 8 Hz), 7.73 (1 H, d, J 8 Hz), 8.05 (1 H, s).

### Intermediate 102 : 5-(Methyloxy)-3-[3-methyl-6-(trifluoromethyl)-1-benzothien-2-yl]-1-pentanol

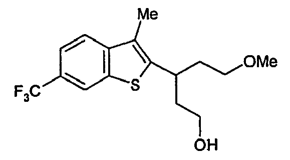

A solution of ethyl 5-(methyloxy)-3-[-methyl-6-(trifluoromethyl)-1-benzothien-2-yl]pentanoate (400 mg, 1.07 mmol) in anhydrous THF (10 mL) was stirred at 0°C under nitrogen and treated dropwise with 1.5M diisobutylaluminium hydride in toluene (2 mL). The mixture was stirred at 0°C for 1 h and at 20°C for 2 h. Sufficient water was carefully added to the mixture to destroy excess reagent, and then diluted with 0.5M hydrochloric acid (70 mL) and extracted with EtOAc (3 x 40 mL). The combined organic extracts were washed with water (60 mL) and brine (60 mL), dried over MgSO₄ and evaporated to give an oil. This was eluted through a 90 g Biotage^{™} silica cartridge with EtOAc: cyclohexane (1 : 3, then 1 : 2) to afford the title compound as a colourless oil (332 mg). ¹H NMR (400 MHz; CDCl₃) δ: 1.28 (1H, m), 1.81-1.96 (2H, m), 2.01-2.15 (2H, m), 2.40 (3H, s), 3.17 (1 H, m). 3.27 (3H, s), 3.36 (1 H, m), 3.51 (1 H, m), 3.65 (2H, m), 7.60 (1 H, d J 8 Hz), 7.73 (1 H, d, J 8 Hz), 8.05 (1 H, s).

### Intermediate 103 : 2-{3-Bromo-1-[2-(methyloxy)ethyl]propyl}-3-methyl-6-(trifluoromethyl)-1-benzothiophene

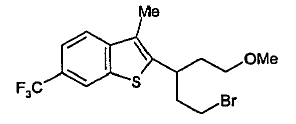

A solution of 5-(methyloxy)-3-[3-methyl-6-(trifluoromethyl)-1-benzothien-2-yl]-1-pentanol (330 mg, 1.0 mmol) in DCM (12 mL) was treated with triphenylphosphine (420 mg, 1.6 mmol) and carbon tetrabromide (520 mg, 1.6 mmol). The mixture was stirred at 20°C under nitrogen overnight, and then evaporated to dryness. The residue was eluted through a 40 g Biotage^{™} silica cartridge with EtOAc : cyclohexane (1 : 4) to afford the title compound as a colourless oil (204 mg). ¹H NMR (400 MHz; CDCl₃) δ: 1.85 (1H, m), 2.09 (1H, m), 2.18-2.35 (2H, m), 2.44 (3H, s), 3.11-3.22 (2H, m). 3.28 (3H, s), 3.30-3.44 (2H, m), 3.72 (1H, m), 7.61 (1H, d J 8 Hz), 7.75 (1 H, d, J 8 Hz), 8.06 (1 H, s).

### Intermediate 104 : Ethyl {[2-methyl-4-({5-(methyloxy)-3-[3-methyl-6-(trifluoromethyl)-1-benzothien-2-yl]pentyl}thio)phenyl]oxy}acetate

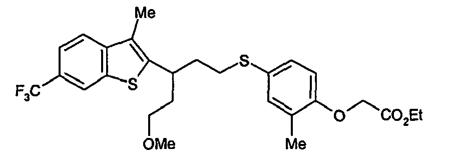

A solution of 2-{3-bromo-1-[2-(methyloxy)ethyl]propyl}-3-methyl-6-(trifluoromethyl)-1-benzothiophene (80 mg, 0.2 mmol) in anhydrous MeCN (3 mL) was treated with cesium carbonate (200 mg) and a solution of ethyl [(4-mercapto-2-methylphenyl)oxy]acetate (90 mg, 0.4 mmol) in anhydrous MeCN (3 mL). The mixture was stirred at 20°C under nitrogen overnight, and then evaporated to dryness and eluted through a 10g SPE silica cartridge with EtOAc : cyclohexane (1 : 4) to afford after evaporation a mixture of the title compound and the disulphide of the starting reagent. This material was eluted through a 12 g Biotage^{™} silica cartridge with EtOAc: cyclohexane (1 : 4) and evaporated to yield the title compound (75 mg). ¹H NMR (400 MHz; CDCl₃) δ: 1.30 (3H, t, J 7 Hz), 1.74-2.09 (4H, m), 2.23 (3H, s), 2.39 (3H, s), 2.64 (1 H, m), 2.77 (1 H, m), 3.12 (1 H, m), 3.26 (3H, s), 3.30 (1 H, m), 3.61 (1 H, m), 4.27 (2H, q, J 7 Hz), 4.62 (2H, s), 6.60 (1H, d, J 8 Hz), 7.10 (1 H, d, J 8 Hz), 7.13 (1 H, s), 7.60 (1 H, d, J 8 Hz), 7.72 (1 H, d, J 8 Hz), 8.05 (1 H, s).

### Intermediate 105 : Ethyl [4-methyl-2-({5-(methyloxy)-3-[3-methyl-6-(trifluoromethyl)-1-benzothien-2-yl]pentyl}thio)-1,3-thiazol-5-yl]acetate

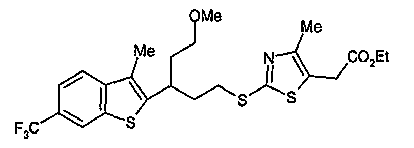

A solution of [1-(2-bromo-ethyl)-3-methoxy-propyl]-3-methyl-6-trifluoromethyl-benzo[b]thiophene (80 mg, 0.2 mmol) in anhydrous MeCN (3 mL) was treated with cesium carbonate (200 mg) and a solution of ethyl (2-mercapto-4-methyl-1,3-thiazol-5-yl)acetate (88 mg, 0.4 mmol) in anhydrous MeCN (3 mL). The mixture was stirred at 20°C under nitrogen overnight, and then evaporated to dryness and eluted through a 10 g SPE silica cartridge with EtOAc : cyclohexane (1 : 4) to afford the title compound as a gum (60 mg). LC/MS: m/z 532 [M+H]⁺, Rₜ 4.15 min.

### Intermediate 106 : Ethyl {[2-methyl-4-({5-(methyloxy)-3-[3-methyl-6-(trifluoromethyl)-1-benzothien-2-yl]pentyl}oxy)phenyl]oxy}acetate

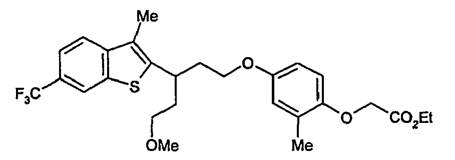

A solution of 2-{3-bromo-1-[2-(methyloxy)ethyl]propyl}-3-methyl-6-(trifluoromethyl)-1-benzothiophene (50 mg, 0.125 mmol) in anhydrous MeCN (3 mL) was treated with cesium carbonate (200 mg) and a solution of ethyl [(4-hydroxy-2-methylphenyl)oxy]acetate (84 mg, 0.4 mmol) in anhydrous MeCN (3 mL). The mixture was stirred at 20°C under nitrogen for 4 days, and then evaporated to dryness and eluted through a 10 g SPE silica cartridge with EtOAc : cyclohexane (1 : 4) to afford the title compound as a gum (51 mg). ¹H NMR (400 MHz; CDCl₃) δ: 1.29 (3H, t, J 7 Hz), 1.83-2.38 (4H, m), 2.23 (3H, s), 2.30 (3H, s), 3.18 (1 H, m), 3.27 (3H, s), 3.36 (1 H, m), 3.64 (1 H, m), 3.75 (1 H, m), 3.87 (1 H, m), 4.25 (2H, q, J 7 Hz), 4.55 (2H, s), 6.53 (1H, dd, J 8 Hz, 3 Hz), 6.60 (1 H, d, J 8 Hz), 6.65 (1 H, J 3 Hz), 7.58 (1 H, d, J 8 Hz), 7.68 (1 H, d, J 8 Hz), 8.05 (1 H, s).

### Intermediate 107: Ethyl {[2-ethyl-4-({5-(methyloxy)-3-[3-methyl-6-(trifluoromethyl)-1-benzothien-2-yl]pentyl}oxy)phenyl]oxy}acetate

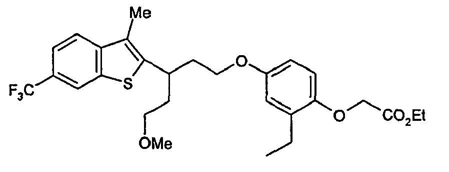

A solution of 2-{3-bromo-1-[2-(methyloxy)ethyl]propyl}-3-methyl-6-(trifluoromethyl)-1-benzothiophene (50 mg, 0.125 mmol) in anhydrous MeCN (3 mL) was treated with cesium carbonate (200 mg) and a solution of ethyl [(2-ethyl-4-hydroxyphenyl)oxy]acetate (90 mg, 0.4 mmol) in anhydrous MeCN (3 mL). The mixture was stirred at 20°C under nitrogen for 4 days, and then evaporated to dryness and eluted through a 10 g SPE silica cartridge with EtOAc : cyclohexane (1 : 4) to afford the title compound as a gum (45 mg). ¹H NMR (400 MHz; CDCl₃) δ: 1.18 (3H, t, J 7 Hz), 1.28 (3H, t, J 7 Hz), 1.83-2.38 (4H, m), 2.30 (3H, s), 2.64 (2H, q, J 7 Hz), 3.18 (1 H, m), 3.27 (3H, s), 3.36 (1 H, m), 3.66 (1 H, m), 3.75 (1 H, m), 3.86 (1 H, m), 4.25 (2H, q, J 7 Hz), 4.55 (2H, s), 6.53 (1 H, dd, J 8 Hz, 3 Hz), 6.60 (1 H, d, J 8 Hz), 6.67 (1 H, J 3 Hz), 7.58 (1 H, d, J 8 Hz), 7.68 (1 H, d, J 8 Hz), 8.05 (1 H, s).

### Intermediate 108 : 1-[3-Methyl-6-(trifluoromethyl)-1-benzothien-2-yl]-3-[(phenylmethyl)oxy]-1-propanone

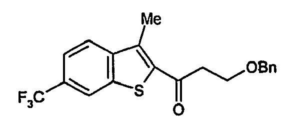

A solution of 1-[3-methyl-6-(trifluoromethyl)-1-benzothien-2-yl]-2-propen-1-one (540 mg, 2 mmol) in anhydrous DCM (5 mL) was treated with benzyl alcohol (0.23 mL, 2.2 mmol) and palladium dichlorobisMeCN (50 mg, 0.2 mmol). The mixture was stirred at 20°C under nitrogen overnight. The mixture was diluted with DCM (50 mL) and washed with brine (30 mL). The organic solution was dried by filtration through a hydrophobic frit, and evaporated to dryness. The residue was eluted through a 90 g Biotage^{™} silica cartridge with EtOAc: cyclohexane (1 : 8) and evaporated to yield an oil which crystallised to a pale yellow solid of the title compound (555 mg). LC/MS: m/z 379 [M+H]⁺, Rₜ 3.90 min.

### Intermediate 109: 1-[3-Methyl-6-(trifluoromethyl)-1-benzothien-2-yl]-3-[(phenylmethyl)oxy]-1-propanol

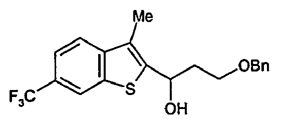

A solution of 1-[3-methyl-6-(trifluoromethyl)-1-benzothien-2-yl]-3-[(phenylmethyl)oxy]-1-propanone (360 mg, 0.95 mmol) in anhydrous THF (10 mL) was stirred at 20°C under nitrogen and treated dropwise with 1.5M diisobutylaluminium hydride in toluene (2 mL). The mixture was stirred at 20°C for 2 h. Sufficient water was carefully added to the mixture to destroy excess reagent, and then diluted with 1 M hydrochloric acid (50 mL) and extracted with EtOAc (3 x 50 mL). The combined organic extracts were washed with water (50 mL) and brine (50 mL), dried over MgSO₄ and evaporated to give an oil. This was eluted through a 40g Biotage^{™} silica cartridge with EtOAc: cyclohexane (1 : 4) to afford the title compound as a white solid (273 mg). ¹H NMR (400 MHz; CDCl₃) δ: 2.09 (1 H, m), 2.23 (1 H, m), 2.38 (3H, s), 3.69-3.82 (3H, m). 4.58 (2H, s), 5.45 (1H, m), 7.30-7.41 (5H, m), 7.59 (1H, d, J 8 Hz), 7.73 (1 H, d, J 8 Hz), 8.10 (1 H, s).

### Intermediate 110 : 3-Methyl-2-[3-[(phenylmethyl)oxy]-1-(2-propen-1-yloxy)propyl]-6-(trifluoromethyl)-1-benzothiophene

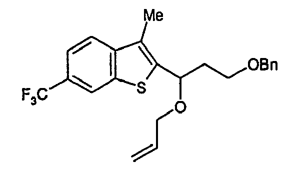

A solution of 1-[3-methyl-6-(trifluoromethyl)-1-benzothien-2-yl]-3-[(phenylmethyl)oxy]-1-propanol (360 mg, 0.95 mmol) in anhydrous THF (15 mL) was treated with 60% sodium hydride oil dispersion (36 mg, 0.9 mmol) and stirred at 20°C under nitrogen for 5 minutes. Allyl bromide (0.117 mL, 162 mg, 1.35 mmol) was added and the mixture was stirred at 60°C under nitrogen overnight. The reaction mixture was diluted with water (100 mL), acidifed with 2M hydrochloric acid, and extracted with EtOAc (2 x 50 mL). The organic extracts were combined, washed with aqueous sodium bicarbonate (50 mL) and brine (50 mL), dried over MgSO₄ and evaporated. The residue was eluted through a 40 g Biotage^{™} silica cartridge with EtOAc : cyclohexane (1 : 6) to afford the title compound as a colourless oil (243 mg). ¹H NMR (400 MHz; CDCl₃) δ: 2.05 (1 H, m), 2.29 (1 H, m), 2.39 (3H, s), 3.45 (1 H, m), 3.69 (1 H, m), 3.85 (1 H, m), 4.03 (1 H, m), 4.50 (2H, q, J 7 Hz), 5.08 (1 H, t, J 6 Hz), 5:19 (1 H, d, J 10 Hz), 5.25 (1 H, d, J 16 Hz), 5.90 (1 H, m), 7.28-7.41 (5H, m), 7.61 (1H, d, J 8 Hz), 7.75 (1 H, d, J 8 Hz), 8.09 (1 H, s).

### Intermediate 111 : 3-[3-Methyl-6-(trifluoromethyl)-1-benzothien-2-yl]-3-(propyloxy)-1-propanol

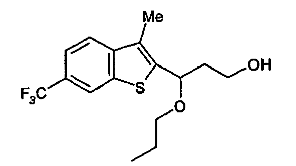

A solution of 3-methyl-2-[3-[(phenylmethyl)oxy]-1-(2-propen-1-yloxy)propyl]-6-(trifluoromethyl)-1-benzothiophene (270 mg, 0.64 mmol) in EtOAc (20 mL) was added to 10% palladium on carbon (wetted, 120 mg), and the mixture was stirred under a hydrogen atmosphere at 20°C overnight. The mixture was filtered through a Celite pad, washing through with EtOAc. The filtrate and washings were evaporated to dryness and eluted through a 40 g Biotage^{™} silica cartridge with EtOAc : cyclohexane (1 : 3) to afford the title compound as a colourless oil (185 mg). ¹H NMR (400 MHz; CDCl₃) δ: 0.94 (3H, t, J 7 Hz), 1.64 (2H, m), 1.99 (1 H, m), 2.22 (1H, m), 2.41 (1H, m), 2.43 (3H, s), 3.38 (1H, m). 3.47 (1H, m), 3.87 (2H, m), 5.02 (1H, m), 7.61 (1 H, d J 8 Hz), 7.76 (1H, d, J 8 Hz), 8.10 (1 H, s).

### Intermediate 112 : 2-[3-Bromo-1-(propyloxy)propyl]-3-methyl-6-(trifluoromethyl)-1-benzothiophene

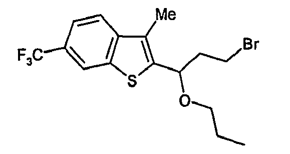

A solution of 3-[3-methyl-6-(trifluoromethyl)-1-benzothien-2-yl]-3-(propyloxy)-1-propanol (183 mg, 0.55 mmol) in DCM (6 mL) was treated with triphenylphosphine (232 mg, 0.88 mmol) and carbon tetrabromide (286 mg, 0.88 mmol). The mixture was stirred at 20°C under nitrogen overnight, and then evaporated to dryness. The residue was eluted through a 40 g Biotage^{™} silica cartridge with EtOAc: cyclohexane (1 : 4) to afford the title compound as a colourless oil (132 mg). ¹H NMR (400 MHz; CDCl₃) δ: 0.94 (3H, t, J 7 Hz), 1.63 (2H, m), 2.21 (1 H, m), 2.47 (1 H, m), 2.46 (3H, s), 3.36 (1 H, m). 3.46 (2H, m), 3.68 (1H, m), 5.04 (1 H, m), 7.61 (1 H, d J 8 Hz), 7.76 (1 H, d, J 8 Hz), 8.10 (1 H, s).

### Intermediate 113 : Ethyl [(2-methyl-4-{[3-[3-methyl-6-(trifluoromethyl)-1-benzothien-2-yl]-3-(propyloxy)propyl]thio}phenyl)oxy]acetate

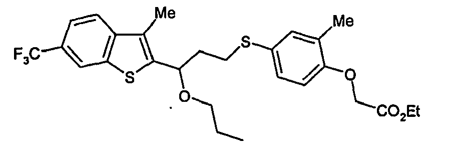

A solution of 2-[3-bromo-1-(propyloxy)propyl]-3-methyl-6-(trifluoromethyl)-1-benzothiophene (65 mg, 0.16 mmol) in anhydrous MeCN (2.5 mL) was treated with cesium carbonate (165 mg) and a solution of ethyl [(4-mercapto-2-methylphenyl)oxy]acetate (75 mg, 0.33 mmol) in anhydrous MeCN (2.5 mL). The mixture was stirred at 20°C under nitrogen overnight, and then evaporated to dryness. The residue was eluted through a 12 g Biotage^{™} silica cartridge with EtOAc : cyclohexane (1 : 4) and evaporated to yield the title compound as a colourless oil (70 mg). ¹H NMR (400 MHz; CDCl₃) δ: 0.92 (3H, t, J 7 Hz), 1.30 (3H, t, J 7 Hz), 1.60 (2H, m), 1.95 (1 H, m), 2.20 (1 H, m), 2.26 (3H, s), 2.39 (3H, s), 2.92-3.06 (2H, m), 3.29 (1 H, m), 3.41 (1 H, m), 4.27 (2H, q, J 7 Hz), 4.62 (2H, s), 4.97 (1 H, m), 6.62 (1 H, d, J 8 Hz), 7.18 (1H, d, J 8 Hz), 7.22 (1H, s), 7.59 (1H, d, J 8 Hz), 7.73 (1H, d, J 8 Hz), 8.08 (1H, s).

### Intermediate 114 : Ethyl [(2-methyl-4-{[3-[3-methyl-6-(trifluoromethyl)-1-benzothien-2-yl]-3-(propyloxy)propyl]oxy}phenyl)oxy]acetate

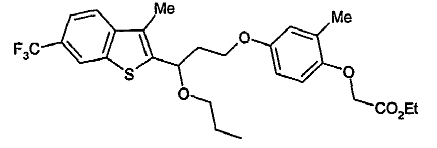

A solution of 2-[3-bromo-1-(propyloxy)propyl]-3-methyl-6-(trifluoromethyl)-1-benzothiophene (65 mg, 0.16 mmol) in anhydrous MeCN (2.5 mL) was treated with cesium carbonate (165 mg) and a solution of ethyl [(4-hydroxy-2-methylphenyl)oxy]acetate (75 mg, 0.33 mmol) in anhydrous MeCN (2.5 mL). The mixture was stirred at 20°C under nitrogen for 5 days, and then evaporated to dryness. The residue was eluted through a 12 g Biotage^{™} silica cartridge with EtOAc: cyclohexane (1 : 4) to afford the title compound as a colourless oil (70 mg). ¹H NMR (400 MHz; CDCl₃) δ: 0.91 (3H, t, J 7 Hz), 1.31 (3H, t, J 7 Hz), 1.60 (2H, m), 2.15 (1 H, m), 2.28 (3H, s), 2.38 (1 H, m), 2.38 (3H, s), 3.34 (1 H, m), 3.43 (1H, m), 3.84 (1 H, m), 4.11 (1H, m), 4.27 (2H, q, J 7 Hz), 4.59 (2H, s), 5.07 (1 H, m), 6.63 (1 H, dd, J 8 Hz, 2 Hz), 6.67 (1 H, d, J 8 Hz), 6.74 (1H, d, J 2 Hz), 7.59 (1H, d, J 8 Hz), 7.73 (1 H, d, J 8 Hz), 8.09 (1 H, s).

### Intermediate 115 : Methyl 6-fluoro-3-methyl-1-benzothiophene-2-carboxylate

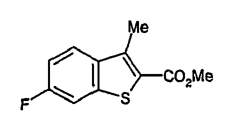

Methyl thioglycolate (3.5 g, 33 mmol) was added to a stirred suspension of sodium hydride (60% dispersion in oil, 2.0 g, 50 mmol) in anhydrous THF (15 mL) and anhydrous DMSO (40 mL). The mixture was stirred for 5 min to allow effervescence to subside, and then a solution of 2',4'-difluoroacetophenone (4 g, 25 mmol) in anhydrous DMSO (4 mL) was added. The mixture, which got hot, was stirred at ambient temperature for 2 h, and then diluted with water (500 mL) and extracted with EtOAc (3 x 200 mL). The combined organic extracts were washed with aqueous sodium bicarbonate (2 x 200 mL), water (200 mL) and brine (200 mL), dried over MgSO₄ and evaporated. The impure residue was eluted through a 50 g SPE silica cartridge with EtOAc : cyclohexane (1 :10), and the less polar fraction was collected and evaporated to yield the title compound as a white solid (1.39 g). LC/MS: m/z 225 [M+H]⁺, Rₜ 3.47 min.

### Intermediate 116 : 6-Fluoro-3-methyl-1-benzothiophene-2-carboxylic acid

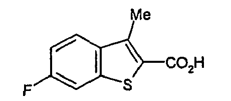

A solution of methyl 6-fluoro-3-methyl-1-benzothiophene-2-carboxylate (1.73 g, 7.7 mmol) in MeOH (125 mL) was treated with 2M aqueous sodium hydroxide (15 mL, 30 mmol) and stirred under reflux for 3 h. The mixture was cooled and evaporated to dryness. The residue was diluted with water (200 mL), acidified (2M hydrochloric acid) and filtered. The solid was washed with water and dried *in vacuo* at 50°C to afford the title compound as a white solid (1.65 g). LC/MS: m/z 209 [M-H]⁻, Rₜ 3.45 min.

### Intermediate 117 : 6-Fluoro-N,3-dimethyl-N-(methyloxy)-1-benzothiophene-2-carboxamide

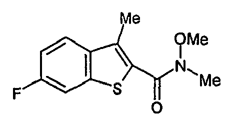

A suspension of 6-fluoro-3-methyl-1-benzothiophene-2-carboxylic acid (1.6 g, 7.6 mmol) in DCM (100 mL) was treated with carbonyldiimidazole (1.6 g, 10 mmol) and stirred at 20°C under nitrogen for 1.5 h. A solution of N,O-bismethylhydroxylamine, generated from the hydrochloride salt (1.5 g, 15 mmol) by partitioning between aqueous sodium carbonate solution (30 mL) and DCM (2 x 30 mL), was added portionwise and the mixture was stirred at 20°C for 3 days. The DCM solution was then washed with aqueous sodium bicarbonate solution (2 x 100 mL), and brine (100 mL), dried by filtration through a hydrophobic frit and evaporated. The residue was eluted through a 50 g SPE silica cartridge with EtOAc : cyclohexane (1 : 9, then 1 : 4) to afford the title compound, which was isolated as a white solid (600 mg). LC/MS: m/z 254 [M+H]⁺, Rₜ 3.09 min.

### Intermediate 118: 1-(6-Fluoro-3-methyl-1-benzothien-2-yl)-1-pentanone

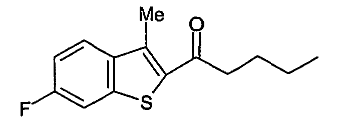

A solution of 6-fluoro-*N*,3-dimethyl-*N*-(methyloxy)-1-benzothiophene-2-carboxamide (595 mg, 2.35 mmol) in anhydrous THF (10 mL) was stirred under nitrogen at -70°C and treated with 20% butylmagnesium chloride in THF/toluene (3 mL, 4.8 mmol). The mixture was stirred at -70°C for 1 h, and then at 20°C overnight. The mixture was then diluted with aqueous ammonium chloride (75 mL) and extracted with DCM (3 x 50 mL). The combined organic extracts were washed with water (50 mL), filtered through a hydrophobic frit and evaporated to dryness. The residue was eluted through a 20 g SPE silica cartridge with EtOAc : cyclohexane (1 : 19) to afford the title compound as a white solid (420 mg). LC/MS: m/z 251 [M+H]⁺, Rₜ 3.88 min.

### Intermediate 119 : Ethyl 3-(6-fluoro-3-methyl-1-benzothien-2-yl)-2-heptenoate

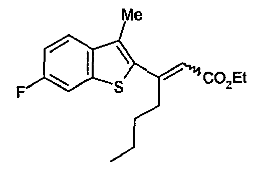

A solution of triethyl phosphonoacetate (645 mg, 2.8 mmol) in anhydrous THF (10 mL) was stirred at 0°C under nitrogen and treated with sodium hydride (60% dispersion in oil, 112 mg, 2.8 mmol). After 10 min a solution of 1-(6-fluoro-3-methyl-1-benzothien-2-yl)-1-pentanone (420 mg, 1.7 mmol) in anhydrous THF (5 mL) was added, and the mixture was stirred at 20°C for 5 days. The mixture was diluted with water (100 mL) and extracted with DCM (3 x 50 mL). The organic extracts were combined, washed with water (75 mL), filtered through a hydrophobic frit, and evaporated to dryness. The residue was eluted through a 20 g SPE silica cartridge with EtOAc : cyclohexane (1 : 19) to afford a mixture of isomers of the title compound as a colourless oil (525 mg). LC/MS: m/z 321 [M+H]⁺, Rₜ 4.01 min and 4.20 min.

### Intermediate 120: Ethyl 3-(6-fluoro-3-methyl-1-benzothien-2-yl)heptanoate

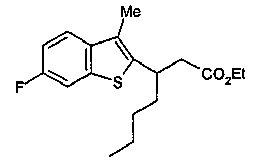

A solution of ethyl 3-(6-fluoro-3-methyl-1-benzothien-2-yl)-2-heptenoate (210 mg, 0.66 mmol) in EtOAc (12 mL) was added to 10% palladium on carbon (wetted, 50 mg), and the mixture was stirred under a hydrogen atmosphere at 20°C for 5 h. The mixture was filtered through a Celite pad, washing through with EtOAc. The filtrate and washings were evaporated to dryness to afford the title compound as an oil (182 mg). ¹H NMR (400 MHz; CDCl₃) δ: 0.84 (3H, t, J 7 Hz), 1.14 (3H, t, J 7 Hz), 1.12-1.38 (4H, m), 1.57-1.79 (2H, m), 2.33 (3H, s), 2.65 (2H, dq, J 7 Hz, 15 Hz). 3.64 (1 H, m), 4.05 (2H, q, J 7 Hz), 7.09 (1 H, dt J 2 Hz, 8 Hz), 7.44 (1 H, dd, J 2 Hz, 8 Hz), 7.54 (1 H, dd, J 8 Hz, 5 Hz).

### Intermediate 121 : 3-(6-Fluoro-3-methyl-1-benzothien-2-yl)-1-heptanol

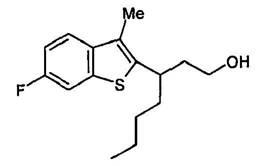

A solution of ethyl 3-(6-fluoro-3-methyl-1-benzothien-2-yl)heptanoate (180 mg, 0.56 mmol) in anhydrous THF (4 mL) was stirred at 0°C under nitrogen and treated dropwise with 1.5M diisobutylaluminium hydride in toluene (1 mL). The mixture was stirred at 0°C for 1 h and at 20° for 2 h. Sufficient water was carefully added to the mixture to destroy excess reagent, and then diluted with EtOAc (25 mL) and 1 M hydrochloric acid (25 mL). The phases were separated and the aqueous layer extracted with EtOAc (2 x 25 mL). The combined organic extracts were washed with water (25 mL) and brine (25 mL), dried over MgSO₄ and evaporated to give an oil. This was eluted through a 20 g SPE silica cartridge with EtOAc : cyclohexane (1 4) to afford the title compound as a colourless oil (121 mg). ¹H NMR (400 MHz; CDCl₃) δ: 0.84 (3H, t, J 7 Hz), 1.15-1.37 (5H, m), 1.58-1.88 (3H, m), 2.01 (1H, m), 2.32 (3H, s), 3.30 (1H, m). 3.50 (1 H, m), 3.63 (1 H, m), 7.09 (1 H, dt J 2 Hz, 8 Hz), 7.45 (1 H, dd, J 2 Hz, 8 Hz), 7.54 (1 H, dd, J 8 Hz, 5 Hz).

### Intermediate 122 : 2-[1-(2-Bromoethyl)pentyl]-6-fluoro-3-methyl-1-benzothiophene

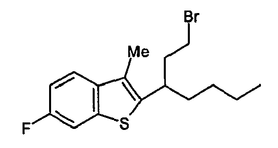

A solution of 3-(6-fluoro-3-methyl-1-benzothien-2-yl)-1-heptanol (60 mg, 0.21 mmol) in DCM (5 mL) was treated with triphenylphosphine (100 mg, 0.37 mmol) and carbon tetrabromide (120 mg, 0.37 mmol). The mixture was stirred at 20°C under nitrogen overnight, and then evaporated to dryness. The residue was dissolved in a little DCM and eluted through a 5 g SPE silica cartridge with cyclohexane to afford the title compound as a colourless oil (62 mg). ¹H NMR (400 MHz; CDCl₃) δ: 0.85 (3H, t, J 7 Hz), 1.16-1.36 (3H, m), 1.59-1.77 (2H, m), 2.09-2.29 (2H, m), 2.37 (3H, s), 3.18 (1 H, m). 3.34-3.44 (2H, m), 3.63 (1H, m), 7.10 (1H, dt J 2 Hz, 8 Hz), 7.45 (1H, dd, J 2 Hz, 8 Hz), 7.55 (1 H, dd, J 8 Hz, 5 Hz).

### Intermediate 123 : Ethyl [(4-{[3-(6-fluoro-3-methyl-1-benzothien-2-yl)heptyl]thio}-2-methylphenyl)oxy]acetate

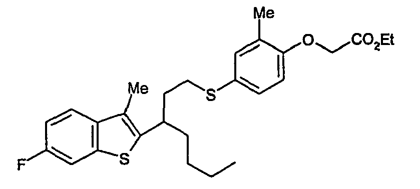

A solution of 2-[1-(2-bromoethyl)pentyl]-6-fluoro-3-methyl-1-benzothiophene (61 mg, 0.18 mmol) in anhydrous MeCN (2 mL) was treated with cesium carbonate (100 mg) and a solution of ethyl [(4-mercapto-2-methylphenyl)oxy]acetate (50 mg, 0.22 mmol) in anhydrous MeCN (2 mL). The mixture was stirred at 20°C for 3 h, and then diluted with water (25 mL), and extracted with EtOAc (2 x 20 mL). The combined organic extracts were washed with brine (20 mL), dried over MgSO₄, and evaporated to dryness to afford the title compound as an oil (56 mg). LC/MS: m/z489 [M+H]⁺, Rₜ 4.50 min.

### Intermediate 124 : Ethyl [(4-{[3-(6-fluoro-3-methyl-1-benzothien-2-yl)heptyl]oxy}-2-methylphenyl)oxy]acetate

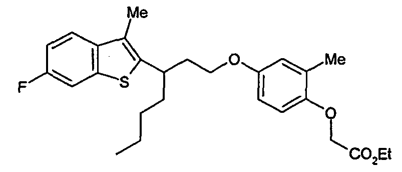

A solution of 3-(6-fluoro-3-methyl-1-benzothien-2-yl)-1-heptanol (56 mg, 0.2 mmol) in anhydrous THF (5 mL) was cooled to 0°C and treated with ethyl [(4-hydroxy-2-methylphenyl)oxy]acetate (50 mg, 0.24 mmol) and 1,1'-(azodicarbonyl)dipiperidine (110 mg, 0.4 mmol). The mixture was stirred for 5 min, then tri-n-butylphosphine (81 mg, 0.4 mmol) was added and stirred at 20°C under nitrogen overnight. The reaction mixture was diluted with water (40 mL) and extracted with EtOAc (2 x 25 mL). The organic extracts were combined, washed with water (25 mL) and brine (25 mL), dried over MgSO₄ and evaporated to dryness. The residue was eluted through a 20 g SPE silica cartridge with EtOAc : cyclohexane (1 : 20) to afford the title compound as an oil (60 mg). ¹H NMR (400 MHz; CDCl₃) δ: 0.85 (3H, t, J 7 Hz), 1.19-1.40 (4H, m), 1.28 (3H, t, J 7 Hz), 1.59-1.81 (2H, m), 1.92 (1 H, m), 2.21 (1 H, m), 2.22 (3H, s), 2.23 (3H, s), 3.42 (1 H, m), 3.63 (1 H, m), 3.86 (1 H, m), 4.24 (2H, q, J 7 Hz), 4.55 (2H, s), 6.53 (1 H, dd, J 8 Hz, 3 Hz), 6.60 (1 H, d, J 8 Hz), 6.65 (1 H, J 3 Hz), 7.08 (1 H, dt J 2 Hz, 8 Hz), 7.45 (1 H, dd, J 2 Hz, 8 Hz), 7.50 (1 H, dd, J 8 Hz, 5 Hz).

### Intermediate 125 : N,3,6-Trimethyl-N-(methyloxy)-1-benzofuran-2-carboxamide

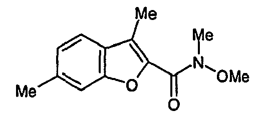

A suspension of 3,6-dimethyl-benzofuran-2-carboxylic acid (2.1 g, 11 mmol) in DCM (201 mL) was treated with carbonyldiimidazole (2.50 g, 15.2 mmol) and stirred at 20°C under nitrogen for 1.5 h. A solution of N,O-dimethylhydroxylamine, generated from the hydrochloride salt (3.2 g, 33 mmol) by partitioning between aqueous sodium carbonate solution and DCM, was added portionwise and the mixture was stirred at 20°C for 3 days. The DCM solution was then washed with aqueous sodium bicarbonate solution (2 x 200 mL), and brine (200 mL), dried by filtration through a hydrophobic frit and evaporated. The residue was eluted through a 50 g SPE silica cartridge with EtOAc : cyclohexane (1 : 10) to afford the title compound, which was isolated as a white solid (2.01 g). LC/MS: m/z 234 [M+H]⁺, Rₜ 3.12 min.

### Intermediate 126 : 1-(3,6-Dimethyl-1-benzofuran-2-yl)-1-pentanone

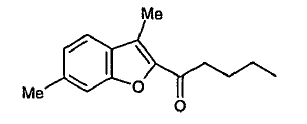

A solution of *N*,3,6-trimethyl-*N*-(methyloxy)-1-benzofuran-2-carboxamide (2.00 g, 8.6 mmol) in anhydrous THF (50 mL) was stirred under nitrogen at -70°C and treated with 2M butylmagnesium chloride in THF (10 mL, 20 mmol). The mixture was stirred at -70°C for 1 h, and then at 20°C overnight. The mixture was then diluted with aqueous ammonium chloride (50 mL), diluted with water (400 mL) and extracted with DCM (2 x 200 mL). The combined organic extracts were washed with water (200 mL), filtered through a hydrophobic frit and evaporated to dryness. The residue was eluted through a 50 g SPE silica cartridge with EtOAc : cyclohexane (1 : 10) to afford the title compound as a white solid (1,90 g). LC/MS: m/z 231 [M+H]⁺, Rₜ 3.93 min.

### Intermediate 127: Ethyl 3-(3,6-dimethyl-1-benzofuran-2-yl)-2-heptenoate

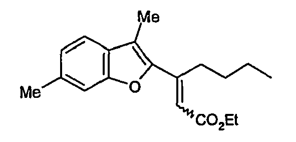

A solution of triethyl phosphonoacetate (3.17 g, 13.8 mmol) in anhydrous THF (50 mL) was stirred at 20°C under nitrogen and treated with sodium hydride (60% dispersion in oil, 550 mg, 13.8 mmol). After 10 min a solution of 1-(3,6-dimethyl-1-benzofuran-2-yl)-1-pentanone (1.90 g, 8.3 mmol) in anhydrous THF (25 mL) was added, and the mixture was stirred at 20°C for 7 days. The mixture was diluted with water (600 mL) and extracted with DCM (3 x 200 mL). The organic extracts were combined, washed with water (300 mL), filtered through a hydrophobic frit, and evaporated to dryness. The residue was eluted through a 90 g Biotage^{™} silica cartridge with EtOAc : cyclohexane (1 : 19) to afford a mixture of isomers of the title compound as an oil (2.35 g). LC/MS: m/z 301 [M+H]⁺, Rₜ 4.09 min.

### Intermediate 128 : Ethyl 3-(3,6-dimethyl-1-benzofuran-2-yl)heptanoate

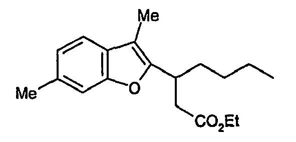

A solution of ethyl 3-(3,6-dimethyl-1-benzofuran-2-yl)-2-heptenoate (2.35 g, 7.8 mmol) in EtOAc (150 mL) was added to 10% palladium on carbon (wetted, 750 mg), and the mixture was stirred under a hydrogen atmosphere at 20°C overnight. The mixture was filtered through a Celite pad, washing through with EtOAc. The filtrate and washings were evaporated to dryness to afford the title compound as a colourless oil (2.35 g). LC/MS: m/z 303 [M+H]⁺, Rₜ 4.12 min.

### Intermediate 129 : 3-(3,6-Dimethyl-1-benzofuran-2-yl)-1-heptanol

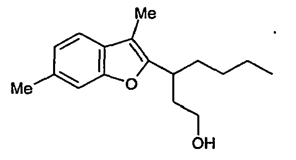

A solution of ethyl 3-(3,6-dimethyl-1-benzofuran-2-yl)heptanoate (2.35 g, 7.78 mmol) in anhydrous THF (60 mL) was stirred at 0°C under nitrogen and treated dropwise with 1.5M diisobutylaluminium hydride in toluene (16 mL). The mixture was stirred at 0°C for 1 h and at 20° for 2 h. More 1.5M diisobutylaluminium hydride in toluene (8 mL) was then added and the mixture was stirred at 20° for a further 1 h. Sufficient water was carefully added to the mixture to destroy excess reagent, and then diluted with EtOAc (200 mL) and 1 M hydrochloric acid (500 mL). The phases were separated and the aqueous layer extracted with EtOAc (2 x 200 mL). The combined organic extracts were washed with water (200 mL) and brine (200 mL), dried over MgSO₄ and evaporated to give an oil. This was eluted through a 90 g Biotage^{™} silica cartridge with EtOAc : cyclohexane (1 : 4) to afford the title compound as a colourless oil which solidified on standing (1.95 g). LC/MS: m/z 261 [M+H]⁺, Rₜ 3.77 min.

### Intermediate 130: 2-[1-(2-Bromoethyl)pentyl]-3,6-dimethyl-1-benzofuran

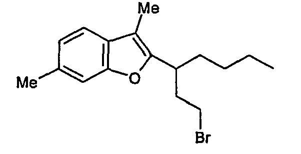

A solution of 3-(3,6-dimethyl-1-benzofuran-2-yl)-1-heptanol (1.95 g, 7.5 mmol) in DCM (90 mL) was treated with triphenylphosphine (3 g, 11.4 mmol) and carbon tetrabromide (3.75 g, 11.4 mmol). The mixture was stirred at 20°C under nitrogen overnight, and then evaporated to dryness. The residue was eluted through a 90 g Biotage^{™} silica cartridge with cyclohexane to afford the title compound as a colourless oil which solidified on standing (1.65 g). ¹H NMR (400 MHz; CDCl₃) δ: 0.85 (3H, t, J 7 Hz), 1.10-1.38 (4H, m), 1.64 (1 H, m), 1.80 (1 H, m), 2.14 (1 H, m), 2.20 (3H, s), 2.36 (1H. m), 2.47 (3H, s), 3.08-3.18 (2H, m). 3.39 (1 H, m), 7.06 (1 H, d J 8 Hz), 7.20 (1 H, s), 7.33 (1 H, d, J 8 Hz).

### Intermediate 131 : Ethyl 3-methyl-6-(trifluoromethyl)-1-benzofuran-2-carboxylate

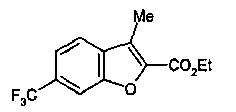

A solution of 1-[2-hydroxy-4-(trifluoromethyl)phenyl]ethanone (2.32 g, 11.4 mmol) in anhydrous DMF (35 mL) was treated with potassium carbonate (3.93 g, 28.5 mmol) ethyl bromoacetate (1.45 mL, 12.1 mmol) and stirred at 20°C under nitrogen for 4 h. The reaction mixture was evaporated to dryness. The residue was dissolved in water (200 mL), and acidifed (2M hydrochloric acid, 18 mL) and the gelatinous white precipitate filtered. The solid was washed with water and dried *in vacuo* at 50°C to afford the title compound (1.28 g). LC/MS: m/z 273 [M+H]⁺, Rₜ 3.63 min.

### Intermediate 132 : 3-Methyl-6-(trifluoromethyl)-1-benzofuran-2-carboxylic acid

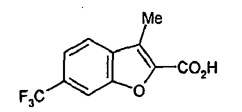

A solution ethyl 3-methyl-6-(trifluoromethyl)-1-benzofuran-2-carboxylate (1.66g, 6.1 mmol) in MeOH (80 mL) was treated with 2M aqueous sodium hydroxide (12.9 mL) and stirred under reflux for 2 h. The reaciton mixture was cooled and evaporated to dryness. The residue was dissolved in water (200 mL), and acidifed (2M hydrochloric acid, 18 mL) and the gelatinous white precipitate filtered. The solid was washed with water and dried *in vacuo* at 50°C to afford the title compound (1.28 g). LC/MS: m/z 243 [M-H]⁻, Rₜ 3.72 min.

### Intermediate 133 : N,3-Dimethyl-N-(methyloxy)-6-(trifluoromethyl)-1-benzofuran-2-carboxamide

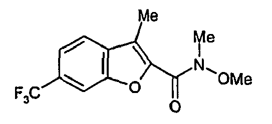

A suspension of 3-methyl-6-(trifluoromethyl)-1-benzofuran-2-carboxylic acid (1.28 g, 5.2 mmol) in DCM (100 mL) was treated with carbonyldiimidazole (1.25 g, 7.6 mmol) and stirred at 20°C under nitrogen for 1.5 h. A solution of N,O-bismethylhydroxylamine, generated from the hydrochloride salt (1.6 g, 17mmol) by partitioning between aqueous sodium carbonate solution and DCM, was added portionwise and the mixture was stirred at 20°C for 3 days. The DCM solution was then washed with aqueous sodium bicarbonate solution (2 x 200 mL), and brine (200 mL), dried by filtration through a hydrophobic frit and evaporated. The residue was eluted through a 50 g SPE silica cartridge with EtOAc : cyclohexane (1 : 10) to afford the title compound, which was isolated as a white solid (0.93 g). LC/MS: m/z 288 [M+H]⁺, Rₜ 3.30 min.

### Intermediate 134 : 1-[3-Methyl-6-(trifluoromethyl)-1-benzofuran-2-yl]-1-pentanone

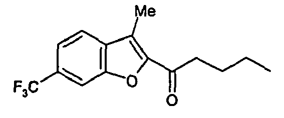

A solution of N,3-dimethyl-N-(methyloxy)-6-(trifluoromethyl)-1-benzofuran-2-carboxamide (0.92 g, 3.2 mmol) in anhydrous THF (25 mL) was stirred under nitrogen at -70°C and treated with 2M butylmagnesium chloride in THF (4 mL, 8 mmol). The mixture was stirred at -70°C for 1 h, and then at 20°C overnight. The mixture was then diluted with aqueous ammonium chloride (50 mL), diluted with water (200 mL) and extracted with DCM (2 x 100 mL). The combined organic extracts were washed with water (100 mL), filtered through a hydrophobic frit and evaporated to dryness. The residue was eluted through a 50 g SPE silica cartridge with EtOAc : cyclohexane (1 : 10) to afford the title compound as a white solid (0.81 g). LC/MS: m/z 285 [M+H]⁺, Rₜ 4.00 min.

### Intermediate 135 : Ethyl 3-[3-methyl-6-(trifluoromethyl)-1-benzofuran-2-yl]-2-heptenoate

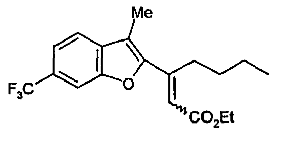

A solution of triethyl phosphonoacetate (1.1 g, 4.75 mmol) in anhydrous THF (15 mL) was stirred at 20°C under nitrogen and treated with sodium hydride (60% dispersion in oil, 190 mg, 4.75 mmol). After 10 min a solution of 1-[3-methyl-6-(trifluoromethyl)-1-benzofuran-2-yl]-1-pentanone (0.81 g, 8.3 mmol) in anhydrous THF (10 mL) was added, and the mixture was stirred at 20°C overnight. The mixture was diluted with water (200 mL) and extracted with DCM (3 x 100 mL). The organic extracts were combined, washed with water (150 mL), filtered through a hydrophobic frit, and evaporated to dryness. The residue was eluted through a 90 g Biotage^{™} silica cartridge with EtOAc: cyclohexane (1 : 19) to afford a mixture of isomers of the title compound as an oil (0.98 g). LC/MS: m/z 355 [M+H]⁺, Rₜ4.17 min, 4.36 min.

### Intermediate 136 : Ethyl 3-[3-methyl-6-(trifluoromethyl)-1-benzofuran-2-yl]heptanoate

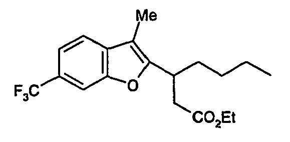

A solution of ethyl 3-[3-methyl-6-(trifluoromethyl)-benzofuran-2-yl]-2-heptenoate (0.98 g, 2.8 mmol) in EtOAc (50 mL) was added to 10% palladium on carbon (wetted, 250 mg), and the mixture was stirred under a hydrogen atmosphere at 20°C overnight. The mixture was filtered through a Celite pad, washing through with EtOAc. The filtrate and washings were evaporated to dryness to afford the title compound as a colourless oil (0.96 g). LC/MS: m/z 357 [M+H]⁺, Rₜ4.05 min.

### Intermediate 137 : 3-[3-Methyl-6-(trifluoromethyl)-1-benzofuran-2-yl]-1-heptanol

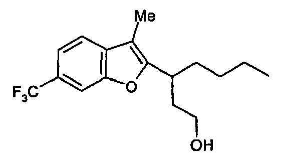

A solution of ethyl 3-[3-methyl-6-(trifluoromethyl)-1-benzofuran-2-yl]heptanoate (0.96 g, 2.7 mmol) in anhydrous THF (20 mL) was stirred at 0°C under nitrogen and treated dropwise with 1.5M diisobutylaluminium hydride in toluene (5.3 mL). The mixture was stirred at 0°C for 1 h and at 20° for 2 h. More 1.5M diisobutylaluminium hydride in toluene (2.6 mL) was then added and the mixture was stirred at 20° for a further 1 h. Sufficient water was carefully added to the mixture to destroy excess reagent, and then diluted with EtOAc (75 mL) and 1 M hydrochloric acid (150 mL). The phases were separated and the aqueous layer extracted with EtOAc (2 x 75 mL). The combined organic extracts were washed with water (75 mL) and brine (75 mL), dried over MgSO₄ and evaporated to give an oil. This was eluted through a 90 g Biotage^{™} silica cartridge with EtOAc: cyclohexane (1 : 4) to afford the title compound as a colourless oil (0.75 g). LC/MS: m/z 315 [M+H]⁺, Rₜ3.97 min.

### Intermediate 138 : 2-[1-(2-Bromoethyl)pentyl]-3-methyl-6-(trifluoromethyl)-1-benzofuran

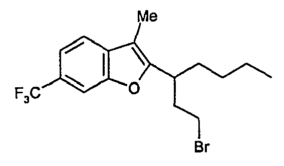

A solution of 3-[3-methyl-6-(trifluoromethyl)-1-benzofuran-2-yl]-1-heptanol (0.75 g, 2.4 mmol) in DCM (30 mL) was treated with triphenylphosphine (1 g, 3.8 mmol) and carbon tetrabromide (1.25 g, 3.8 mmol). The mixture was stirred at 20°C under nitrogen overnight, and then evaporated to dryness. The residue was eluted through a 40 g Biotage^{™} silica cartridge with cyclohexane to afford the title compound as a colourless oil (0.85 g). ¹H NMR (400 MHz; CDCl₃) δ 0.85 (3H, t, J 7 Hz), 1.07-1.37 (4H, m), 1.68 (1 H, m), 1.80 (1 H, m), 1.91-2.07 (2H, m), 2.21 (3H, s), 3.13 (1H. m), 3.45 (1 H, m), 3.61 (1 H, m), 7.50 (2H, q J 8 Hz), 7.67 (1 H, s).

### Intermediate 139 : Ethyl (2-{[3-(3,6-dimethyl-1-benzofuran-2-yl)heptyl]thio}-4-methyl-1,3-thiazol-5-yl)acetate

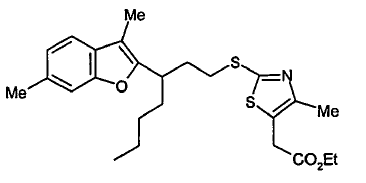

A solution of 2-[1-(2-bromoethyl)pentyl]-3,6-dimethyl-1-benzofuran (65 mg, 0.2 mmol) in anhydrous MeCN (3 mL) was treated with cesium carbonate (200 mg) and a solution of ethyl (2-mercapto-4-methyl-1,3-thiazol-5-yl)acetate (88 mg, 0.4 mmol) in anhydrous MeCN (5 mL). The mixture was stirred at 20°C under nitrogen overnight, and then evaporated to dryness and eluted through a 10 g SPE silica cartridge with EtOAc : cyclohexane (1 : 20) to afford the title compound as an oil (57 mg). LC/MS: m/z 460 [M+H]⁺, Rₜ 4.45 min.

### Intermediate 140 : Ethyl [(4-{[3-(3,6-dimethyl-1-benzofuran-2-yl)heptyl]oxy}-2-methylphenyl)oxy]acetate

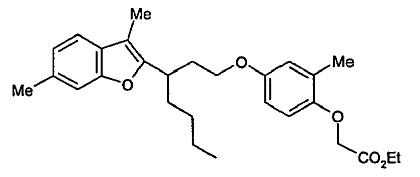

A solution of 2-[1-(2-bromoethyl)pentyl]-3,6-dimethyl-1-benzofuran (65 mg, 0.2 mmol) in anhydrous MeCN (3 mL) was treated with cesium carbonate (200 mg) and a solution of ethyl [(4-hydroxy-2-methylphenyl)oxy]acetate (85 mg, 0.4 mmol) in anhydrous MeCN (5 mL). The mixture was stirred at 20°C under nitrogen for 4 days, and then evaporated to dryness and eluted through a 10 g SPE silica cartridge with EtOAc : cyclohexane (1 : 20) to afford the title compound as a gum (54 mg). ¹H NMR (400 MHz; CDCl₃) δ: 0.84 (3H, t, J 7 Hz), 1.13-1.35 (7H, m), 1.68 (1 H, m), 1.82 (1 H, m), 2.06 (3H, s), 2.12 (2H, m) 2.23 (3H, s), 2.46 (3H, s), 3.17 (1 H, m), 3.61 (1 H, m), 3.82 (1 H, m), 4.25 (2H, q, J 7 Hz), 4.56 (2H, s), 6.54 (1 H, m), 6.61 (1 H, m), 6.65 (1 H, m), 7.03 (1 H, m), 7.21 (1 H, s), 7.29 (1 H, m).

### Intermediate 141 : Ethyl [(4-([3-(3,6-dimethyl-1-benzofuran-2-yl)heptyl]thio}-2-methylphenyl)oxy]acetate

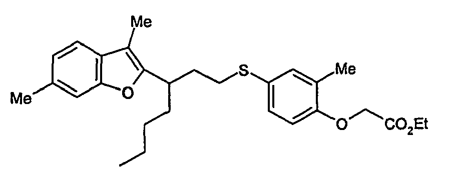

A solution of 2-[1-(2-bromoethyl)pentyl]-3,6-dimethyl-1-benzofuran (65 mg, 0.2 mmol) in anhydrous MeCN (3 mL) was treated with cesium carbonate (200 mg) and a solution of ethyl [(4-mercapto-2-methylphenyl)oxy]acetate (90 mg, 0.4 mmol) in anhydrous MeCN (5 mL). The mixture was stirred at 20°C under nitrogen overnight, and then evaporated to dryness and eluted through a 10 g SPE silica cartridge with EtOAc : cyclohexane (1 : 20) to afford the title compound as an oil (83 mg). LC/MS: m/z 469 [M+H]⁺, Rₜ 4.61 min.

### Intermediate 142 : Ethyl [4-methyl-2-({3-[3-methyl-6-(trifluoromethyl)-1-benzofuran-2-yl]heptyl}thio)-1,3-thiazol-5-yl]acetate

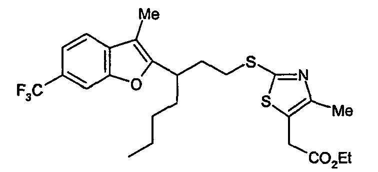

A solution of 2-[1-(2-bromoethyl)pentyl]-3-methyl-6-(trifluoromethyl)-1-benzofuran (75 mg, 0.2 mmol) in anhydrous MeCN (3 mL) was treated with cesium carbonate (200 mg) and a solution of ethyl (2-mercapto-4-methyl-1,3-thiazol-5-yl)acetate (88 mg, 0.4 mmol) in anhydrous MeCN (5 mL). The mixture was stirred at 20°C under nitrogen overnight, and then evaporated to dryness and eluted through a 10g SPE silica cartridge with EtOAc : cyclohexane (1 : 20) to afford the title compound as an oil (67 mg). LC/MS: m/z 514 [M+H]⁺, Rₜ 4.49 min.

### Intermediate 143 : Ethyl {[2-methyl-4-({3-[3-methyl-6-(trifluoromethyl)-1-benzofuran-2-yl]heptyl}oxy)phenyl]oxy}acetate

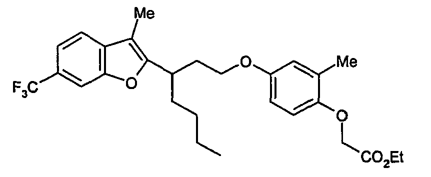

A solution of 2-[1-(2-bromoethyl)pentyl]-3-methyl-6-(trifluoromethyl)-1-benzofuran (75 mg, 0.2 mmol) in anhydrous MeCN (3 mL) was treated with cesium carbonate (200 mg) and a solution of ethyl [(4-hydroxy-2-methylphenyl)oxy]acetate (85 mg, 0.4 mmol) in anhydrous MeCN (5 mL). The mixture was stirred at 20°C under nitrogen for 4 days, and then evaporated to dryness and eluted through a 10 g SPE silica cartridge with EtOAc : cyclohexane (1 : 20) to afford the title compound as a gum (64 mg). ¹H NMR (400 MHz; CDCl₃) δ: 0.85 (3H, t, J 7 Hz), 1.10-1.37 (7H, m), 1.71 (1H. m), 1.84 (1H. m), 2.10 (3H, s), 2.15 (2H, m) 2.24 (3H, s), 3.25 (1 H, m), 3.58 (1 H, m), 3.84 (1 H, m), 4.25 (2H, q, J 7 Hz), 4.56 (2H, s), 6.53 (1 H, m), 6.60 (1 H, m), 6.63 (1 H, m), 7.48 (2H, m), 7.67 (1 H, s).

### Intermediate 144 : Ethyl {[2-methyl-4-({3-[3-methyl-6-(trifluoromethyl)-1-benzofuran-2-yl]heptyl}thio)phenyl]oxy}acetate

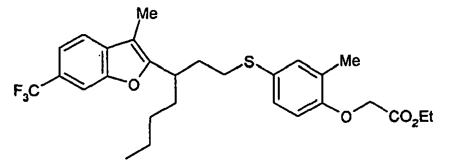

A solution of 2-[1-(2-bromoethyl)pentyl]-3-methyl-6-(trifluoromethyl)-1-benzofuran (75 mg, 0.2 mmol) in anhydrous MeCN (3 mL) was treated with cesium carbonate (200 mg) and a solution of ethyl [(4-mercapto-2-methylphenyl)oxy]acetate (90 mg, 0.4 mmol) in anhydrous MeCN (5 mL). The mixture was stirred at 20°C under nitrogen overnight, and then evaporated to dryness and eluted through a 10 g SPE silica cartridge with EtOAc : cyclohexane (1 : 20) to afford the title compound as an oil (76 mg). ¹H NMR (400 MHz; CDCl₃) δ: 0.83 (3H, t, J 7 Hz), 1.06-1.34 (7H, m), 1.62 (1 H, m), 1.76 (1 H, m), 1.90 (1 H, m), 2.08 (1 H, m), 2.20 (3H, s), 2.24 (3H, s), 2.58 (1 H, m), 2.75 (1 H, m), 3.12 (1 H, m), 4.27 (2H, q, J 7 Hz), 4.61 (2H, s), 6.60 (1 H, d J 8Hz), 7.09 (1 H, d, J 8 Hz, 2 Hz), 7.13 (1H. d, J 2 Hz), 7.50 (2H, q, J 8 Hz), 7.65 (1 H, s).

### Intermediate 145 : Methyl 3-methyl-6-(methyloxy)-1-benzothiophene-2-carboxytate

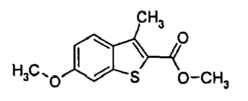

Methyl thioglycolate (5.32 mL, 59.5 mmol) was added dropwise to a suspension of 60% sodium hydride in mineral oil (3.87 g, 96.6 mmol) in dry THF (20 mL) and dry DMSO (80 mL). The suspension was stirred under nitrogen for 10 mins, then a solution of 2'-fluoro-4'-methoxyacetophenone in dry DMSO (20 mL) was added dropwise over 3-4 minutes. The solution turned orange and was stirred at room temperature for 4 hours and then at 50°C for 18 hours. The solution was diluted with water (1000 mL) and extracted with EtOAc (3 x 200 mL). The combined organic layers were washed with a saturated solution of sodium bicarbonate (200 mL) and then with brine (200 mL). The organic was separated, concentrated at the rotoevaporator and coevaporated with chloroform, to give the title compound (8.26 g) as a brown solid. LC/MS: m/z 237 [M+H]⁺, Rₜ 3.58 min

### Intermediate 146: 3-Methyl-6-(methyloxy)-1-benzothiophene-2-carboxylic acid

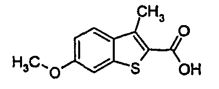

A solution of methyl 3-methyl-6-(methyloxy)-1-benzothiophene-2-carboxylate in MeOH (500 mL) was treated with 2M sodium hydroxide (60 mL) and stirred at 60°C for 18 hours. The solvent was evaporated, the residue was taken up in water (600 mL) and the pH was adjusted to 1 by adding 2M HCl. A precipitate was formed. This was isolated by filtration, washed with 2M HCl and dried under reduced pressure, at 50°C for 3 days. After triturating with DCM, the pure title compound was obtained as a light brown solid (4.47 g). ¹H NMR (400 MHz, DMSO-d₆) □: 13.5-12.9 (1 H), 7.82 (1H, d, J 8.9 Hz), 7.55 (1H, d, J 2.3 Hz), 7.07 (1H, dd, J 8.9, 2.3 Hz), 3.84 (3H, s), 2.66 (3H, s).

### Intermediate 147: N,3-Dimethyl-N,6-bis(methyloxy)-1-benzothiophene-2-carboxamide

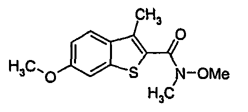

A suspension of 3-methyl-6-(methyloxy)-1-benzothiophene-2-carboxylic acid in dry DCM (820 mL) was treated with carbonyl-diimidazole (6.89 g, 42.5 mmol), and stirred under nitrogen, at room temperature for 3 hours. Meanwhile N,O-dimethylhydroxilamine hydrochloride (12.4 g, 0.127 mmol), was dissolved in DCM (150 mL) and then washed with a saturated solution of sodium carbonate (2 x 100 mL). The organic layer was dried over MgSO₄, filtered and then added to the solution of the acid. The reaction was left stirred under nitrogen, at room temperature for 7 days. The solution was then washed with 2M HCl (2 x200 mL), saturated sodium bicarbonate (2 x 200 mL) and brine. The organic was dried over MgSO₄, filtered and concentrated at the rotoevaporator to a brown solid (9.11 g). This was purified by Si-SPE (50 g cartridge), eluting with DCM (3 volumes), DCM/ether 9/1 (2), DCM/ether 8/2 (2), DCM/ether 1/1 (2) and MeOH (3). The relevant fractions were combined and concentrated to give the title compound (7.60 g) as a white solid. LC/MS: m/z 266 [M+H]⁺, Rₜ 3.04 min

### Intermediate 148 : 1-[3-Methyl-6-(methyloxy)-1-benzothien-2-yl]-1-pentanone

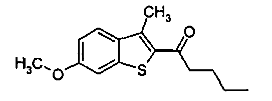

To a solution of *N*,3-dimethyl-*N*,6-bis(methyloxy)-1-benzothiophene-2-carboxamide (7.58 g, 28.6 mmol) in dry THF (160 mL), under nitrogen, at -70°C, 2M butylmagnesium chloride in THF(33.3 mL, 66.6 mmol) was added dropwise. The solution was stirred at -70°C for 1 hour and then for 18 hours at room temperature. The excess Grignard reagent was quenched by adding saturated ammonium chloride (500 mL) and the resulting mixture was extracted with ether (2 x 300 mL). The combined organic layers were washed with water, separated, dried over MgSO₄, filtered and concentrated at the rotoevaporator to yield the title compound as a yellow solid (7.20 g). ¹H NMR (400 MHz, CDCl₃) δ 7.74 (1 H, d, J 9.0 Hz), 7.26 (1 H, d, J 2.5 Hz), 7.05 (1 H, dd, J 9.0, 2.5 Hz), 3.90 (3H, s), 2.90 (2H, t, J 7.2 Hz), 2.73 (3H, s), 1.80-1.70 (2H, m), 1.49-1.37 (2H, m), 0.96 (3H, t, J 7.4 Hz).

### Intermediate 149 : Ethyl 3-[3-methyl-6-(methyloxy)-1-benzothien-2-yl]-2-heptenoate

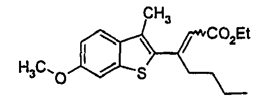

To a solution of triethylphosphonoacetate (7.20 mL, 36.3 mmol), under nitrogen and at 0°C, 60% sodium hydride in mineral oil was added (1.45 g, 36.3 mmol). The suspension was stirred for 15 minutes, then a solution of 1-[3-methyl-6-(methyloxy)-1-benzothien-2-yl]-1-pentanone (7.16 g, 27.3 mmol) in dry THF (75 mL) was added dropwise. The solution was stirred a room temperature for 18 hours. Water (200 mL) and saturated solution of sodium carbonate (50 mL) were added then the mixture was extracted with ether (2 x 200 mL). The combined organics were dried over MgSO₄, filtered and concentrated at the rotoevaporator to yield the crude product (9.46 g). This was purified by Si-SPE (50 g cartridge), eluting with DCM/cyclohexane 1/4 (2 volumes), DCM/cyclohexane 1/1 (2), DCM (2), ether (2) and MeOH (1). The relevant fractions were combined and concentrated to give the title compound (3.56 g) as a white solid, which was obtained as a mixture of geometrical isomers (and also some 3-heptenoate isomers). LC/MS: m/z 333 [M+H]⁺, Rₜ 3.87, 3.91, 3.96, 4.20 min.

### Intermediate 150 : Ethyl 3-[3-methyl-6-(methyloxy)-1-benzothien-2-yl]heptanoate

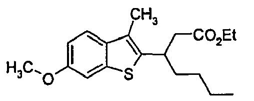

To a solution of ethyl 3-[3-methyl-6-(methyloxy)-1-benzothien-2-yl]-2-heptenoate (3.54 g, 10.6 mmol) in ethanol (200 mL), 10% palladium on carbon (3 g) was added. The reaction mixture was stirred under an atmosphere of hydrogen for 18 hours, then filtered through Celite and the filter was washed with ethanol (3x100 mL). The filtrate was concentrated and the residue was purified using a 2 x 90 g silica Biotage^{™} columns on a Quad 3 system; each column being eluted with DCM/cyclohexane 1/1. The fractions containing the title compound were combined and concentrated to give the title compound (1.70 g), contaminated with -25% of starting alkene. LC/MS: m/z 335 [M+H]⁺, Rₜ 3.97 min.

### Intermediate 151 : 3-[3-Methyl-6-(methyloxy)-1-benzothien-2-yl]heptanoic acid

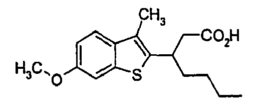

Potassium hydroxide (5.67 g, 101 mmol) in water (20 mL) was added dropwise to a solution of ethyl 3-[3-methyl-6-(methyloxy)-1-benzothien-2-yl]heptanoate (1.69 g, 5.05 mmol) in 1,4-dioxane (40 mL), at 0°C. The reaction was left to stir at room temperature for 18 hours, then the solvents were evaporated and replaced with water (20 mL) and MeOH (40 mL). The reaction was stirred for a further 18 hours. The pH was adjusted to 2 by adding 2M hydrochloric acid, the resulting mixture was extracted with ether (3 x 50 mL), the combined organic phases were dried over MgSO₄, filtered and evaporated. The title compound (1.56 g) was obtained as a white solid, contaminated with ~25% of the corresponding alkenyl carboxylic acid formed from the unreacted starting material of the previous synthetic step. ¹H NMR (400 MHz, CDCl₃) δ 7.49 (1 H, d, J 8.8 Hz), 7.26 (1 H, d, J 2.3 Hz), 6.98 (1 H, dd, J 8.8, 2.3 Hz), 3.86 (3H, s), 3.66-3.55 (1 H, m), 2.73-2.62 (2H, m), 2.30 (3H, s), 1.81-1.69 (1H, m), 1.69-1.56 (1H, m), 1.38-1.15 (4H, m), 0.84 (3H, t, J 7.0 Hz)

### Intermediate 152 : 3-[3-Methyl-6-(methyloxy)-1-benzothien-2-yl]-1-heptanol

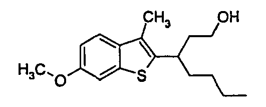

To a solution of 3-[3-methyl-6-(methyloxy)-1-benzothien-2-yl]heptanoic acid (4.48 g, 14.6 mmol) in dry THF (45 mL), at 0°C, under nitrogen, 1M borane in THF (30.7 mL, 30.7 mmol) was added dropwise. The reaction was left to stir for 3 hours then 2M hydrochloric acid was added (30 mL) and after a further 5 minutes stirring, water was added (30 mL) and the mixture was extracted with ether (2 x 70 mL). The combined organic phases wer dried over MgSO₄, filtered and evaporated to yield the product as a yellow oil. This was purified by Si-SPE (50 g cartridge), eluting with DCM/cyclohexane 1/1 (2 volumes), DCM (2), ether (2), EtOAc (2) and MeOH (2). The fractions containing the pure product were combined and concentrated to give 1.77 g of material. A further 2.34 g of material were isolated in not as pure form. These were purified again by Si-SPE (50 g cartridge), eluting with DCM (2 volumes), DCM/ether 9/1 (2), DCM/ether 8/2 (2), DCM/ether 1/1 (2), ether (2). The two combined batches of purified material gave 3.87 g of pure title compound. ¹H NMR (400 MHz, CDCl₃) δ: 7.49 (1 H, d, J 8.8 Hz), 7.27 (1 H, d, J 2.5 Hz), 6.98 (1 H, dd, J 8.8, 2.5 Hz), 3.86 (3H, s), 3.66-3.57 (1 H, m), 3.55-3.45 (1 H, m), 3.32-3.22 (1H. m), 2.29 (3H, s), 2.05-1.93 (1 H, m), 1.88-1.55 (3H, m), 1.36-1.15 (4H, m), 0.84 (3H, t, J 7.0 Hz)

### Intermediates 153 and 154 : (3R)-3-[3-methyl-6-(methyloxy)-1-benzothien-2-yl]-1-heptanol and (3S)-3-[3-methyl-6-(methyloxy)-1-benzothien-2-yl]-1-heptanol

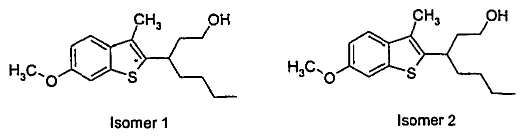

The resolution of 3-(6-methoxy-3-methyl-1-benzothien-2-yl)heptan-1-ol (2.95 g) was performed by chiral HPLC. Column: 2" x 20 cm Chiralpak AD (self packed) eluting with 2% EtOH/heptane (f = 60 mL/min, detection: UV 230 nm) to give two fractions Rₜ (mins) = 14.19 (isomer 1), 18.41 (isomer 2)

### Intermediates 155 and 156 : (3R)-3-[3-methyl-6-(trifluoromethyl)-1-benzothien-2-yl]-1-heptanol and (3S)-3-[3-methyl-6-(trifluoromethyl)-1-benzothien-2-yl]heptan-1-ol

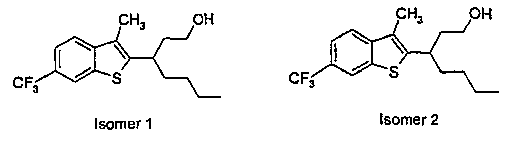

The resolution of 3-[3-methyl-6-(trifluoromethyl)-l-benzothien-2-yl]heptan-1-ol (4.33g) was performed by chiral HPLC. Column; 2"x 20 cm Chiralpak AS (self packed) eluting with 3% EtOH/heptane (f = 75 mL/min, detection: UV 230 nm) to give two fractions Rₜ (mins) = 7.00 (isomer 1), 11.5 (isomer 2)

### Intermediate 157 : 2-[1-(2-Bromoethyl)pentyl]-3-methyl-6-(methyloxy)-1-benzothiophene (isomer 1)

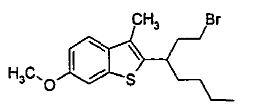

A solution of 3-(6-methoxy-3-methyl-1-benzothien-2-yl)heptan-1-ol (isomer 1) (100 mg, 0.342 mmol) in dry DCM (4 mL) was treated with triphenylphosphine (135 mg, 0.513 mmol) and carbon tetrabromide (170 mg, 0.513 mmol). The solution was stirred overnight at room temperature then was concentrated *in vacuo.* The residue was purified by Si-SPE (2 g cartridge), eluting with DCM/cyclohexane 1/1 (3 volumes), DCM (2) and MeOH (1). The fractions containing the pure product were combined and concentrated to give the title compound (121 mg) as a white solid. LC/MS: m/z 355, 357 [M+H]⁺, Rₜ 4.31 min.

### Intermediate 158 : 2-[1-(2-Bromoethyl)pentyl]-3-methyl-6-(methyloxy)-1-benzothiophene (isomer 2)

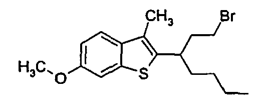

This compound was prepared from 3-(6-methoxy-3-methyl-1-benzothien-2-yl)heptan-1-ol (isomer 2) by the same procedure used for Intermediate 157. LC/MS: m/z 355, 357 [M+H]⁺, Rₜ 4.31 min.

### Intermediate 159 : 2-[1-(2-Bromoethyl)pentyl]-3-methyl-6-(trifluoromethyl)-1-benzothiophene (isomer 1)

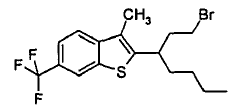

This compound was prepared from 3-[3-methyl-6-(trifluoromethyl)-1-benzothien-2-yl]-1-heptanol (isomer 1) by the same procedure used for Intermediate 157. ¹H NMR (400 MHz, CDCl₃) δ: 8.05 (1 H, s), 7.73 (1 H, d, J 8.3 Hz), 7.60 (1 H, dd, J 8.3, 1.0 Hz), 3.53-3.36 (2H, m), 3.23-3.11 (1 H, m), 2.43 (3H, s), 2.33-2.11 (2H, m), 1.84-1.58 (2H, m), 1.39-1.11 (4H, m), 0.85 (3H, t, J 7.2 Hz).

### Intermediate 160 : 2-[1-(2-Bromoethyl)pentyl]-3-methyl-6-(trifluoromethyl)-1-benzothiophene (isomer 2)

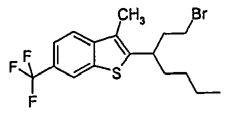

This compound was prepared 3-[3-methyl-6-(trifluoromethyl)-1-benzothien-2-yl]-1-heptanol (isomer 2) by the same procedure used for Intermediate 157. ¹H NMR (400 MHz, CDCl₃) δ 8.05 (1 H, s), 7.73 (1 H, d, J 8.3 Hz), 7.60 (1 H, dd, J 8.3, 1.0 Hz), 3.53-3.36 (2H, m), 3.23-3.11 (1H. m), 2.43 (3H, s), 2.33-2.11 (2H, m), 1.84-1.58 (2H, m), 1.39-1.11 (4H, m), 0.85 (3H, t, J 7.2 Hz).

### Intermediate 161 : Ethyl [2-({3-[3-methyl-6-(methyloxy)-1-benzothien-2-yl]heptyl}thio)-1,3-thiazol-4-yl]acetate (isomer 1)

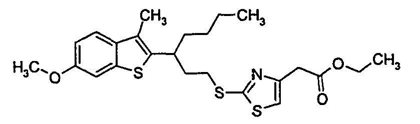

A mixture of 2-[1-(2-bromoethyl)pentyl]-3-methyl-6-(methyloxy)-1-benzothiophene (isomer 1) (71 mg, 0.20 mmol), ethyl (2-mercapto-1,3-thioazol-4-yl)acetate (51 mg, 0.25 mmol), and cesium carbonate (137 mg, 0.42 mmol), in dry MeCN (3 mL) was stirred at room temperature for 2 days. The solvent was evaporated, the residue triturated with DCM/cyclohexane 1/1 and filtered. The organic solution was then applied to a Si-SPE cartridge (1 g) and eluted with cyclohexane/DCM 1/1 (2 volumes), DCM (2), and ether (2). The relevant fractions were combined and evaporated to give the product (69 mg) as a colourless gum. LC/MS: m/z 478 [M+H]⁺, Rₜ 4.28 min.

### Intermediate 162 Ethyl [2-({3-[3-methyl-6-(methyloxy)-1-benzothien-2-yl]heptyl)thio)-1,3-thiazol-4-yl]acetate (isomer 2)

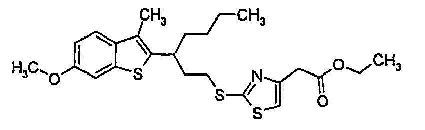

This compound was prepared 2-[1-(2-bromoethyl)pentyl]-3-methyl-6-(methyloxy)-1-benzothiophene (isomer 2) by the same procedure used Intermediate 161. LC/MS: m/z 478 [M+H]⁺, Rₜ 4.28 min.

### Intermediate 163 : Ethyl {[2-methyl-4-({3-[3-methyl-6-(methyloxy)-1-benzothien-2-yl]heptyl}oxy)phenyl]oxy}acetate (isomer 1)

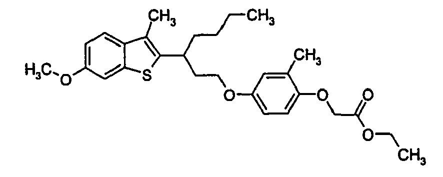

A mixture of 2-[1-(2-bromoethyl)pentyl]-3-methyl-6-(methyloxy)-1-benzothiophene (isomer 1) (71 mg, 0.20 mmol), ethyl [(4-hydroxy-2-methylphenyl)oxy]acetate (53 mg, 0.25 mmol), and cesium carbonate (137 mg, 0.42 mmol), in dry MeCN (3 mL) was stirred at room temperature for 5 days and for 1 day at 50°C. The solvent was evaporated, the residue triturated with DCM/cyclohexane 1/1 and filtered. The organic solution was then applied to a Si-SPE cartridge (1 g) and eluted with cyclohexane/DCM 1/1 (2 volumes), DCM (2), and ether (2). The relevant fractions were combined and evaporated to give the product (67 mg) as a colourless gum. LC/MS: m/z 502 [M+NH₄]⁺, Rₜ 4.34 min.

### Intermediate 164 : Ethyl (4-{[3-(6-methoxy-3-methyl-1-benzothien-2-yl)heptyl]oxy}-2-methylphenoxy)acetate (isomer 2)

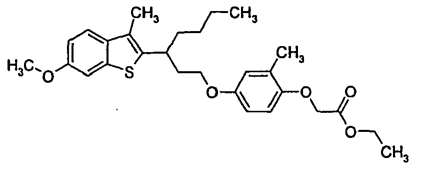

This compound was prepared from 2-[1-(2-bromoethyl)pentyl]-3-methyl-6-(methyloxy)-1-benzothiophene (isomer 2) by the same procedure used for Intermediate163. LC/MS: m/z 502 [M+NH₄]⁺, Rₜ 4.34 min.

### Intermediate 165 : Ethyl [4-({3-[3-methyl-6-(methyloxy)-1-benzothien-2-yl]heptyl}oxy)-3-(methyloxy)phenyl]acetate (isomer 1)

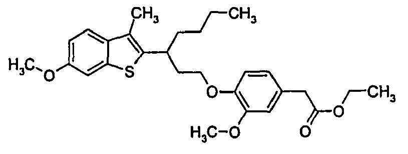

A mixture of 2-[1-(2-bromoethyl)pentyl]-3-methyl-6-(methyloxy)-1-benzothiophene (isomer 1) (71 mg, 0.20 mmol), ethyl [4-hydroxy-3-(methyloxy)phenyl]acetate (53 mg, 0.25 mmol), and cesium carbonate (137 mg, 0.42 mmol), in dry MeCN (3 mL) was stirred at room temperature for 5 days and for 1 day at 50°C. The solvent was evaporated, the residue triturated with DCM/cyclohexane 9/1 and filtered. The organic solution was then applied to a Si-SPE cartridge (1 g) and eluted with cyclohexane/DCM 9/1 (2 volumes), cyclohexane/DCM 8/2 (2), cyclohexane/DCM 6/4 (2) and DCM (2). The relevant fractions were combined and evaporated to give the product (25 mg) as a colourless gum. LC/MS: m/z 502 [M+NH₄]⁺, Rₜ 4.18 min.

### Intermediate 166 : Ethyl [4-({3-[3-methyl-6-(methyloxy)-1-benzothien-2-yl]heptyl}oxy)-3-(methyloxy)phenyl]acetate (isomer 2)

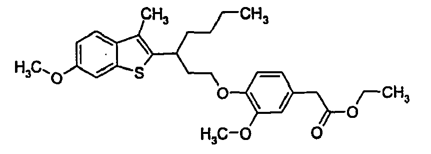

This compound was prepared from 2-[1-(2-bromoethyl)pentyl]-3-methyl-6-(methyloxy)-1-benzothiophene (isomer 2) by the same procedure used for Intermediate 165. LC/MS: m/z 502 [M+NH₄]⁺, Rₜ 4.18 min.

### Intermediate 167 : Ethyl [2-({3-[3-methyl-6-(trifluoromethyl)-1-benzothien-2-yl]heptyl}thio)-1,3-thiazol-4-yl]acetate (isomer 1)

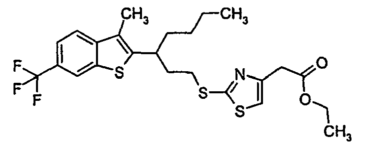

A mixture of 2-[1-(2-bromoethyl)pentyl]-3-methyl-6-(trifluoromethyl)-1-benzothiophene (isomer 1) (79mg, 0.20 mmol), ethyl (2-mercapto-1,3-thioazol-4-yl)acetate (51 mg, 0.25 mmol), cesium carbonate (137 mg, 0.42 mmol), in dry MeCN (3 mL) was stirred at room temperature for 1 day. The solvent was evaporated, the residue taken up with DCM and washed with saturated sodium carbonate and water. The organic layer was separated by filtration on a hydrophobic frit and evaporated to give the title compound (71 mg) as a colourless oil. LC/MS: m/z 516 [M+H]⁺, m/z 560 [M+HCO₂]⁺, Rₜ 4.42 min.

### Intermediate 168 : Ethyl [2-({3-[3-methyl-6-(trifluoromethyl)-1-benzothien-2-yl]heptyl}thio)-1,3-thiazol-4-yl]acetate (isomer 2)

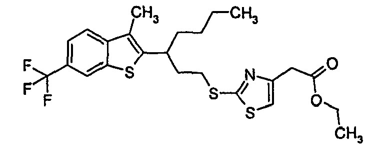

This compound was prepared from 2-[1-(2-bromoethyl)pentyl]-3-methyl-6-(trifluoromethyl)-1-benzothiophene (isomer 2) by the same procedure used for Intermediate 167. LC/MS: m/z 516 [M+H]⁺, m/z 560 [M+HCO₂]⁺, Rₜ 4.42 min.

### Intermediate 169 : Ethyl 3-[4-({3-[3-methyl-6-(trifluoromethyl)-1-benzothien-2-yl]heptyl)oxy)phenyl]propanoate (isomer 1)

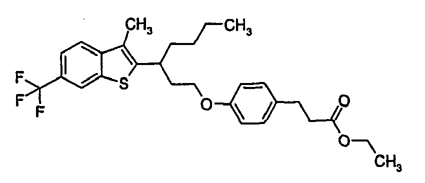

A mixture of 2-[1-(2-bromoethyl)pentyl]-3-methyl-6-(trifluoromethyl)-1-benzothiophene (isomer 1) (79 mg, 0.20 mmol), ethyl 3-(4-hydroxyphenyl)propanoate (51 mg, 0.25 mmol), and cesium carbonate (137 mg, 0.42 mmol), in dry MeCN (3 mL) was stirred at room temperature for 6 day. The solvent was evaporated, the residue triturated with cyclohexane and filtered. The organic solution was then applied to a Si-SPE cartridge (1 g) and eluted with cyclohexane (3 volumes), DCM (2) and ether (2). The relevant fractions were combined and evaporated to give the product (82 mg) as a colourless gum. LC/MS: m/z 524 [M+NH₄]⁺, m/z 551 [M+HCO₂]⁺, Rₜ 4.57 min.

### Intermediate 170 : Ethyl 3-[4-({3-[3-methyl-6-(trifluoromethyl)-1-benzothien-2-yl]heptyl}oxy)phenyl]propanoate (isomer 2)

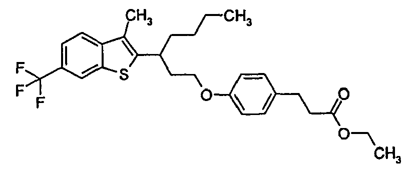

This compound was prepared from 2-[1-(2-bromoethyl)pentyl]-3-methyl-6-(trifluoromethyl)-1-benzothiophene (isomer 2) by the same procedure used for Intermediate 169. LC/MS: m/z 524 [M+NH₄]⁺, m/z 551 [M+HCO₂]⁺, Rₜ 4.57 min.

### Intermediate 171 : Ethyl [3-(methyloxy)-4-({3-[3-methyl-6-(trifluoromethyl)-1-benzothien-2-yl]heptyl)oxy)phenyl]acetate (isomer 1)

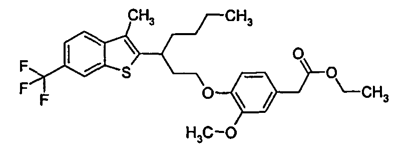

This compound was prepared from 2-[1-(2-bromoethyl)pentyl]-3-methyl-6-(trifluoromethyl)-1-benzothiophene (isomer 1) and ethyl [4-hydroxy-3-(methyloxy)phenyl]acetate by the same procedure used for Intermediate 169. LC/MS: m/z 540 [M+NH₄]⁺, m/z 567 [M+HCO₂]⁺ Rₜ 4.39 min.

### Intermediate 172 : Ethyl [3-methoxy-4-({3-[3-methyl-6-(trifluoromethyl)-1-benzothien-2-yl]heptyl)oxy)phenyl]acetate (isomer 2)

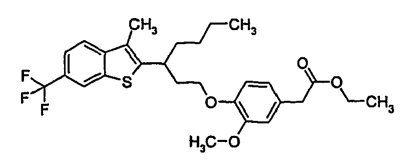

This compound was prepared from 2-[1-(2-bromoethyl)pentyl]-3-methyl-6-(trifluoromethyl)-1-benzothiophene (isomer 2) and ethyl [4-hydroxy-3-(methyloxy)phenyl]acetate by the same procedure used for Intermediate 169. LC/MS: m/z 540 [M+NH₄]⁺, m/z 567 [M+HCO₂]⁺, Rₜ 4.39 min.

### Example 1 : [(4-{[3-(5-Bromo-3-methyl-1-benzothien-2-yl)propyl]thio}-2-methylphenyl)oxy]acetic acid

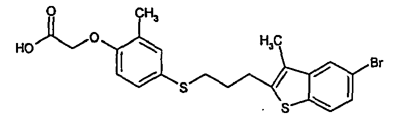

To ethyl [(4-{[3-(5-bromo-3-methyl-1-benzothien-2-yl)propyl]thio}-2-methylphenyl)oxy]acetate (0.075 g, 0.15 mmol) in MeOH was added sodium hydroxide (0.3 mL 2M solution, 0.6 mmol) and the mixture was heated at 60°C for 0.5 hours. The solution was concentrated *in vacuo* and the residues dissolved in water (20 mL). The solution was acidified with 2M HCl and the liberated acid was extracted with chloroform (3x 10 mL). The combined extracts were washed with water (10 mL), brine (10 mL) and dried. Removal of solvent gave the title compound as a white solid (0.055 g). ¹H NMR (400MHz; CDCl₃) δ: 1.95 (2H, m), 2.25 (3H, s), 2.27 (3H, s), 2.86 (2H, t, J 7 Hz), 2.98 (2H, t, J 7.5 Hz), 4.67 (2H, s), 6.65 (1 H, d, J 8 Hz), 7.17 (1 H, dd, J 8, 2 Hz), 7.2 (1 H, m) 7.36 (1 H, dd, J 8, 2 Hz), 7.59 (1 H, d, J 8 Hz), 7.72 (1H, d, J 2 Hz), -CO₂H not observed; LC/MS: m/z 463, 465 [M-H]⁻, Rₜ 4.47 min.

### Example 2 : [(4-{[3-(6-Bromo-3-methyl-1-benzothien-2-yl)propyl]thio}-2-methylphenyl)oxy]acetic acid

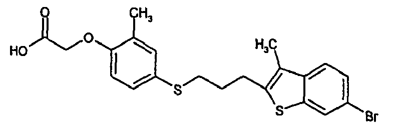

Prepared in a similar manner to Example 1 from ethyl [(4-{[3-(6-bromo-3-methyl-1-benzothien-2-yl)propyl]thio}-2-methylphenyl)oxy]acetate (0.1 g, 0.2 mmol) to give the title compound as a white solid (0.085 g). ¹H NMR (400MHz; CDCl₃) δ: 1.95 (2H, m), 2.25 (3H, s), 2.28 (3H, s), 2.87 (2H, t, J 6.5 Hz), 2.98 (2H, t, J 7 Hz), 4.67 (2H, s), 6.66 (1 H, d, J 8 Hz), 7.18 (1 H, dd, J 8, 2 Hz), 7.21 (1 H, m), 7.44 (2H, m), 7.87 (1 H, m), -CO₂H not observed; LC/MS: m/z 463,465 [M-H]⁻, Rₜ 4.73 min.

### Example 3 : {[2-Methyl-4-({3-[3-methyl-5-(trifluoromethyl)-1-benzothien-2-yl]propyl}thio)phenyl]oxy}acetic acid

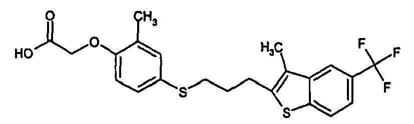

Prepared in a similar manner to Example 1 from ethyl {[2-methyl-4-({3-[3-methyl-5-(trifluoromethyl)-1-benzothien-2-yl]propyl}thio)phenyl]oxy}acetate (0.15 g, 0.3 mmol) to give the title compound as a white solid (0.1 g). ¹H NMR (400MHz; CDCl₃) δ: 1.97 (2H, m), 2.25 (3H, s), 2.34 (3H, s), 2.88 (2H, t, J 7 Hz), 3.03 (2H, t, J 7 Hz), 4.68 (2H, s), 6.63 (1 H, d, J 8 Hz), 7.18 (1 H, dd, J 8, 2 Hz), 7.22 (1H. m) 7.5 (1 H, dd, J, 8, 2 Hz), 7.84 (2H, m), -CO₂H not observed; LC/MS: m/z 453 [M-H]⁻, Rₜ 5.36 min.

### Example 4 : {[2-Ethyl-4-({3-[3-methyl-5-(trifluoromethyl)-1-benzothien-2-yl]propyl}thio)phenyl]oxy}acetic acid

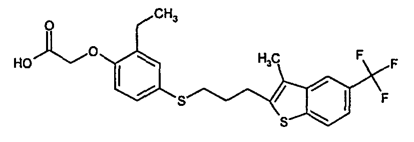

Prepared in a similar manner to Example 1 from ethyl {[2-ethyl-4-({3-[3-methyl-5-(trifluoromethyl)-1-benzothien-2-yl]propyl}thio)phenyl]oxy}acetate (0.14 g, 0.29 mmol) to give the title compound as a white solid (0.1 g). ¹H NMR (400MHz; CDCl₃) δ: 1.2 (3H, t, J 8 Hz), 1.97 (2H, m), 2.33 (3H, s), 2.67 (2H, q, J 7 Hz), 2.89 (2H, t, J 8 Hz), 3.03 (2H, t, J 8 Hz), 4.67 (2H, s), 6.65 (1 H, d, J 8 Hz), 7.18 (1 H, dd, J 8, 2 Hz), 7.23 (1 H, m), 7.49 (1 H, dd, J, 8, 2 Hz), 7.83 (2H, m), -CO₂H not observed; LC/MS: m/z 467 [M-H]⁻, Rₜ 4.7 min.

### Example 5 : {[4-({3-[3-Ethyl-6-(trifluoromethyl)-1-benzothien-2-yl]propyl}thio)-2-methylphenyl]oxy}acetic acid

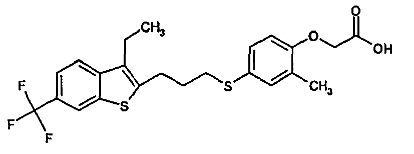

Prepared in a similar manner to Example 1 from ethyl {[4-({3-[3-ethyl-6-(trifluoromethyl)-1-benzothien-2-yl]propyl}thio)-2-methylphenyl]oxy}acetate (0.12 g, 0.24 mmol) to give the title compound as a white solid (0.09 g). ¹H NMR (400MHz; CDCl₃) δ: 1.19 (3H, t, J 7 Hz), 1.98 (2H, m), 2.25 (3H, s), 2 .82 (2H, q, J 8 Hz), 2.9 (2H, t, J 7 Hz), 3.04 (2H, t, J 8 Hz), 4.67 (2H, s), 6.66 (1 H, d, J 8 Hz), 7.2 (1 H, dd, J 8, 2 Hz), 7.23 (1 H, m), 7.55 (1 H, d, J 8 Hz), 7.71 (1 H, d, J 8 Hz), 8.03 (1 H, s), -CO₂H not observed; LC/MS: m/z 467 [M-H]⁻.

### Example 6: {[2-Ethyl-4-({3-[3-ethyl-6-(trifluoromethyl)-1-benzothien-2-yl]propyl}thio)phenyl]oxy}acetic acid

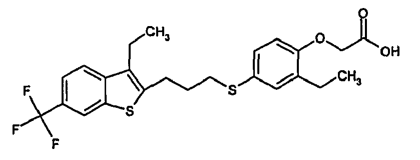

Prepared in a similar manner to Example 1 from ethyl {[2-ethyl-4-({3-[3-ethyl-6-(trifluoromethyl)-1-benzothien-2-yl]propyl}thio)phenyl]oxy}acetate (0.13 g, 0.24 mmol) to give the title compound as a white solid (0.09 g). ¹H NMR (400MHz; CDCl₃) δ: 1.19 (6H, m), 1.99 (2H, m), 2.65 (2H, q, J 8 Hz), 2.81 (2H, q, J 8 Hz), 2.91 (2H, t, J 8 Hz), 3.04 (2H, t, J 8 Hz), 4.68 (2H, s), 6.66 (1 H, d, J 8 Hz), 7.2 (1 H, dd, J 8, 2 Hz), 7.24 (1 H, m), 7.55 (1 H, dd, J 8, 2 Hz), 7.71 (1 H, d, J 8 Hz), 8.03 (1 H, s) -CO₂H not observed; LC/MS: m/z 481 [M-H]⁻.

### Example 7 : {[2-Methyl-4-({3-[6-(trifluoromethyl)-1-benzothien-2-yl]propyl}thio)phenyl]oxy}acetic acid

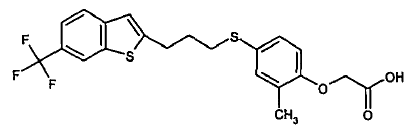

Prepared in a similar manner to Example 1 from ethyl {[2-methyl-4-({3-[6-(trifluoromethyl)1-benzothien-2-yl]propyl}thio)phenyl]oxy}acetate (0.13 g, 0.28 mmol) to give the title compound as a white solid (0.12 g). ¹H NMR (400MHz; CDCl₃) δ: 2.03 (2H, m), 2.25 (3H, s), 2.88 (2H, t, J 7 Hz), 3.07 (2H, t, 7 Hz), 4.67 (2H, s), 6.66 (1 H d, 8 Hz), 7.05 (1 H, s), 7.19 (1H, dd, J 8, 2 Hz), 7.22 (1H, m), 7.53 (1H. dd, J 8, 2 Hz), 7.73 (1H, d, J 8 Hz), 8.0 (1H, s), -CO₂H not observed; LC/MS: m/z 439 [M-H]⁻. Rₜ 4.71 min.

### Example 8: {[2-Ethyl-4-({3-[6-(trifluoromethyl)-1-benzothien-2-yl]propyl}thio)phenyl]oxy}acetic acid

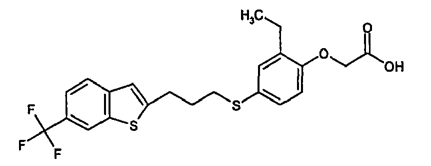

Prepared in a similar manner to Example 1 from ethyl {[2-ethyl-4-({3-[6-(trifluoromethyl)-1-benzothien-2-yl]propyl}thio)phenyl]oxy}acetic acid (0.13 g, 0.25 mmol) to give the title compound as a white solid (0.12 g). ¹H NMR (400MHz; CDCl₃) δ: 1.2 (3H, t, J 8 Hz), 2.04 (2H, m), 2.66 (2H, q, J 7 Hz), 2.89 (2H, t, J 7 Hz), 3.08 (2H, t, J 8 Hz), 4.63 (2H, s), 6.67 (1 H, d, J 8 Hz), 7.05 (1 H, s), 7.2 (1H. dd, J 8, 2 Hz), 7.23 (1H. m), 7.53 (1H. dd, J 8, 2 Hz), 7.73 (1 H, d, J 8 Hz), 8.03 (1 H, m), -CO₂H not observed; LC/MS: m/z 453 [M-H]⁻. Rₜ 4.88 min.

### Example 9 : {[2-(Trifluoromethyl)-4-({3-[6-(trifluoromethyl)-1-benzothien-2-yl]propyl}thio)phenyl]oxy}acetic acid

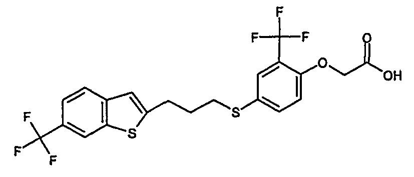

Prepared in a similar manner to Example 1 from ethyl {[2-(trifluoromethyl)-4-({3-[6-(trifluoromethyl)-1-benzothien-2-yl]propyl}thio)phenyl]oxy}acetate (0.14 g, 0.26 mmol) to give the title compound as a white solid (0.12 g). ¹H NMR (400MHz; CDCl₃) δ: 2.05 (2H, m), 2.93 (2H, t, J 7 Hz), 3.08 (2H, t, 7 Hz), 4.75 (2H, s), 6.85 (1 H d, 8 Hz), 7.07 (1H. s), 7.5 (1H, dd, J 8, 2 Hz), 7.55 (1 H, dd, J 8, 2 Hz), 7.64 (1 H, d, J 2 Hz) 7.74 (1 H, d, J 8Hz), 8.04 (1 H, m) -CO₂H no observed; LC/MS: m/z 493 [M-H]⁻. Rₜ 4.8 min.

### Example 10 : {[2-Ethyl-4-({3-[3-methyl-6-(trifluoromethyl)-1-benzothien-2-yl]propyl}thio)phenyl]oxy}acetic acid

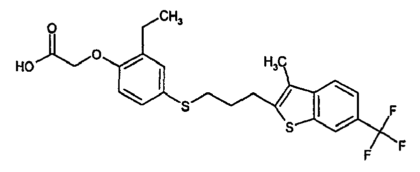

Prepared in a similar manner to Example 1 from ethyl {[2-ethyl-4-({3-[3-methyl-6-(trifluoromethyl)-1-benzothien-2-yl]propyl}thio)phenyl]oxy}acetate (0.21 g, 0.41 mmol) to give the title compound as a white solid (0.2 g). ¹H NMR (400MHz; CDCl₃) δ: 1.2 (3H, t, J 8 Hz), 1.98 (2H, m), 2.33 (3H, s), 2.66 (2H, q, J 7 Hz), 2.88 (2H, t, J 7 Hz), 3.04 (2H, t, J 8 Hz), 4.68 (2H, s), 6.67 (1 H, d, J 8 Hz), 7.18 (1 H, dd, J 8, 2 Hz), 7.23 (1 H, m), 7.56 (1 H, dd, J 8, 2Hz), 7.68 (1 H, d, J 8 Hz), 8.02 (1 H, m), -CO₂H not observed; LC/MS: m/z 467 [M-H]⁻. Rₜ 5.05 min.

### Example 11 : 2-Methyl-2-{[2-methyl-4-({3-[3-methyl-6-(trifluoromethyl)-1-benzothien-2-yl]propyl}thio)phenyl]oxy}propanoic acid

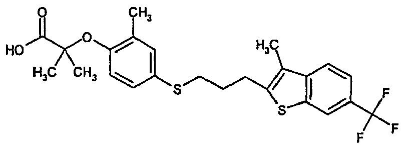

Prepared in a similar manner to Example 1 from ethyl 2-methyl-2-{[2-methyl-4-({3-[3-methyl-6-(trifluoromethyl)-1-benzothien-2-yl]propyl}thio)phenyl]oxy}propanoate (0.21 g, 0.41 mmol) to give the title compound as a white solid (0.23 g). ¹H NMR (400MHz; CDCl₃) δ: 1.61 (6H, s), 1.98 (2H, m), 2.2 (3H, s), 2.33 (3H, s), 2.9 (2H, t, J 7 Hz), 3.04 (2H, t, 7 Hz), 6.74 (1 H d, 8 Hz), 7.12 (1H, dd, J 8,2 Hz), 7.18 (1H, m), 7.56 (1H, dd, J 8, 2 Hz), 7.68 (1H, d, J 8 Hz), 8.02 (1 H, s), -CO₂H not observed; LC/MS: m/z 481 [M-H]⁻. Rₜ 4.57 min.

### Example 12 : {[4-({3-[3-Methyl-6-(trifluoromethyl)-1-benzothien-2-yl]propyl}thio)-2-(trifluoromethyl)phenyl]oxy}acetic acid

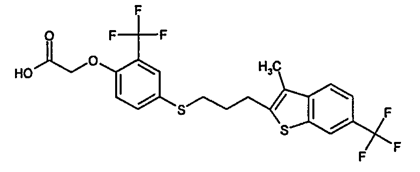

Prepared in a similar manner to Example 1 from ethyl {[4-({3-[3-methyl-6-(trifluoromethyl)-1-benzothien-2-yl]propyl}thio)-2-(trifluoromethyl)phenyl]oxy}acetate (0.2 g, 0.37 mmol) to give the title compound as a white solid (0.09 g). ¹H NMR (400MHz; CDCl₃) δ: 1.99 (2H, m), 2.34 (3H s), 2.93 (2H, t, J 7 Hz), 3.05 (2H, t, J 8 Hz), 4.75 (2H, s), 6.83 (1 H, d, J 8 Hz), 7.5 (1 H, dd, J 8, 2 Hz), 7.57 (1 H, dd, J 8, 2 Hz), 7.63 (1 H, d, J 2 Hz), 7.68 (1 H, d, J 8 Hz), 8.03 (1 H, m), -CO₂H not observed; LC/MS: m/z 507 [M-H]⁻. Rₜ 4.75 min.

### Example 13 : {[2-Methyl-4-({(2E)-3-[3-methyl-5-(trifluoromethyl)-1-benzothien-2-yl]-2-propen-1-yl}thio)phenyl]oxy}acetic acid

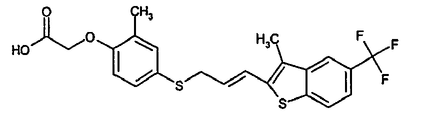

Prepared in a similar manner to Example 1 from ethyl {[2-methyl-4-({(2E)-3-[3-methyl-5-(trifluoromethyl)-1-benzothien-2-yl]-2-propen-1-yl}thio)phenyl]oxy}acetate (0.036 g, 0.075 mmol) to give the title compound as a yellow solid (0.028 g). ¹H NMR (400MHz; CDCl₃) δ: 2.24 (3H s), 2.3 (3H, s), 3.62 (2H, d, J 7 Hz), 4.67 (2H, s), 6.16 (1 H, dt J 15, 7 Hz), 6.58 (1 H, d, 15H Hz), 6.66 (1H. d, J 9 Hz), 7.24 (1H. dd, J 8, 2 Hz), 7.28 (1 H, m), 7.51 (1H. m), 7.82 (2H, m), - CO₂H not observed; LC/MS: m/z 451 [M-H]⁻. Rₜ 5.27 min.

### Example 14 : 2-[(4-{[3-(6-Bromo-3-methyl-1-benzothien-2-yl)propyl]oxy}-2-methylphenyl)oxy]-2-methylpropanoic acid

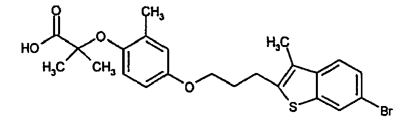

Prepared in a similar manner to Example 1 from ethyl 2-[(4-{[3-(6-bromo-3-methyl-1-benzothien-2-yl)propyl]oxy}-2-methylphenyl)oxy]-2-methylpropanoate (0.076 g, 0.075 mmol) to give the title compound as an oil (0.05 g). ¹H NMR (400MHz; CDCl₃) δ: 1.55 (6H, s), 2.14 (2H, m), 2.22 (3H, s), 2.27 (3H, s), 3.05 (2H, t, J 7 Hz), 3.93 (2H, t, J 6Hz), 6.62 (1 H, dd, J 8, 2 Hz), 6.72 (1 H, d, J 3 Hz), 6.82 (1 H, d, J 9 Hz), 7.44 (2H, m), 7.88 (1 H, m), -CO₂H not observed; LC/MS: m/z 475,477 [M-H]⁻. Rₜ 4.41 min.

### Example 15 : 2-Methyl-2-{[2-methyl-4-({3-[3-methyl-5-(trifluoromethyl)-1-benzothien-2-yl]propyl}oxy)phenyl]oxy}propanoic acid

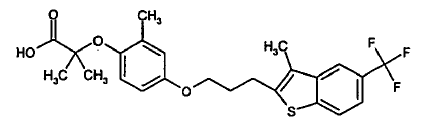

Prepared in a similar manner to Example 1 from ethyl 2-methyl-2-{[2-methyl-4-({3-[3-methyl-5-(trifluoromethyl)-1-benzothien-2-yl]propyl}oxy)phenyl]oxy}propanoate (0.11 g, 0.22 mmol) to give the title compound as an oil (0.1g). ¹H NMR (400MHz; CDCl₃) δ: 1.55 (6H, s), 2.16 (2H, m), 2.22 (3H, s), 2.33 (3H, s), 3.11 (2H, t, J 7 Hz), 3.94 (2H, t, J 7 Hz), 6.63 (1 H, dd, J 8, 2 Hz), 6.72 (1 H, d, J 3 Hz), 6.82 (1 H, d, J 8 Hz), 7.5 (1 H, dd, J 8, 2 Hz), 7.85 (2H, m) -CO₂H not observed; LC/MS: m/z 465 [M-H]⁻. Rₜ 4.47 min.

### Example 16 : 2-Methyl-2-{[2-methyl-4-({3-[3-methyl-6-(trifluoromethyl)-1-benzothien-2-yl]propyl}oxy)phenyl]oxy}propanoic acid

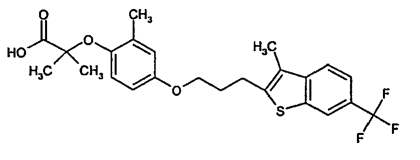

Prepared in a similar manner to Example 1 from ethyl 2-methyl-2-{[2-methyl-4-({3-[3-methyl-6-(trifluoromethyl)-1-benzothien-2-yl]propyl}oxy)phenyl]oxy}propanoate (0.15 g, 0.31 mmol) to give the title compound as a white solid (0.15 g). ¹H NMR (400MHz; CDCl₃) δ: 1.55 (6H, s), 2.15 (2H, m), 2.22 (3H, s), 2.32 (3H, s), 3.11 (2H, t, J 7 Hz), 3.94 (2H, t, J 7Hz), 6.63 (1 H, dd, J 8, 2 Hz), 6.73 (1 H, d, J 3 Hz), 6.82 (1 H, d, J 8 Hz), 7.57 (1 H, dd, J 8, 2 Hz), 7.67 (1 H, d, J 8 Hz), 8.03 (1 H, m), -CO₂H not observed; LC/MS: m/z 465 [M-H]⁻. Rₜ 4.48 min.

### Example 17 : {[2-Methyl-4-({3-[6-(trifluoromethyl)-1-benzothien-2-yl]heptyl}thio)phenyl]oxy}acetic acid

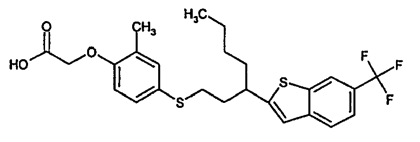

Prepared in a similar manner to Example 1 from ethyl {[2-methyl-4-({3-[6-(trifluoromethyl)-1-benzothien-2-yl]heptyl}thio)phenyl]oxy}acetate (0.09 g, 0.17 mmol) to give the title compound as an oil (0.08 g). ¹H NMR (400MHz; CDCl₃) δ: 0.84 (3H, t, J 7 Hz), 1.15-1.35 (4H, m), 1.58-1.75 (2H, m), 1.85-2.04 (2H, m), 2.22 (3H, s), 2.66 (1 H, m), 2.8 (1 H, m), 3.16 (1H. m), 4.77 (2H, s), 6.64 (1H, d, J 8 Hz), 7.13 (1H, dd, J 8, 2 Hz), 7.05 (1H, s), 7.15 (1H m), 7.54 (1 H, dd, J 8, 2 Hz), 7.75 (1 H, d, J 8 Hz), 8.0 (1 H, m), -CO₂H not observed; LC/MS: m/495 [M-H]⁻. Rₜ 5.14 min.

### Example 18 : {[2-(Trifluoromethyl)-4-({3-[6-(trifluoromethyl)-1-benzothien-2-yl]heptyl}thio)phenyl]oxy}acetic acid

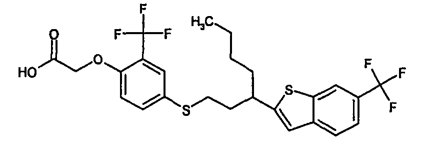

Prepared in a similar manner to Example 1 from ethyl {[2-(trifluoromethyl)-4-({3-[6-(trifluoromethyl)-1-benzothien-2-yl]heptyl}thio)phenyl]oxy}acetate (0.08 g, 0.15 mmol) to give the title compound as an oil (0.07 g). ¹H NMR (400MHz; CDCl₃) δ: 0.85 (3H, t, J 7 Hz), 1.15-1.35 (4H, m), 1.6-1.75 (2H, m), 1.85-2.05 (2H, m), 2.72 (1 H, m), 2.84 (1 H, m), 3.16 (1 H, m), 4.77 (2H, s), 6.64 (1 H, d, J 8 Hz), 7.05 (1 H, s), 7.13 (1 H, dd, J 8, 2 Hz), 7.18 (1H. m), 7.54 (1 H, dd, J 8, 2 Hz), 7.76 (1 H, d, J 8 Hz), 8.04 (1 H, m), -CO₂H not observed; LC/MS: m/z 549 [M-H]⁻. Rₜ 4.98 min.

### Example 19: {[2-Methyl-4-({3-[5-(trifluoromethyl)-1-benzothien-2-yl]heptyl}thio)phenyl]oxy}acetic acid

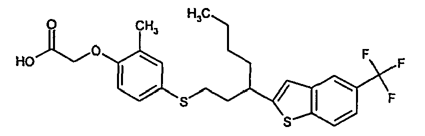

To a solution of ethyl {[2-methyl-4-({3-[5-(trifluoromethyl)-1-benzothien-2-yl]heptyl}thio)phenyl]oxy}acetate (89 mg, 0.17 mmol) in MeOH (5 mL) was added 2M sodium hydroxide (0.34 mL). After stirring for 1 h at 60°C the solvent was evaporated and the residue was diluted with water (20mL), acidified with 2M HCl and extracted with EtOAc (2 x 20 mL). The organic extracts were washed with brine (20 mL), dried over MgSO₄, and evaporated to a gum. This was purified through a 5 g silica SPE cartridge, eluting with DCM, EtOAc and MeCN, to afford the title compound as a colourless gum (57 mg) which was isolated as a white solid on freeze-drying a dioxan solution. ¹H NMR (400 MHz; DMSO-d₆) δ: 0.80 (3H, t, J 7 Hz), 1.05-1.30 (4H, m), 1.57 (1 H, m), 1.68 (1H. m), 1.80 (1 H, m), 1.91 (1 H, m), 2.10 (3H, s), 2.72 (2H, m), 3.20 (1 H, m), 4.40 (2H, s), 6.68 (1 H, d, J 8 Hz), 7.08 (1 H, m), 7.09 (1 H, m), 7.31 (1 H, s), 7.58 (1 H, d, J 8 Hz), 8.15 (1 H, d, J 8 Hz), 8.16 (1 H, s), -CO₂H not observed; LC/MS: m/z 495 [M-H]⁻, Rₜ 4.82 min.

### Example 20 : {[2-Methyl-4-({3-[5-(trifluoromethyl)-1-benzothien-2-yl]heptyl}oxy)phenyl]oxy}acetic acid

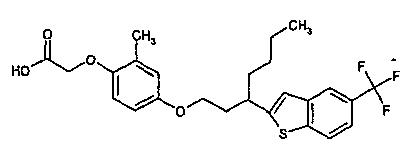

To a solution of ethyl {[2-methyl-4-({3-[5-(trifluoromethyl)-1-benzothien-2-yl]heptyl}oxy)phenyl]oxy}acetate (90 mg, 0.18 mmol) in MeOH (5 mL) was added 2M sodium hydroxide (0.34 mL). After stirring for 1 h at 60°C the solvent was evaporated and the residue was diluted with water (20mL), acidified with 2M HCl and extracted with DCM (2 x 20 mL). The organic extracts were washed with brine (20 mL), dried over MgSO₄, and evaporated to a gum This was purified through a 5g silica SPE cartridge, eluting with DCM, EtOAc and MeCN, to afford the title compound as a colourless gum (23 mg) which was isolated as a white solid on freeze-drying a dioxan solution. ¹H NMR (400 MHz; DMSO-d₆) δ: 0.82 (3H, t, J 7 Hz), 1.15-1.35 (4H, m), 1.65 (1H. m), 1.78 (1H. m), 1.97 (1 H, m), 2.16 (1H. m), 2.11 (3H, s), 3.27 (1 H, m), (1H. m), 3.85 (1 H, m), 4.56 (2H, s), 6.60 (1 H, dd, J 8 Hz, 3Hz), 6.67 (1 H, d, J 3 Hz), 6.68 (1 H, d, J 8 Hz), 7.36 (1H. s), 7.58 (1H. d, J 8 Hz), 8.14 (1 H, d, J 8 Hz), 8.16 (1H. s), -CO₂H not observed; LC/MS: m/z 479 [M-H]⁻, Rₜ 4.64 min.

### Example 21 : {[2-Ethyl-4-({3-[5-(trifluoromethyl)-1-benzothien-2-yl]heptyl}oxy)phenyl]oxy}acetic acid

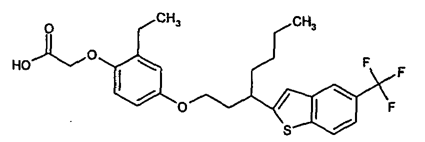

To a solution of ethyl {[2-ethyl-4-({3-[5-(trifluoromethyl)-1-benzothien-2-yl]heptyl}oxy)phenyl]oxy}acetate (80 mg, 0.15 mmol) in MeOH (5 mL) was added 2M sodium hydroxide (0.34 mL). After stirring for 1 h at 60°C the solvent was evaporated and the residue was diluted with water (20mL), acidified with 2M HCl and extracted with DCM (2 x 20 mL). The organic extracts were washed with brine (20 mL), dried over MgSO₄, and evaporated to a gum This was purified through a 5 g silica SPE cartridge, eluting with DCM, EtOAc and MeCN, to afford the title compound as a colourless gum (23 mg) which was isolated as a white solid on freeze-drying a dioxan solution. ¹H NMR (400 MHz; DMSO-d₆) δ: 0.82 (3H, t, J 7 Hz), 1.08 (3H t, J 7 Hz), 1.15-1.35 (4H, m), 1.65 (1 H, m), 1.78 (1H. m), 1.97 (1H, m), 2.16 (1 H, m), 2.52 (2H, q, J 7 Hz, partly hidden by DMSO), 3.27 (1 H, m), 3.79 (1 H, m), 3.85 (1 H, m), 4.56 (2H, s), 6.60 (1H, dd, J 8 Hz, 3Hz), 6.65 (1 H, d, J 3 Hz), 6.68 (1 H, d, J 8 Hz), 7.37 (1H, s), 7.58 (1 H, d, J 8 Hz), 8.15 (1 H, d, J 8 Hz), 8.15 (1 H, s), -CO₂H not observed; LC/MS: m/z 493 [M-H]⁻, Rₜ 4.7 min.

### Example 22 : {[2-Methyl-4-({3-[3-methyl-6-(trifluoromethyl)-1-benzothien-2-yl]hexyl}thio)phenyl]oxy}acetic acid

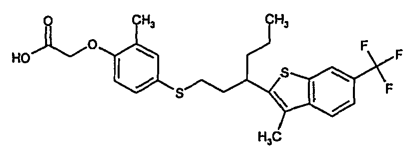

To a solution of ethyl {[2-methyl-4-({3-[3-methyl-6-(trifluoromethyl)-1-benzothien-2-yl]hexyl}thio)phenyl]oxy}acetate (125 mg, 0.24 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (49 mg). ¹H NMR (400 MHz; DMSO-d₆) δ: 0.82 (3H, t, J 7 Hz), 1.10-1.25 (2H, m), 1.52 (1 H, m), 1.68 (1 H, m), 1.78 (1 H, m), 1.93 (1 H, m), 2.10 (3H, s), 2.35 (3H, s), 2.72 (2H, m), 3.41 (1H, m), 4.67 (2H, s), 6.74 (1 H, d, J 8 Hz), 7.09 (1H, m), 7.10 (1H, m), 7.67 (1H. d, J 8 Hz), 7.88 (1H. d, J 8 Hz), 8.37 (1H, s) 13.0 (1H, s); LC/MS: m/z 495 [M-H]⁻, Rₜ 4.75 min.

### Example 23 : [4-Methyl-2-({3-[3-methyl-6-(trifluoromethyl)-1-benzothien-2-yl]hexyl}thio)-1,3-thiazol-5-yl]acetic acid

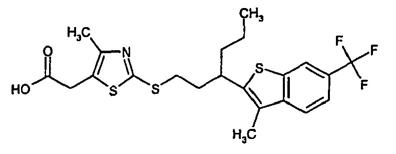

To a solution of ethyl [4-methyl-2-({3-[3-methyl-6-(trifluoromethyl)-1-benzothien-2-yl]hexyl}thio)-1,3-thiazol-5-yl]acetate (80 mg, 0.16 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (40 mg). ¹H NMR (400 MHz; DMSO-d₆) δ: 0.84 (3H, t, J 7 Hz), 1.10-1.28 (2H, m), 1.55 (1 H, m), 1.75 (1H, m). 1.95 (1 H, m), 2.13 (1H, m). 2.14 (3H, s), 2.36 (3H, s), 3.00 (1H. m), 3.08 (1 H, m), 3.41 (1H, m), 3.72 (2H, s), 7.67 (1 H, d, J 8 Hz), 7.89 (1 H, d, J 8 Hz), 8.38 (1 H, s) 12.6 (1 H, s). LC/MS: m/z 488 [M+H]⁺, Rₜ 4.28 min.

### Example 24 : {[2-Methyl-4-({3-[3-methyl-6-(trifluoromethyl)-1-benzothien-2-yl]hexyl}oxy)phenyl]oxy}acetic acid

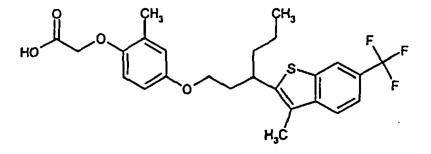

To a solution of ethyl {[2-methyl-4-({3-[3-methyl-6-(trifluoromethyl)-1-benzothien-2-yl]hexyl}oxy)phenyl]oxy}acetate (35 mg, 0.07 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (3 mg). LC/MS: m/z 479 [M-H]⁻, Rₜ 4.56 min.

### Example 25 : {[2-Ethyl-4-({3-[3-methyl-6-(trifluoromethyl)-1-benzothien-2-yl]hexyl}oxy)phenyl]oxy}acetic acid

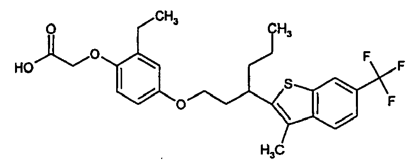

To a solution of {[2-ethyl-4-({3-[3-methyl-6-(trifluoromethyl)-1-benzothien-2-yl]hexyl}oxy)phenyl]oxy}acetic acid (83 mg, 0.16 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (32 mg). ¹H NMR (400 MHz; DMSO-d₆) δ: 0.86 (3H, t, J 7 Hz), 1.08 (3H, t, J 7 Hz), 1.14-1.30 (2H, m), 1.62 (1 H, m), 1.78 (1 H, m), 1.88 (1 H, m), 2.20 (1 H, m), 2.29 (3H, s), 2.52 (2H, q, J 7 Hz), 3.50 (1 H, m), 3.64 (1 H, m), 3.86 (1 H, m), 4.57 (2H, s), 6.60 (1 H, dd, J 8 Hz, 3Hz), 6.65 (1 H, d, J 3 Hz), 6.68 (1 H, d, J 8 Hz), 7.65 (1 H, d, J 8 Hz), 7.85 (1 H, d, J 8 Hz), 8.38 (1 H, s) 12.9 (1 H, s); LC/MS: m/z 493 [M-H]⁻, Rₜ 4.56 min.

### Example 26 : {[2-Methyl-4-({3-[3-methyl-6-(trifluoromethyl)-1-benzothien-2-yl]heptyl}thio)phenyl]oxy}acetic acid

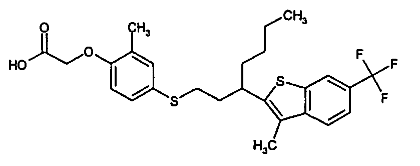

To a solution of ethyl {[2-methyl-4-({3-[3-methyl-6-(trifluoromethyl)-1-benzothien-2-yl]heptyl}thio)phenyl]oxy}acetate (95 mg, 0.18 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (42 mg). ¹H NMR (400 MHz; DMSO-d₆) δ: 0.79 (3H, t, J 7 Hz), 1.02-1.32 (4H, m), 1.52 (1 H, m), 1.66-1.83 (2H, m), 1.93 (1 H, m), 2.10 (3H, s), 2.35 (3H, s), 2.72 (2H, m), 3.39 (1 H, m), 4.67 (2H, s), 6.74 (1H, d, J 8 Hz), 7.09 (1H, m), 7.10 (1 H, m), 7.67 (1H, d, J 8 Hz), 7.88 (1H, d, J 8 Hz), 8.37 (1 H, s) 13.0 (1 H, s); LC/MS: m/z 509 [M-H]⁻, Rₜ 4.84 min.

### Example 27 : [4-Methyl-2-({3-[3-methyl-6-(trifluoromethyl)-1-benzothien-2-yl]heptyl}thio)-1,3-thiazol-5-yl]acetic acid

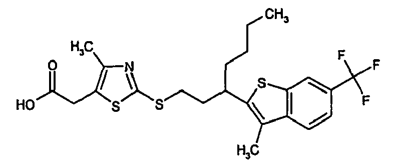

To a solution of ethyl [4-methyl-2-({3-[3-methyl-6-(trifluoromethyl)-1-benzothien-2-yl]heptyl}thio)-1,3-thiazol-5-yl]acetate (75 mg, 0.14 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (62 mg). ¹H NMR (400 MHz; DMSO-d₆) δ: 0.80 (3H, t, J 7 Hz), 1.05-1.32 (4H, m), 1.56 (1 H, m), 1.77 (1 H, m), 1.95 (1 H, m), 2.14 (1 H, m), 2.14 (3H, s), 2.36 (3H, s), 3.00 (1H, m), 3.08 (1 H, m), 3.40 (1 H, m), 3.72 (2H, s), 7.67 (1 H, d, J 8 Hz), 7.89 (1H, d, J 8 Hz), 8.38 (1 H, s) 12.6 (1 H, s); LC/MS: m/z 502 [M+H]⁺, Rₜ 4.39 min.

### Example 28 : {[2-Methyl-4-({3-[3-methyl-6-(trifluoromethyl)-1-benzothien-2-yl]heptyl}oxy)phenyl]oxy}acetic acid

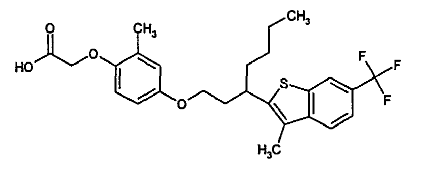

To a solution of ethyl {[2-methyl-4-({3-[3-methyl-6-(trifluoromethyl)-1-benzothien-2-yl]heptyl}oxy)phenyl]oxy}acetate (82 mg, 0.16 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (34 mg). ¹H NMR (400 MHz; DMSO-d₆) δ: 0.81 (3H, t, J 7 Hz), 1.07-1.36 (4H, m), 1.62 (1 H, m), 1.80 (1 H, m), 1.87 (1 H, m), 2.11 (3H, s), 2.20 (1 H, m), 2.27 (3H, s), 3.48 (1 H, m), 3.62 (1 H, m), 3.84 (1 H, m), 4.57 (2H, s), 6.59 (1 H, dd, J 8 Hz, 3Hz), 6.68 (1H, d, J 3 Hz), 6.66 (1H, d, J 8 Hz), 7.65 (1H, d, J 8 Hz), 7.86 (1H, d, J 8 Hz), 8.39 (1H, s), 12.9 (1 H, s); LC/MS: m/z 493 [M-H]⁻, Rₜ 4.62 min.

### Example 29 : {[2-Ethyl-4-({3-[3-methyl-6-(trifluoromethyl)-1-benzothien-2-yl]heptyl}oxy)phenyl]oxy}acetic acid

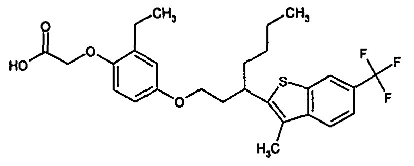

To a solution of ethyl {[2-ethyl-4-({3-[3-methyl-6-(trifluoromethyl)-1-benzothien-2-yl]heptyl}oxy)phenyl]oxy}acetate (80 mg, 0.15 mmol) in MeOH (2 mL) and THF (2 mL) was added 2M sodium hydroxide (0.2 mL). After stirring for 1 h at 20°C the solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM, EtOAc and MeCN, to afford the title compound as a colourless gum (61 mg). ¹H NMR (400 MHz; DMSO-d₆) δ: 0.81 (3H, t, J 7 Hz), 1.08 (3H, t, J 7Hz), 1.15-1.35 (4H, m), 1.62 (1 H, m), 1.80 (1 H, m), 1.88 (1 H, m), 2.20 (1 H, m), 2.28 (3H, s), 2.52 (2H, q, J 7 Hz), 3.48 (1 H, m), 3.64 (1 H, m), 3.85 (1 H, m), 4.57 (2H, s), 6.59 (1H, dd, J 8 Hz, 3Hz), 6.65 (1 H, d, J 3 Hz), 6.68 (1 H, d, J 8 Hz), 7.64 (1H, d, J 8 Hz), 7.86 (1 H, d, J 8 Hz), 8.39 (1 H, s), 12.9 (1 H, s); LC/MS: m/z 507 [M-H]⁻, Rₜ 4.65 min.

### Example 30 : [4-({3-[3-Methyl-6-(trifluoromethyl)-1-benzothien-2-yl]heptyl}oxy)phenyl]acetic acid

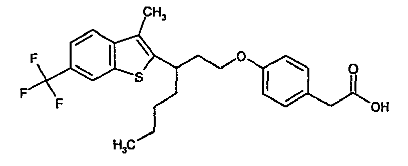

To a solution of ethyl [4-({3-[3-methyl-6-(trifluoromethyl)-1-benzothien-2-yl]heptyl}oxy)phenyl]acetate (45 mg, 0.09 mmol) in MeOH (2 mL) and THF (2 mL) was added 2M sodium hydroxide (0.2 mL). After shaking for 3 h at 20°C, further 2M sodium hydroxide (0.2 mL) was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5g silica SPE cartridge, eluting with DCM and EtOAc to afford the title compound as a colourless oil (17 mg). ¹H NMR (400 MHz; DMSO-d₆) δ 0.81 (3H, t, J 7 Hz), 1.07-1.35 (4H, m), 1.63 (1 H, m), 1.82 (1 H, m), 1.90 (1 H, m), 2.23 (1 H, m), 2.28 (3H, s), 3.45 (2H, s), 3.50 (1 H, m), 3.67 (1 H, m), 3.90 (1 H, m), 6.80 (2H, d, J 8 Hz), 7.11 (2H, d, J 8 Hz), 7.64 (1 H, d, J 8 Hz), 7.85 (1 H, d, J 8 Hz), 8.39 (1 H, s), 12.3 (1 H, s); LC/MS: m/z 463 [M-H]⁻, Rₜ 4.33 min.

### Example 31 : 3-[4-({3-[3-Methyl-6-(trifluoromethyl)-1-benzothien-2-yl]heptyl}oxy)phenyl]propanoic acid

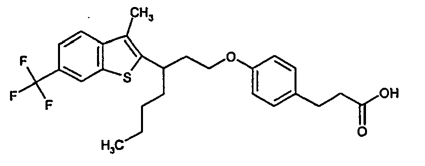

To a solution of ethyl 3-[4-({3-[3-methyl-6-(trifluoromethyl)-1-benzothien-2-yl]heptyl}oxy)phenyl]propanoate (53 mg, 0.11 mmol) in MeOH (2 mL) and THF (2 mL) was added 2M sodium hydroxide (0.2 mL). After shaking for 3 h at 20°C, further 2M sodium hydroxide (0.2 mL) was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM and EtOAc to afford the title compound as a colourless oil (30 mg). ¹H NMR (400 MHz; **DMSO-d₆)** δ: 0.81 (3H, t, J 7 Hz), 1.07-1.35 (4H, m), 1.63 (1H, m), 1.82 (1 H, m), 1.89 (1 H, m), 2.22 (1 H, m), 2.27 (3H, s), 2.45 (2H, t, J 7 Hz), 2.72 (2H, t, J 7 Hz), 3.49 (1 H, m), 3.66 (1 H, m), 3.89 (1 H, m), 6.77 (2H, d, J 8 Hz), 7.07 (2H, d, J 8 Hz), 7.85 (1 H, d, J 8 Hz), 7.85 (1 H, d, J 8 Hz), 8.39 (1 H, s), 12.1 (1 H, s); LC/MS: m/z 477 [M-H]⁻, Rₜ 4.41 min.

### Example 32 : [3-Chloro-4-({3-[3-methyl-6-(trifluoromethyl)-1-benzothien-2-yl]heptyl}oxy)phenyl]acetic acid

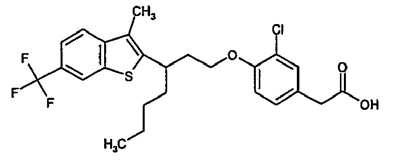

To a solution of ethyl [3-chloro-4-({3-[3-methyl-6-(trifluoromethyl)-1-benzothien-2-yl]heptyl}oxy)phenyl]acetate (65 mg, 0.13 mmol) in MeOH (2 mL) and THF (2 mL) was added 2M sodium hydroxide (0.2 mL). After shaking for 3 h at 20°C, further 2M sodium hydroxide (0.2 mL) was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM and EtOAc to afford the title compound as a colourless oil (31 mg). ¹H NMR (400 MHz; DMSO-d₆) δ: 0.81 (3H, t, J 7 Hz), 1.08-1.37 (4H, m), 1.64 (1 H, m), 1.84 (1 H, m), 1.94 (1 H, m), 2.25 (1 H, m), 2.27 (3H, s), 3.50 (2H, s), 3.57 (1 H, m), 3.75 (1 H, m), 4.04 (1 H, m), 6.95 (1 H, d, J 8 Hz), 7.09 (1 H, dd, J 8 Hz, J 2 Hz), 7.32 (1 H, d, J 2 Hz), 7.64 (1 H, d, J 8 Hz), 7.85 (1 H, d J 8 Hz) 8.39 (1 H, s), 12.4 (1 H, s); LC/MS: m/z 497, 499 [M-H]⁻, Rₜ 4.45 min.

### Example 33 : [3-(Methyloxy)-4-({3-[3-methyl-6-(trifluoromethyl)-1-benzothien-2-yl]heptyl}oxy)phenyl]acetic acid

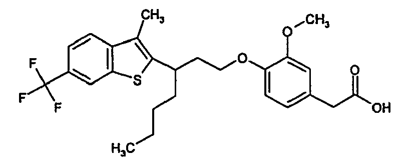

To a solution of ethyl [3-(methyloxy)-4-({3-[3-methyl-6-(trifluoromethyl)-1-benzothien-2-yl]heptyl}oxy)phenyl]acetate (32 mg, 0.06 mmol) in MeOH (2 mL) and THF (2 mL) was added 2M sodium hydroxide (0.2 mL). After shaking for 3 h at 20°C, further 2M sodium hydroxide (0.2 mL) was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5g silica SPE cartridge, eluting with DCM and EtOAc to afford the title compound as a colourless oil (5 mg). ¹H NMR (400 MHz; DMSO-d₆) δ: 0.81 (3H, t, J 7 Hz), 1.08-1.34 (4H, m), 1.62 (1 H, m), 1.83 (1 H, m), 1.89 (1 H, m), 2.22 (1 H, m), 2.28 (3H, s), 3.44 (2H, s), 3.51 (1 H, m), 3.69 (1 H, m), 3.73 (3H, s), 3.89 (1 H, m), 6.67 (1 H, dd, J 8 Hz, J 2 Hz), 6.75 (1H, d, J 8 Hz), 6.85 (1H, d, J 2 Hz, 7.64 (1H, d, J 8 Hz), 7.86 (1H, d J 8 Hz) 8.39 (1H, s), 12.3 (1H, s); LC/MS: m/z 493 [M-H]⁻, Rₜ 4.20 min.

### Example 34 : 3-[3-(Methyloxy)-4-({3-[3-methyl-6-(trifluoromethyl)-1-benzothien-2-yl]heptyl}oxy)phenyl]propanoic acid

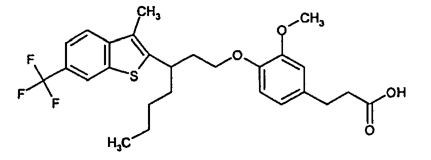

To a solution of methyl 3-[3-(methyloxy)-4-({3-[3-methyl-6-(trifluoromethyl)-1-benzothien-2-yl]heptyl}oxy)phenyl]propanoate (55 mg, 0.10 mmol) in MeOH (2 mL) and THF (2 mL) was added 2M sodium hydroxide (0.2 mL). After shaking for 3 h at 20°C, further 2M sodium hydroxide (0.2 mL) was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM and EtOAc to afford the title compound as a colourless oil (23 mg). ¹H NMR (400 MHz; DMSO-d₆) δ: 0.81 (3H, t, J 7 Hz), 1.06-1.36 (4H, m), 1.61 (1 H, m), 1.82 (1 H, m), 1.88 (1 H, m), 2.21 (1 H, m), 2.28 (3H, s), 2.48 (2H, t, J 7 Hz), 2.72 (2H, t, J 7 Hz), 3.50 (1 H, m), 3.67 (1 H, m), 3.73 (3H, s), 3.87 (1 H, m), 6.62 (1 H, dd, J 8 Hz, J 2 Hz), 6.71 (1 H, d, J 8 Hz), 6.83 (1H, d, J 2 Hz, 7.65 (1H, d, J 8 Hz), 7.85 (1 H, d J 8 Hz) 8.38 (1 H, s), 12.1 (1H, s); LC/MS: m/z 507 [M-H]⁻, Rₜ 4.25 min.

### Example 35 : [2-({3-[3-Methyl-6-(trifluoromethyl)-1-benzothien-2-yl]heptyl}thio)-1,3-thiazol-4-yl]acetic acid

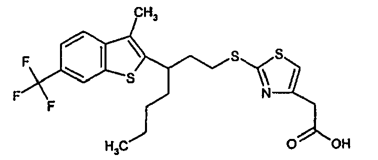

To a solution of ethyl [2-({3-[3-methyl-6-(trifluoromethyl)-1-benzothien-2-yl]heptyl}thio)-1,3-thiazol-4-yl]acetate (73 mg, 0.14 mmol) in MeOH (2 mL) and THF (2 mL) was added 2M sodium hydroxide (0.2 mL). After shaking for 3 h at 20°C, further 2M sodium hydroxide (0.2 mL was added and the mixture was shaken for a further 1 h. The solvent was evaporated and the residue was diluted with water (4mL), acidified with 2M HCl and extracted with EtOAc (2 x 3 mL). The organic extracts were filtered through a hydrophobic frit, evaporated to dryness, and purified through a 5 g silica SPE cartridge, eluting with DCM and EtOAc to afford the title compound as a colourless oil (22 mg). ¹H NMR (400 MHz; DMSO-d₆) δ: 0.80 (3H, t, J 7 Hz), 1.04-1.33 (4H, m), 1.57 (1 H, m), 1.77 (1 H, m), 1.97 (1 H, m), 2.15 (1 H, m), 2.36 (3H, s), 3.04 (1 H, m), 3.09 (1 H, m), 3.39 (1 H, m), 3.62 (2H, s), 7.35 (1 H, s), 7.68 (1 H, d, J 8 Hz), 7.89 (1 H, d J 8 Hz), 8.39 (1 H, s), 12.4 (1H, s); LC/MS: m/z 486 [M-H]⁻, Rₜ 4.29 min.

### Example 36 : [(4-{[3-(6-Fluoro-1-benzothien-2-yl)heptyl]oxy}-2-methylphenyl)oxy]acetic acid

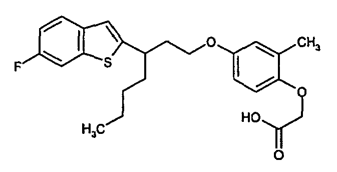

To a solution of ethyl [(4-{[3-(6-fluoro-1-benzothien-2-yl)heptyl]oxy}-2-methylphenyl)oxy]acetate (73 mg, 0.14 mmol) in MeOH (6 mL) was added 2M sodium hydroxide (0.2 mL). After stirring for 2 h at 60°C, the solvent was evaporated and the residue was diluted with water (6mL), acidified with 2M HCl and extracted with chloroform (2 x 5 mL). The organic extracts were filtered through a hydrophobic frit, evaporated to dryness, and purified through a 5 g silica SPE cartridge, eluting with chloroform, EtOAc and MeCN to afford the title compound as a colourless gum (14 mg). ¹H NMR (400 MHz; DMSO-d₆) δ: 0.82 (3H, t, J 7 Hz), 1.10-1.34 (4H, m), 1.62 (1 H, m), 1.75 (1 H, m), 1.93 (1H, m), 2.13 (1H, m), 2.12 (3H, s), 3.19 (1 H, m), 3.77 (1 H, m), 3.85 (1 H, m), 4.55 (2H, s), 6.60 (1 H, dd, J 9 Hz, 3Hz), 6.67 (1H, d, J 9 Hz), 6.69 (1H, d, J 3 Hz), 7.18 (1H, s), 7.19 (1H, dt, J 9, 2 Hz), 7.74 (1H, dd, J 9, 6 Hz), 7.81 (1 H, dd J 9, 2 Hz), -CO₂H not observed; LC/MS: m/z 429 [M-H]⁻, Rₜ 4.39 min.

### Example 37 : [(4-{[3-(6-Fluoro-3-methyl-1-benzothien-2-yl)heptyl]oxy}-2-methylphenyl)oxy]acetic acid

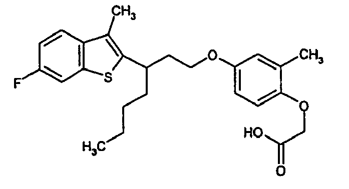

To a solution of ethyl [(4-{[3-(6-fluoro-3-methyl-1-benzothien-2-yl)heptyl]oxy}-2-methylphenyl)oxy]acetate (60 mg, 0.13 mmol) in MeOH (6 mL) was added 2M sodium hydroxide (0.2 mL). After stirring for 2 h at 60°C, the solvent was evaporated and the residue was diluted with water (6mL), acidified with 2M HCl and extracted with chloroform (2 x 5 mL). The organic extracts were filtered through a hydrophobic frit, evaporated to dryness, and purified through a 5 g silica SPE cartridge, eluting with chloroform, EtOAc and MeCN to afford the title compound as a colourless gum (15 mg). ¹H NMR (400 MHz; DMSO-d₆) δ: 0.81 (3H, t, J 7 Hz), 1.10-1.32 (4H, m), 1.59 (1H, m), 1.76 (1 H, m), 1.84 (1 H, m), 2.15 (1 H, m), 2.12 (3H, s), 2.21 (3H, s), 3.40 (1 H, m), 3.61 (1 H, m), 3.83 (1 H, m), 4.55 (2H, s), 6.59 (1 H, dd, J 9 Hz, 3Hz), 6.67 (1 H, d, J 9 Hz), 6.69 (1 H, d, J 3 Hz), 7.21 (1 H, dt, J 9 Hz, 2 Hz), 7.65 (1 H, dd, J 9 Hz, 6 Hz), 7.79 (1 H, dd J 9 Hz, 2 Hz), -CO₂H not observed; LC/MS: m/z 443 [M-H]⁻, Rₜ 4.52 min.

### Example 38 : {[4-({3-[3-Ethyl-5-(trifluoromethyl)-1-benzothien-2-yl]heptyl}oxy)-2-methylphenyl]oxy}acetic acid

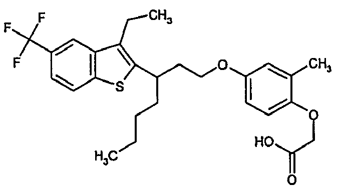

To a solution of ethyl {[4-({3-[3-ethyl-5-(trifluoromethyl)-1-benzothien-2-yl]heptyl}oxy)-2-methylphenyl]oxy}acetate (58 mg, 0.11 mmol) in MeOH (6 mL) was added 2M sodium hydroxide (0.2 mL). After stirring for 2 h at 60°C, the solvent was evaporated and the residue was diluted with water (6mL), acidified with 2M HCl and extracted with chloroform (2 x 5 mL). The organic extracts were filtered through a hydrophobic frit, evaporated to dryness, and purified through a 5 g silica SPE cartridge, eluting with chloroform, EtOAc and MeCN to afford the title compound as a colourless gum (13 mg). ¹H NMR (400 MHz; DMSO-d₆) δ: 0.81 (3H, t, J 7 Hz), 1.09 (3H, t, J 7 Hz), 1.20-1.35 (4H, m), 1.59 (1 H, m), 1.80 (1 H, m), 1.88 (1 H, m), 2.18 (1 H, m), 2.12 (3H, s), 3.44 (1 H, m), 3.67 (1 H, m), 3.84 (1 H, m), 4.54 (2H, s), 6.59 (1 H, dd, J 9 Hz, 3Hz), 6.67 (1 H, d, J 9 Hz), 6.69 (1 H, d, J 3 Hz), 7.60 (1 H, dd, J 9 Hz, 2 Hz), 8.00 (1H, d, J 2 Hz), 8.15 (1 H, d J 9 Hz), -CO₂H not observed; LC/MS: m/z 507 [M-H]⁻, Rₜ4.68 min.

### Example 39 : [(4-{[3-(5-Fluoro-3-methyl-1-benzothien-2-yl)heptyl]oxy}-2-methylphenyl)oxy]acetic acid

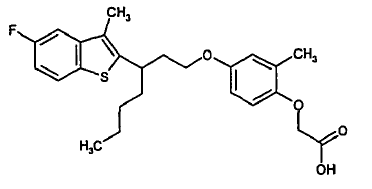

To a solution of ethyl [(4-{[3-(5-fluoro-3-methyl-1-benzothien-2-yl)heptyl]oxy}-2-methylphenyl)oxy]acetate (25 mg, 0.05 mmol) in MeOH (4 mL) was added 2M sodium hydroxide (0.15 mL). After stirring for 2 h at 60°C, the solvent was evaporated and the residue was diluted with water (10 mL), acidified with 2M HCl and extracted with EtOAc (2 x 15 mL). The organic extracts were washed with water and brine, dried over MgSO₄, and evaporated. The residue was purified through a 2 g silica SPE cartridge, eluting with DCM, EtOAc, MeCN and MeOH to afford the title compound as a colourless gum (21 mg). ¹H NMR (400 MHz; DMSO-d₆) δ: 0.81 (3H, t, J 7 Hz), 1.20-1.35 (4H, m), 1.59 (1 H, m), 1.78 (1 H, m), 1.83 (2H, m), 2.12 (3H, s), 2.20 (3H, s), 3.00 (1 H, m), 3.61 (1 H, m), 3.82 (1 H, m), 4.53 (2H, s), 6.59 (1 H, m), 6.65 (1H, s), 6.69 (1H, d, J 3 Hz), 7.17 (1H, dt, J 9 Hz, 2 Hz), 7.47 (1H, dd, J 9 Hz, 2 Hz), 7.90 (1 H, dd, J 9 Hz, 6 Hz), -CO₂H not observed; LC/MS: m/z 443 [M-H]⁻, Rₜ4.48 min.

### Example 40 : [(4-{[3-(6-Fluoro-3-methyl-1-benzothien-2-yl)heptyl]thio}-2-methylphenyl)oxy]acetic acid

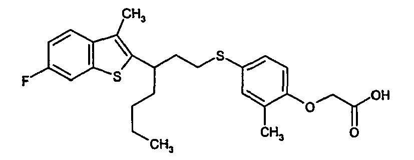

To a solution of ethyl [(4-{[3-(6-fluoro-3-methyl-1-benzothien-2-yl)heptyl]thio}-2-methylphenyl)oxy]acetate (55 mg, 0.11 mmol) in MeOH (4 mL) was added 2M sodium hydroxide (0.15 mL). After stirring for 2 h at 60°C, the solvent was evaporated and the residue was diluted with water (20 mL), acidified with 2M HCl and extracted with EtOAc (2 x 15 mL). The organic extracts were washed with water and brine, dried over MgSO₄, and evaporated. The residue was purified through a 2 g silica SPE cartridge, eluting with DCM, EtOAc, and MeCN to afford the title compound as a colourless gum (36 mg). ¹H NMR (400 MHz; DMSO-d₆) δ: 0.79 (3H, t, J 7 Hz), 1.05-1.30 (4H, m), 1.49 (1 H, m), 1.67 (1 H, m), 1.73 (1 H, m), 1.89 (1 H, m), 2.11 (3H, s), 2.30 (3H, s), 2.33 (1 H, m), 2.69 (1 H, m), 2.73 (1 H, m), 4.66 (2H, s), 6.74 (1 H, d, J 8 Hz), 7.08 (1 H, d, J 2 Hz), 7.10 (1 H, s), 7.24 (1 H, dt, J 8 Hz, 2 Hz), 7.66 (1 H, dd, J 8 Hz, 6 Hz), 7.79 (1 H, dd, J 8 Hz, 2 Hz), -CO₂H not observed; LC/MS: m/z 459 [M-H]⁻, Rₜ 4.76 min.

### Example 41 : {[2-Methyl-4-({3-[3-methyl-5-(trifluoromethyl)-1-benzothien-2-yl]heptyl}thio)phenyl]oxy}acetic acid

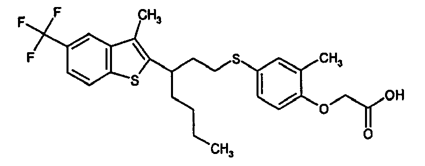

To a solution of ethyl {[2-methyl-4-({3-[3-methyl-5-(trifluoromethyl)-1-benzothien-2-yl]heptyl}thio)phenyl]oxy}acetate (54 mg, 0.10 mmol) in MeOH (3 mL) was added 2M sodium hydroxide (0.20 mL). After stirring for 1 h at 60°C the solvent was evaporated and the residue was diluted with water (10mL), acidified with 2M HCl and extracted with DCM (10 mL). The organic extracts were washed with water, eluted through a hydrophobic frit and evaporated to afford the title compound as a white solid (35 mg). ¹H NMR (400 MHz; DMSO-d₆) δ: 0.79 (3H, t, J 7 Hz), 1.05-1.30 (4H, m), 1.53 (1 H, m), 1.70 (1 H, m), 1.76 (1 H, m), 1.92 (1 H, m), 2.10 (3H, s), 2.37 (3H, s), 2.70 (2H, m), 3.38 (1 H, m), 4.46 (2H, s), 6.69 (1 H, d, J 8 Hz), 7.08 (1 H, m), 7.09 (1H, m), 7.60 (1 H, d, J 8 Hz), 8.02 (1 H, s), 8.14 (1 H, d, J 8 Hz), -CO₂H not observed; LC/MS: m/z 509 [M-H]⁻, Rₜ 4.88 min.

### Example 42 : {[2-Methyl-4-({3-[3-methyl-6-(trifluoromethyl)-1-benzothien-2-yl]heptyl}oxy)phenyl]oxy}acetic acid (isomer 1)

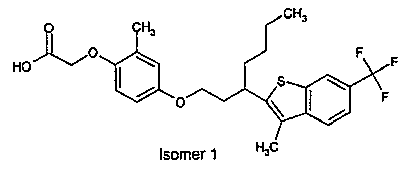

To a solution of ethyl {[2-methyl-4-({3-[3-methyl-6-(trifluoromethyl)-1-benzothien-2-yl]heptyl}oxy)phenyl]oxy}acetate (isomer 1) (45 mg, 0.09 mmol) in MeOH (2 mL) and THF (2 mL) was added 2M sodium hydroxide (0.2 mL). After stirring for 1 h at 20°C the solvent was evaporated and the residue was diluted with water (50mL), acidified with 2M HCl and extracted with EtOAc (3 x 50 mL). The organic extracts were washed with water, dried over MgSO₄, evaporated to dryness and eluted through a 500 mg silica SPE cartridge, with DCM and EtOAc to afford the title compound as a colourless gum (29 mg).¹H NMR (400 MHz; DMSO-d₆) δ: 0.81 (3H, t, J 7 Hz), 1.07-1.36 (4H, m), 1.62 (1 H, m), 1.80 (1 H, m), 1.87 (1 H, m), 2.10 (3H, s), 2.20 (1 H, m),, 2.27 (3H, s), 3.48 (1 H, m), 3.61 (1 H, m), 3.83 (1 H, m), 4.56 (2H, s), 6.58 (1 H, dd, J 8 Hz, 3Hz), 6.67 (1 H, d, J 3 Hz), 6.66 (1 H, d, J 8 Hz), 7.64 (1 H, d, J 8 Hz), 7.85 (1 H, d, J 8 Hz), 8.38 (1 H, s), -CO₂H not observed; LC/MS: m/z 493 [M-H]⁻, Rₜ 4.77 min.

### Example 43 : {[2-Methyl-4-({3-[3-methyl-6-(trifluoromethyl)-1-benzothien-2-yl]heptyl}oxy)phenyl]oxy}acetic acid (Isomer 2)

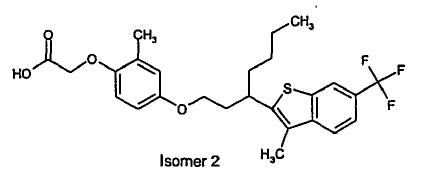

To a solution of ethyl {[2-methyl-4-({3-[3-methyl-6-(trifluoromethyl)-1-benzothien-2-yl]heptyl}oxy)phenyl]oxy}acetate (isomer 2) (45 mg, 0.09 mmol) in MeOH (2 mL) and THF (2 mL) was added 2M sodium hydroxide (0.2 mL). After stirring for 1 h at 20°C the solvent was evaporated and the residue was diluted with water (50mL), acidified with 2M HCl and extracted with EtOAc (3 x 50 mL). The organic extracts were washed with water, dried over MgSO₄, evaporated to dryness and eluted through a 500 mg silica SPE cartridge, with DCM and EtOAc to afford the title compound as a colourless gum (35 mg). ¹H NMR (400 MHz; DMSO-d₆) δ: 0.81 (3H, t, J 7 Hz), 1.07-1.36 (4H, m), 1.62 (1 H, m), 1.80 (1 H, m), 1.87 (1 H, m), 2.10 (3H, s), 2.20 (1 H, m), 2.27 (3H, s), 3.48 (1 H, m), 3.61 (1 H, m), 3.83 (1 H, m), 4.56 (2H, s), 6.58 (1 H, dd, J 8 Hz, 3Hz), 6.67 (1 H, d, J 3 Hz), 6.66 (1 H, d, J 8 Hz), 7.64 (1H, d, J 8 Hz), 7.85 (1 H, d, J 8 Hz), 8.38 (1 H, s), -CO₂H not observed; LC/MS: m/z 493 [M-H]⁻, Rₜ 4.75 min.

### Example 44 : [4-Methyl-2-({3-[3-methyl-6-(trifluoromethyl)-1-benzothien-2-yl]heptyl}thio)-1,3-thiazol-5-yl]acetic acid (isomer 1)

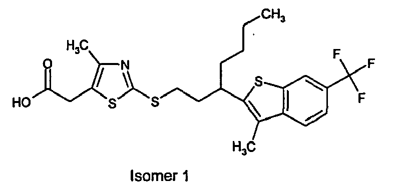

To a solution of ethyl [4-methyl-2-({3-[3-methyl-6-(trifluoromethyl)-1-benzothien-2-yl]heptyl}thio)-1,3-thiazol-5-yl]acetate (isomer 1) (30 mg, 0.06 mmol) in MeOH (2 mL) and THF (2 mL) was added 2M sodium hydroxide (0.2 mL). After stirring for 1 h at 20°C the solvent was evaporated and the residue was diluted with water (20 mL), acidified with 2M HCl and extracted with EtOAc (2 x 20 mL). The organic extracts were washed with water, filtered through a hydrophobic frit, evaporated to dryness and eluted through a 2 g silica SPE cartridge, with DCM and EtOAc to afford the title compound as a colourless gum (20 mg). ¹H NMR (400 MHz; DMSO-d₆) δ: 0.80 (3H, t, J 7 Hz), 1.05-1.32 (4H, m), 1.56 (1 H, m), 1.77 (1 H, m), 1.95 (1 H, m), 2.14 (1H, m), 2.14 (3H, s), 2.36 (3H, s), 3.00 (1 H, m), 3.07 (1 H, m), 3.39 (1 H, m), 3.72 (2H, s), 7.67 (1 H, d, J 8 Hz), 7.89 (1 H, d, J 8 Hz), 8.39 (1 H, s) 12.7 (1 H, s); LC/MS: m/z 502 [M+H]⁺, Rₜ 4.48 min.

### Example 45 : [4-Methyl-2-({3-[3-methyl-6-(trifluoromethyl)-1-benzothien-2-yl]heptyl}thio)-1,3-thiazol-5-yl]acetic acid (Isomer 2)

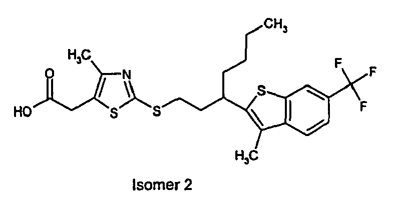

To a solution of ethyl [4-methyl-2-({3-[3-methyl-6-(trifluoromethyl)-1-benzothien-2-yl]heptyl}thio)-1,3-thiazol-5-yl]acetate (isomer 2) (30 mg, 0.06 mmol) in MeOH (2 mL) and THF (2 mL) was added 2M sodium hydroxide (0.2 mL). After stirring for 1 h at 20°C the solvent was evaporated and the residue was diluted with water (20 mL), acidified with 2M HCl and extracted with EtOAc (2 x 20 mL). The organic extracts were washed with water, filtered through a hydrophobic frit, evaporated to dryness and eluted through a 2 g silica SPE cartridge with DCM and EtOAc to afford the title compound as a colourless gum (19 mg). ¹H NMR (400 MHz; DMSO-d₆) δ: 0.80 (3H, t, J 7 Hz), 1.05-1.32 (4H, m), 1.56 (1 H, m), 1.77 (1 H, m), 1.95 (1H, m), 2.14 (1 H, m), 2.14 (3H, s), 2.36 (3H, s), 3.00 (1 H, m), 3.07 (1H, m), 3.39 (1H, m), 3.72 (2H, s), 7.67 (1 H, d, J 8 Hz), 7.89 (1 H, d, J 8 Hz), 8.39 (1 H, s) 12.7 (1H, s); LC/MS: m/z 502 [M+H]⁺, Rₜ 4.49 min.

### Example 46 : (2-{[3-(3,6-Dimethyl-1-benzofuran-2-y)heptyl]thio}-4-methyl-1,3-thiazol-5-yl)acetic acid

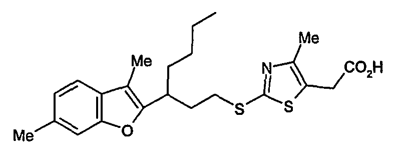

To a solution of ethyl (2-{[3-(3,6-dimethyl-1-benzofuran-2-yl)heptyl]thio}-4-methyl-1,3-thiazol-5-yl)acetate (57 mg, 0.12 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (40 mg). ¹H NMR (400 MHz; DMSO-d₆) δ: 0.79 (3H, t, J 7 Hz), 1.00-1.30 (4H, m), 1.65 (2H, m), 2.03 (2H, m), 2.12 (3H, s), 2.15 (3H, s), 2.39 (3H, s), 2.87-3.10 (3H, m), 3.70 (2H, s), 7.03 (1H, d, J 8 Hz), 7.27 (1H, s), 7.36 (1H, d, J 8 Hz), 12.7 (1 H, s); LC/MS: m/z 432 [M+H]⁺, Rₜ 4.29 min.

### Example 47 : [(4-{[3-(3,6-Dimethyl-1-benzofuran-2-yl)heptyl]oxy}-2-methylphenyl)oxy]acetic acid

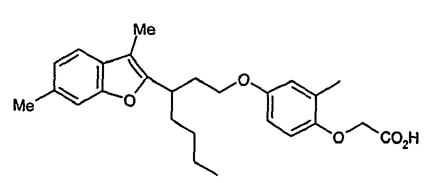

To a solution of ethyl [(4-{[3-(3,6-dimethyl-1-benzofuran-2-yl)heptyl]oxy}-2-methylphenyl)oxy]acetate (54 mg, 0.12 mmol) in MeOH (3 mL) and THF (3 mL) was added 21 sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (35 mg). ¹H NMR (400 MHz; DMSO-d₆) δ: 0.80 (3H, t, J 7 Hz), 1.00-1.32 (4H, m), 1.60-1.78 (2H, m), 1.93-2.09 (2H, m), 2.03 (3H, s), 2.11 (3H, s), 2.39 (3H, s), 3.15 (1 H, m), 3.58 (1 H, m), 3.79 (1 H, m), 4.57 (2H, s), 6.56 (1H, dd, J 9 Hz, 3 Hz), 6.65 (1 H, d, J 9 Hz), 6.65 (1H, s), 7.01 (1 H, d, J 8 Hz), 7.27 (1 H, s), 7.32 (1H, d, J 8 Hz), 12.9 (1 H, s); LC/MS: m/z 425 [M+H]⁺, Rₜ 4.61 min.

### Example 48 : [(4-{[3-(3,6-Dimethyl-1-benzofuran-2-yl)heptyl]thio}-2-methylphenyl)oxy]acetic acid

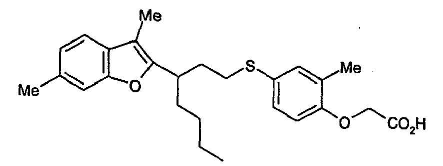

To a solution of ethyl [(4-{[3-(3,6-dimethyl-1-benzofuran-2-yl)heptyl]thio}-2-methylphenyl)oxy]acetate (83 mg, 0.19 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (60 mg). ¹H NMR (400 MHz; DMSO-d₆) δ: 0.78 (3H, t, J 7 Hz), 0.98-1.30 (4H, m), 1.53-1.69 (2H, m), 1.76-1.95 (2H, m), 2.10 (3H, s), 2.12 (3H, s), 2.39 (3H, s), 2.58-2.73 (2H, m), 3.07 (1 H, m), 4.66 (2H, s), 6.72 (1 H, d, J 8 Hz), 7.02 (1 H, d, J 8 Hz), 7.07 (2H, d, J 8 Hz), 7.26 (1H, s), 7.35 (1H, d, J 8 Hz), 13.0 (1H, s); LC/MS: m/z 441 [M+H]⁺, Rₜ 4.86 min.

### Example 49 : [4-Methyl-2-({3-[3-methyl-6-(trifluoromethyl)-1-benzofuran-2-yl]heptyl}thio)-1,3-thiazol-5-yl]acetic acid

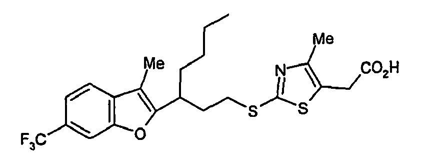

To a solution of ethyl [4-methyl-2-({3-[3.methyl-6-(trifluoromethyl)-1-benzofuran-2-yl]heptyl}thio)-1,3-thiazol-5-yl]acetate (67 mg, 0.13 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (40 mg). ¹H NMR (400 MHz; DMSO-d₆) δ: 0.80 (3H, t, J 7 Hz), 1.02-1.30 (4H, m), 1.69 (2H, m), 2.07 (2H, m), 2.11 (3H, s), 2.20 (3H, s), 2.92-3.08 (2H, m), 3.16 (1 H, m), 3.70 (2H, s), 7.55 (1 H, d, J 8 Hz), 7.73 (1 H, d, J 8 Hz), 7.92 (1H, s), 12.7 (1 H, s); LC/MS: m/z 486 [M+H]⁺, Rₜ 4.33 min.

### Example 50 : {[2-Methyl-4-({3-[3-methyl-6-(trifluoromethyl)-1-benzofuran-2-yl]heptyl}oxy)phenyl]oxy}acetic acid

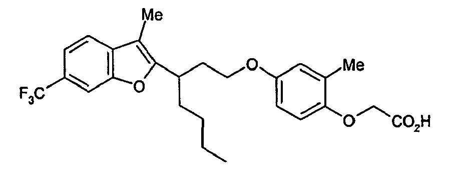

To a solution of ethyl {[2-methyl-4-({3-[3-methyl-6-(trifluoromethyl)-1-benzofuran-2-yl]heptyl}oxy)phenyl]oxy}acetate (64 mg, 0.13 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (45 mg). ¹H NMR (400 MHz; DMSO-d₆) δ: 0.80 (3H, t, J 7 Hz), 1.00-1.32 (4H, m), 1.68-1.80 (2H, m), 1.98-2.15 (2H, m), 2.09 (3H, s), 2.10 (3H, s), 3.24 (1 H, m), 3.61 (1 H, m), 3.81 (1 H, m), 4.57 (2H, s), 6.56 (1 H, dd, J 9 Hz, 3 Hz), 6.60 (1 H, d, J 3 Hz), 6.66 (1 H, d, J 9 Hz), 7.54 (1 H, d, J 8 Hz), 7.69 (1 H, d, J 8 Hz), 7.92 (1H, s), 12.9 (1 H, s); LC/MS: m/z 477 [M-H]⁻, Rₜ 4.69 min.

### Example 51 : {[2-Methyl-4-({3-[3-methyl-6-(trifluoromethyl)-1-benzofuran-2-yl]heptyl}thio)phenyl]oxy}acetic acid

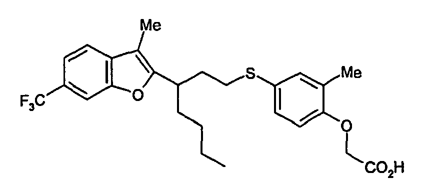

To a solution of ethyl {[2-methyl-4-({3-[3-methyl-6-(tritluoromethyl)-1-benzofuran-2-yl]heptyl}thio)phenyl]oxy}acetate (76 mg, 0.15 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were filtered through a hydrophobic frit, evaporated to dryness and purified through a 2 g silica SPE cartridge, eluting with DCM, EtOAc, MeCN and MeOH, to afford the title compound as a colourless gum (60 mg).¹H NMR (400 MHz; DMSO-d₆) δ: 0.78 (3H, t, J 7 Hz), 0.97-1.30 (4H, m), 1.58-1.71 (2H, m), 1.90-1.96 (2H, m), 2.09 (3H, s), 2.19 (3H, s), 2.62-2.76 (2H, m), 3.17 (1 H, m), 4.66 (2H, s), 6.73 (1 H, d, J 9 Hz), 7.07 (1H, d, J 9 Hz), 7.07 (1H, s), 7.55 (1H, d J 8 Hz), 7.72 (1H, d, J 8 Hz), 7.90 (1H, s), 13.0 (1H, s); LC/MS: m/z 493 [M-H]⁻, Rₜ 4.88 min.

### Example 52 : [4-Methyl-2-({5-(methyloxy)-3-[3-methyl-6-(trifluoromethyl)-1-benzothien-2-yl]pentyl}thio)-1,3-thiazol-5-yl]acetic acid

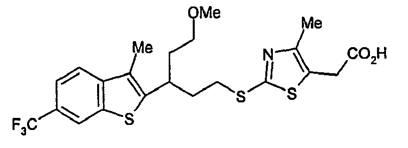

To a solution of ethyl [4-methyl-2-({5-(methyloxy)-3-[3-methyl-6-(trifluoromethyl)-1-benzothien-2-yl]pentyl}thio)-1,3-thiazol-5-yl]acetate (60 mg, 0.11 mmol) in MeOH (3 mL) and THF (3 mL) was added 2M sodium hydroxide (0.6 mL). After stirring for 2.5 h at 20°C the solvent was evaporated and the residue was diluted with water (10 mL), acidified with 2M HCl and extracted with EtOAc (2x10 mL). The organic extracts were washed with water (10 mL) and brine (10 mL), filtered through a hydrophobic frit and evaporated to dryness to afford the title compound as a colourless gum (49 mg). ¹H NMR (400 MHz; DMSO-d₆) δ: 1.74 (1H, m), 1.94-2.08 (2H, m), 2.17 (1 H, m), 2.14 (3H, s), 2.35 (3H, s), 2.94-3.04 (2H, m), 3.04-3.15 (1 H, m), 3.16 (3H, s), 3.25 (1 H, m), 3.56 (1 H, m), 3.72 (2H, s), 7.68 (1 H, d, J 8 Hz), 7.90 (1 H, d, J 8 Hz), 8.40 (1 H, s), 12.7 (1 H, s); LC/MS: m/z 504 [M+H]⁺, Rₜ 3.99 min.

### Example 53 : {[2-Methyl-4-({5-(methyloxy)-3-[3-methyl-6-(trifluoromethyl)-1-benzothien-2-yl]pentyl}thio)phenyl]oxy}acetic acid

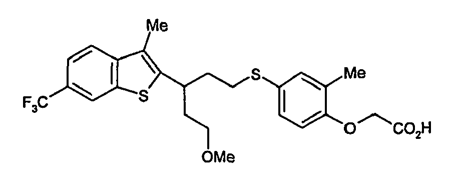

To a solution of ethyl {[2-methyl-4-({5-(methyloxy)-3-[3-methyl-6-(trifluoromethyl)-1-benzothien-2-yl]pentyl}thio)phenyl]oxy}acetate (75 mg, 0.14 mmol) in MeOH (3 mL) and THF (3 mL) was added 2M sodium hydroxide (0.6 mL). After stirring for 2.5 h at 20°C the solvent was evaporated and the residue was diluted with water (10 mL), acidified with 2M HCl and extracted with EtOAc (2x10 mL). The organic extracts were washed with water (10 mL) and brine (10 mL), filtered through a hydrophobic frit and evaporated to dryness to afford the title compound as a colourless gum (70 mg).¹H NMR (400 MHz; DMSO-d₆) δ: 1.70 (1 H, m), 1.82 (1H, m), 1.90-2.04 (2H, m), 2.10 (3H, s), 2.34 (3H, s), 2.65-2.81 (2H, m), 3.08 (1 H, m), 3.15 (3H, s), 3.22 (1 H, m), 3.57 (1H, m), 4.67 (2H, s), 6.74 (1 H, d, J 8 Hz), 7.10 (1 H, d, J 8 Hz), 7.10 (1 H, s), 7.67 (1 H, d J 8 Hz), 7.90 (1 H, d, J 8 Hz), 8.39 (1 H, s), 13.0 (1 H, s); LC/MS: m/z 511 [M-H]⁻, Rₜ4.48 min.

### Example 54 : {[2-Methyl-4-({5-(methyloxy)-3-[3-methyl-6-(trifluoromethyl)-1-benzothien-2-yl]pentyl}oxy)phenyl]oxy}acetic acid

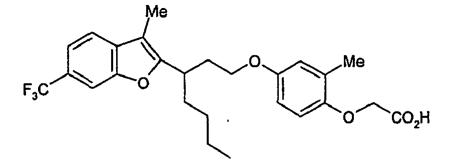

To a solution of ethyl {[2-methyl-4-({5-(methyloxy)-3-[3-methyl-6-(trifluoromethyl)-1-benzothien-2-yl]pentyl}oxy)phenyl]oxy}acetate (51 mg, 0.10 mmol) in MeOH (3 mL) and THF (3 mL) was added 2M sodium hydroxide (0.6 mL). After stirring for 2.5 h at 20°C the solvent was evaporated and the residue was diluted with water (10 mL), acidified with 2M HCl and extracted with EtOAc (2x10 mL). The organic extracts were washed with water (10 mL) and brine (10 mL), filtered through a hydrophobic frit and evaporated to dryness to afford the tittle compound as a colourless gum (45 mg).¹H NMR (400 MHz; DMSO-d₆) δ: 1.79 (1 H, m), 1.91 (1 H, m), 2.07 (1 H, m), 2.21 (1 H, m), 2.10 (3H, s), 2.27 (3H, s), 3.11 (1 H, m), 3.16 (3H, s), 3.27 (1H, m), 3.57-3.70 (2H, m), 3.85 (1 H, m), 4.57 (2H, s), 6.58 (1 H, dd, J 8 Hz, 3 Hz), 6.66 (1 H, d, J 8 Hz), 6.67 (1H, d J 3 Hz), 7.65 (1H, d, J 8 Hz), 7.86 (1H, d, J 8 Hz), 8.40 (1H, s), 12.9 (1H, s); LC/MS: m/z 495 [M-H]⁻, Rₜ 4.25 min.

### Example 55 : {[2-Ethyl-4-({5-(methyloxy)-3-[3-methyl-6-(trifluoromethyl)-1-benzothien-2-yl]pentyl}oxy)phenyl]oxy}acetic acid

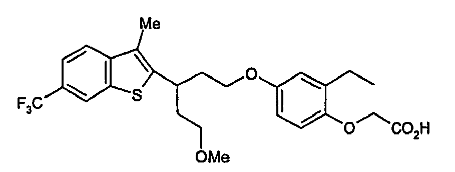

To a solution of ethyl {[2-ethyl-4-({5-(methyloxy)-3-[3-methyl-6-(trifluoromethyl)-1-benzothien-2-yl]pentyl}oxy)phenyl]oxy}acetate (45 mg, 0.08 mmol) in MeOH (3 mL) and THF (3 mL) was added 2M sodium hydroxide (0.6 mL). After stirring for 2.5 h at 20°C the solvent was evaporated and the residue was diluted with water (10 mL), acidified with 2M HCl and extracted with EtOAc (2x10 mL). The organic extracts were washed with water (10 mL) and brine (10 mL), filtered through a hydrophobic frit and evaporated to dryness to afford the title compound as a colourless gum (38 mg). ¹H NMR (400 MHz; DMSO-d₆) δ: 1.07 (3H, t, J 7 Hz), 1.79 (1 H, m), 1.91 (1 H, m), 2.07 (1 H, m), 2.21 (1 H, m), 2.27 (3H, s), 2.51 (2H, q, J 7 Hz), 3.11 (1H, m), 3.16 (3H, s), 3.27 (1 H, m), 3.59-3.70 (2H, m), 3.85 (1 H, m), 4.57 (2H, s), 6.59 (1 H, dd, J 8 Hz, 3 Hz), 6.65 (1 H, d, J 3 Hz), 6.67 (1 H, d J 8 Hz), 7.65 (1 H, d, J 8 Hz), 7.86 (1H, d, J 8 Hz), 8.40 (1 H, s), 12.9 (1 H, s); LC/MS: m/z 509 [M-H]⁻, Rₜ 4.29 min.

### Example 56 : [(2-Methyl-4-{[3-[3-methyl-6-(trifluoromethyl)-1-benzothien-2-yl]-3-(propyloxy)propyl]thio}phenyl)oxy]acetic acid

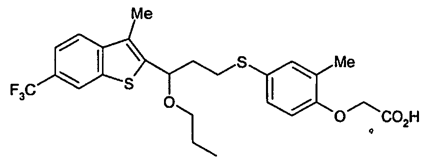

To a solution of ethyl [(2-methyl-4-{[3-[3-methyl-6-(trifluoromethyl)-1-benzothien-2-yl]-3-(propyloxy)propyl]thio}phenyl)oxy]acetate (70 mg, 0.14 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were washed with water (20 mL) and brine (20 mL), filtered through a hydrophobic frit and evaporated to dryness to afford the title compound as a colourless gum (56 mg). ¹H NMR (400 MHz; DMSO-d₆) δ: 0.85 (3H, t, J 7 Hz), 1.50 (2H, m), 1.84 (1 H, m), 2.02 (1 H, m), 2.13 (3H, s), 2.34 (3H, s), 2.94 (2H, m), 3.30 (2H, m), 4.66 (2H, s), 4.99 (1H, m), 6.77 (1H, d, J 8 Hz), 7.16 (1H, dd, J 8 Hz, 2 Hz), 7.20 (1H, d, J 2 Hz), 7.68 (1 H, d J 8 Hz), 7.91 (1 H, d, J 8 Hz), 8.41 (1 H, s), CO₂H not observed; LC/MS: m/z 511 [M-H]⁻, Rₜ 4.75 min.

### Example 57 : [(2-Methyl-4-{[3-[3-methyl-6-(trifluoromethyl)-1-benzothien-2-yl]-3-(propyloxy)propyl]oxy}phenyl)oxy]acetic acid

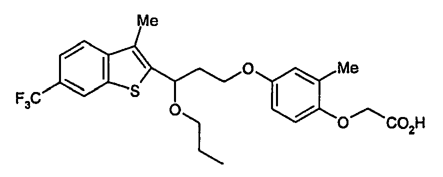

To a solution of ethyl [(2-methyl-4-{[3-[3-methyl-6-(trifluoromethyl)-1-benzothien-2-yl]-3-(propyloxy)propyl]oxy}phenyl)oxy]acetate (70 mg, 0.14 mmol) in MeOH (3 mL) and THF (3 mL) was added 2M sodium hydroxide (0.6 mL). After stirring for 3 h at 20°C the solvent was evaporated and the residue was diluted with water (20 mL), acidified with 2M HCl and extracted with EtOAc (2 x 20 mL). The organic extracts were washed with water (20 mL) and brine (20 mL), filtered through a hydrophobic frit and evaporated to dryness to afford the title compound as a colourless gum (60 mg). ¹H NMR (400 MHz; DMSO-d₆) δ: 0.85 (3H, t, J 7 Hz), 1.51 (2H, m), 2.05 (1 H, m), 2.14 (3H, s), 2.23 (1 H, m), 2.34 (3H, s), 3.39 (2H, m), 3.82 (1 H, m), 4.03 (1H, m), 4.59 (2H, s), 5.07 (1 H, m), 6.67 (1 H, dd, J 8 Hz, 3 Hz), 6.72 (1 H, d, J 8 Hz), 6.74 (1 H, d, J 3 Hz), 7.68 (1 H, d, J 8 Hz), 7.92 (1 H, d J 8 Hz), 8.43 (1 H, s), O₂H not observed; LC/MS: m/z 495 [M-H]⁻, Rₜ 4.52 min.

### Example 58 : [2-({3-[3-Methyl-6-(methyloxy)-1-benzothien-2-yl]heptyl}thio)-1,3-thiazol-4-yl]acetic acid (isomer 1)

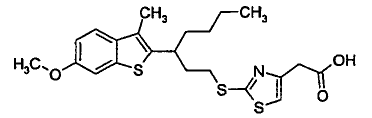

To a solution of ethyl [2-({3-[3-methyl-6-(methyloxy)-1-benzothien-2-yl]heptyl}thio)-1,3-thiazol-4-yl]acetate (isomer 1) (69 mg) in MeOH (3 mL) and THF (3 mL), 2M sodium hydroxide (0.6 mL) was added. The reaction was stirred at room temperature for 3 hours. The solvent was evaporated, water (2 mL) was added followed by a few drop of 2M HCl then the solution was extracted with EtOAc (2 x 3 mL). The organics were combined and washed with brine, separated, dried through a hydrophobic frit and evaporated. The product was isolated after freeze drying with 1,4-dioxane (2 mL) and water (2 drops) as a yellow oil (55 mg). ¹H NMR (400 MHz, DMSO-d₆) δ: 12.44 (1 H, s), 7.55 (1 H, d, J 8.9 Hz), 7.45 (1 H, d, J 2.3 Hz), 7.35 (1 H, s), 6.98 (1H, dd, J 8.9, J 2.3 Hz), 3.80 (3H, s), 3.63 (2H, s), 3.31-3.22 (1 H, m), 3.14-2.94 (2H, m), 2.26 (3H, s), 2.15-2.03 (1 H, m), 1.97-1.85 (1H, m), 1.77-1.65 (1H, m), 1.57-1.45 (1H, m), 1.33-1.04 (4H, m), 0.80 (3H, m). LC/MS: m/z 550 [M+H]⁺, m/z 548 [M-H]⁻, Rₜ 4.13 min.

### Example 59 : [2-({3-[3-Methyl-6-(methyloxy)-1-benzothien-2-yl]heptyl}thio)-1,3-thiazol-4-yl]acetic acid (isomer 2)

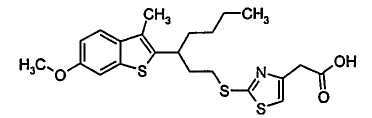

This compound was prepared from ethyl [2-({3-[3-methyl-6-(methyloxy)-1-benzothien-2-yl]heptyl}thio)-1,3-thiazol-4-yl]acetate (isomer 2) by the same procedure used for Example 58. ¹H NMR (400 MHz, DMSO-d₆) δ: 12.44 (1H, s), 7.55 (1H, d, J 8.9 Hz), 7.45 (1 H, d, J 2.3 Hz), 7.35 (1 H, s), 6.98 (1 H, dd, J 8.9, J 2.3 Hz), 3.80 (3H, s), 3.63 (2H, s), 3.31-3.22 (1 H, m), 3.14-2.94 (2H, m), 2.26 (3H, s), 2.15-2.03 (1 H, m), 1.97-1.85 (1 H, m), 1.77-1.65 (1H, m), 1.57-1.45 (1 H, m), 1.33-1.04 (4H, m), 0.80 (3H, m). LC/MS: m/z 550 [M+H]⁺, m/z 548 [M-H]⁻, Rₜ 4.13 min.

### Example 60 : {[2-Methyl-4-({3-[3-methyl-6-(methyloxy)-1-benzothien-2-yl]heptyl}oxy)phenyl]oxy}acetic acid (isomer 1)

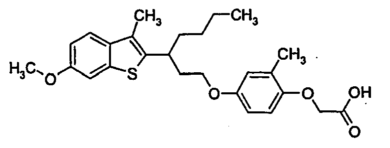

This compound was prepared from ethyl {[2-methyl-4-({3-[3-methyl-6-(methyloxy)-1-benzothien-2-yl]heptyl}oxy)phenyl]oxy}acetate (isomer 1) by the same procedure used for Example 58. ¹H NMR (400 MHz, DMSO-d₆) δ: 13.20-12.85 (1H, s), 7.51 (1H, d, J 8.8 Hz), 7.45 (1 H, d, J 2.3 Hz), 6.95 (1 H, dd, J 8.8, 2.3 Hz), 6.69 (1 H, d, J 3.7 Hz), 6.67 (1 H, J 9.0 Hz), 6.58 (1 H, dd, J 2.8, 8.8 Hz), 4.56 (2H, s), 3.86-3.79 (1H, m), 3.79 (3H, s), 3.65-3.58 (1 H, m), 3.41-3.33 (1 H, m), 2.16 (3H, s), 2.11 (3H, s), 2.11 (1 H), 1.86-1.68 (2H, m), 1.63-1.49 (1 H, m), 1.37-1.07 (4H, m), 0.81 (3H, m). LC/MS: m/z 457 [M+H]⁺, m/z 455 [M-H]⁻, Rₜ 4.52 min.

### Example 61 : {[2-Methyl-4-({3-[3-methyl-6-(methyloxy)-1-benzothien-2-yl]heptyl}oxy)phenyl]oxy}acetic acid (isomer 2)

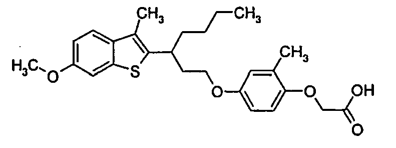

This compound was prepared from ethyl {[2-methyl-4-({3-[3-methyl-6-(methyloxy)-1-benzothien-2-yl]heptyl}oxy)phenyl]oxy}acetate (isomer 2) by the same procedure used for Example 58. ¹H NMR (400 MHz, DMSO-d₆) δ: 13.20-12.85 (1H, s), 7.51 (1 H, d, J 8.8 Hz), 7.45 (1H, d, J 2.3 Hz), 6.95 (1H, dd, J 8.8, 2.3 Hz), 6.69 (1H, d, J 3.7 Hz), 6.67 (1H, J 9.0 Hz), 6.58 (1H, dd, J 2.8, 8.8 Hz), 4.56 (2H, s), 3.86-3.79 (1 H, m), 3.79 (3H, s), 3.65-3.58 (1 H, m), 3.41-3.33 (1H, m), 2.16 (3H, s), 2.11 (3H, s), 2.11 (1 H), 1.86-1.68 (2H, m), 1.63-1.49 (1 H, m), 1.37-1.07 (4H, m), 0.81 (3H, m). LC/MS: m/z 457 [M+H]⁺, m/z 455 [M-H]⁻, Rₜ4.52 min.

### Example 62 : [4-({3-[3-Methyl-6-(methyloxy)-1-benzothien-2-yl]heptyl}oxy)-3-(methyloxy)phenyl]acetic acid (isomer 1)

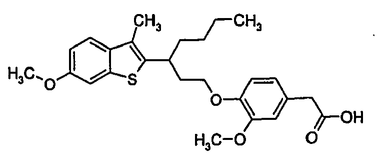

This compound was prepared from ethyl [4-({3-[3-methyl-6-(methyloxy)-1-benzothien-2-yl]heptyl}oxy)-3-(methyloxy)phenyl]acetate (isomer 1) by the same procedure used for Example 58. ¹H NMR (400 MHz, DMSO-d₆) δ: 12.23 (1H, s), 7.51 (1 H, d, J 8.8 Hz), 7.45 (1 H, d, J 2.3 Hz), 6.95 (1 H, dd, J 8.8, J 2.3 Hz), 6.85 (1 H, d, J 1.5 Hz), 6.74 (1 H, J 8.3 Hz), 6.66 (1 H, dd, J 1.5, J 8.3 Hz), 3.90-3.81 (1 H, m), 3.79 (3H, s), 3.74 (3H, s), 3.71-3.64 (1 H, m), 3.45 (2H, s), 3.45-3.37 (1 H, m), 2.17 (3H, s), 2.17-2.11 (1 H, m), 1.89-1.70 (2H, m), 1.62-1.50 (1H, m), 1.37-1.07 (4H, m), 0.81 (3H, m). LC/MS: m/z 457 [M+H]⁺, m/z 455 [M-H]⁻, Rₜ 3.94 min.

### Example 63 : [4-({3-[3-Methyl-6-(methyloxy)-1-benzothien-2-yl]heptyl}oxy)-3-(methyloxy)phenyl]acetic acid (isomer 2)

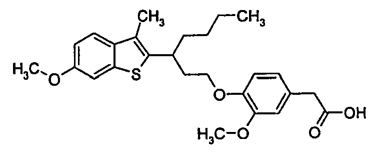

This compound was prepared from ethyl [4-({3-[3-methyl-6-(methyloxy)-1-benzothien-2-yl]heptyl}oxy)-3-(methyloxy)phenyl]acetate (isomer 2) by the same procedure used for Example 58.¹H NMR (400 MHz, DMSO-d₆) δ: 12.23 (1 H, s), 7.51 (1 H, d, J 8.8 Hz), 7.45 (1 H, d, J 2.3 Hz), 6.95 (1 H, dd, J 8.8, J 2.3 Hz), 6.85 (1 H, d, J 1.5 Hz), 6.74 (1 H, J 8.3 Hz), 6.66 (1 H, dd, J 1.5, J 8.3 Hz), 3.90-3.81 (1 H, m), 3.79 (3H, s), 3.74 (3H, s), 3.71-3.64 (1H, m), 3.45 (2H, s), 3.45-3.37 (1 H, m), 2.17 (3H, s), 2.17-2.11 (1 H, m), 1.89-1.70 (2H, m), 1.62-1.50 (1 H, m), 1.37-1.07 (4H, m), 0.81 (3H, m). LC/MS: m/z 457 [M+H]⁺, m/z 455 [M-H]⁻, Rₜ 3.94 min.

### Example 64 : [2-({3-[3-Methyl-6-(trifluoromethyl)-1-benzothien-2-yl]heptyl}thio)-1,3-thiazol-4-yl]acetic acid (isomer 1)

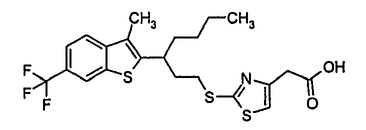

To a solution of ethyl [2-({3-[3-methyl-6-(trifluoromethyl)-1-benzothien-2-yl]heptyl}thio)-1,3-thiazol-4-yl]acetate (isomer 1) (71 mg) in MeOH (3 mL) and THF (3 mL), 2M sodium hydroxide (0.6 mL) was added. The reaction was stirred at room temperature for 18 hours. The solvent was evaporated, water (2 mL) was added followed by a few drop of 2M HCl then the solution was extracted with EtOAc (2 x 3 mL). The organics were combined and washed with brine, separated, dried through a hydrophobic frit and evaporated. The product was isolated after freeze-drying with 1,4-dioxane (2 mL) and water (2 drops) as a yellow oil (66 mg). ¹H NMR (400 MHz, DMSO-d₆) δ: 12.43 (1 H, s), 8.39 (1 H, s), 7.89 (1 H, d, J 8.6 Hz), 7.67 (1 H, dd, J 8.6, J 1.1 Hz), 7.35 (1 H, s), 3.56 (2H, s), 3.44-3.35 (1H, m), 3.15-2.98 (2H, m), 2.35 (3H, s), 2.21-2.09 (1H, m), 2.03 -1.89 (1 H, m), 1.83-1.71 (1 H, m), 1.62-1.49 (1 H, m), 1.36-1.01 (4H, m), 0.80 (3H, m). LC/MS: m/z 488 [M+H]⁺, m/z 486 [M-H]⁻, Rₜ 4.30 min.

### Example 65 : [2-({3-[3-Methyl-6-(trifluoromethyl)-1-benzothien-2-yl]heptyl}thio)-1,3-thiazol-4-yl]acetic acid (isomer 2)

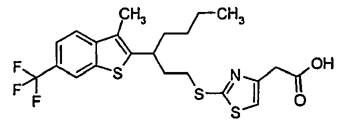

This compound was prepared from ethyl [2-({3-[3-methyl-6-(trifluoromethyl)-1-benzothien-2-yl]heptyl}thio)-1,3-thiazol-4-yl]acetate (isomer 2) by the same procedure used for Example 64.¹H NMR (400 MHz, DMSO-d₆) δ: 12.43 (1 H, s), 8.39 (1 H, s), 7.89 (1 H, d, J 8.6 Hz), 7.67 (1 H, dd, J 8.6, J 1.1 Hz), 7.35 (1 H, s), 3.56 (2H, s), 3.44-3.35 (1 H, m), 3.15-2.98 (2H, m), 2.35 (3H, s), 2.21-2.09 (1H, m), 2.03 -1.89 (1 H, m), 1.83-1.71 (1 H, m), 1.62-1.49 (1 H, m), 1.36-1.01 (4H, m), 0.80 (3H, m). LC/MS: m/z 488 [M+H]⁺, m/z 486 [M-H]⁻, Rₜ 4.30 min.

### Example 66 : 3-[4-({3-[3-Methyl-6-(trifluoromethyl)-1-benzothien-2-yl]heptyl}oxy)phenyl]propanoic acid (isomer 1)

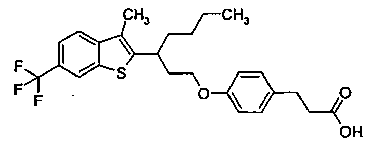

This compound was prepared from ethyl 3-[4-({3-[3-methyl-6-(trifluoromethyl)-1-benzothien-2-yl]heptyl}oxy)phenyl]propanoate (isomer 1) by the same procedure used for Example 64. ¹H NMR (400 MHz, DMSO-d₆) δ: 12.08 (1 H, s), 8.38 (1 H, s), 7.84 (1 H, d, J 8.3 Hz), 7.64 (1 H, dd, J 8.3, J 1.0 Hz), 7.07 (2H, d, J 8.6 Hz), 6.76 (2H, d, J 8.6 Hz), 3.92-3.84 (1 H, m), 3.69-3.60 (1 H, m), 3.54-3.43 (1 H, m), 2.71 (2H, t, J 7.6 Hz), 2.45 (2H, t, J 7.6 Hz), 2.26 (3H, s), 2.26-2.14 (1 H, m), 1.95-1.75 (2H, m), 1.68-1.54 (1 H, m), 1.36-1.05 (4H, m), 0.81 (3H, m). LC/MS: m/z 496 [M+NH₄]⁺, m/z 477 [M-H]⁻, Rₜ 4.44 min.

### Example 67 : 3-[4-({3-[3-Methyl-6-(trifluoromethyl)-1-benzothien-2-yl]heptyl}oxy)phenyl]propanoic acid (isomer 2)

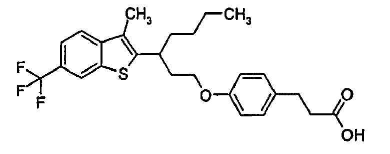

This compound was prepared from ethyl 3-[4-({3-[3-methyl-6-(trifluoromethyl)-1-benzothien-2-yl]heptyl}oxy)phenyl]propanoate (isomer 2) bv the same procedure used for Example 64.¹H NMR (400 MHz, DMSO-d₆) δ: 12.08 (1H, s), 8.38 (1 H, s), 7.84 (1H, d, J 8.3 Hz), 7.64 (1H. dd, J 8.3, J 1.0 Hz), 7.07 (2H, d, J 8.6 Hz), 6.76 (2H, d, J 8.6 Hz), 3.92-3.84 (1 H, m), 3.69-3.60 (1 H, m), 3.54-3.43 (1 H, m), 2.71 (2H, t, J 7.6 Hz), 2.45 (2H, t, J 7.6 Hz), 2.26 (3H, s), 2.26-2.14 (1 H, m), 1.95-1.75 (2H, m), 1.68-1.54 (1 H, m), 1.36-1.05 (4H, m), 0.81 (3H, m). LC/MS: m/z 496 [M+NH4]⁺, m/z 477 [M-H]⁻, Rₜ 4.44 min.

### Example 68 : 3-[3-(Methyloxy)-4-({3-[3-methyl-6-(trifluoromethyl)-1-benzothien-2-yl]heptyl}oxy)phenyl]propanoic acid (isomer 1)

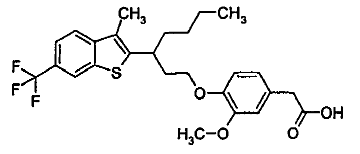

This compound was prepared from ethyl [3-methoxy-4-({3-[3-methyl-6-(trifluoromethyl)-1-benzothien-2-yl]heptyl}oxy)phenyl]acetate (isomer 1) following the same procedure used for Example 64, except a further purification step by auto-prep HPLC was required. ¹H NMR (400 MHz, DMSO-d₆) δ: 12.28 (1 H, s), 8.38 (1 H, s), 7.85 (1 H, d, J 8.3 Hz), 7.64 (1 H, d, J 8.3 Hz), 6.84 (1 H, s), 6.74 (1 H, d, J 8.0 Hz), 6.66 (1 H, d, J 8.0 Hz), 3.93-3.84 (1 H, m), 3.73 (3H, s), 3.73-3.61 (1H, m), 3.57-3.46 (1 H, m), 3.44 (2H, s), 2.28 (3H, s), 2.28-1.15 (1 H, m), 1.96-1.77 (2H, m), 1.68-1.54 (1 H, m), 1.37-1.17 (3H, m), 1.17-1.04 (1 H, m), 0.81 (3H, m). LC/MS: m/z 512 [M+NH₄]⁺, m/z 493 [M+HCO₂]⁻, Rₜ 4.20 min.

### Example 69 : 3-[3-(Methyloxy)-4-({3-[3-methyl-6-(trifluoromethyl)-1-benzothien-2-yl]heptyl}oxy)phenyl]propanoic acid (isomer 2)

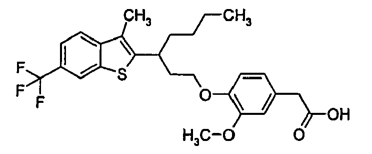

This compound was prepared from ethyl [3-methoxy-4-({3-[3-methyl-6-(trifluoromethyl)-1-benzothien-2-yl]heptyl}oxy)phenyl]acetate (isomer 2) following the same procedure used for Example 64, except a further purification step by NH₂-SPE (1 g; eluent: MeOH, MeOH + 5% formic acid) was required. ¹H NMR (400 MHz, DMSO-d₆) δ: 12.28 (1 H, s), 8.38 (1 H, s), 7.85 (1H, d, J 8.3 Hz), 7.64 (1H, d, J 8.3 Hz), 6.84 (1H, s), 6.74 (1H, d, J 8.0 Hz), 6.66 (1H, d, J 8.0 Hz), 3.93-3.84 (1 H, m), 3.73 (3H, s), 3.73-3.61 (1 H, m), 3.57-3.46 (1 H, m), 3.44 (2H, s), 2.28 (3H, s), 2.28-1.15 (1 H, m), 1.96-1.77 (2H, m), 1.68-1.54 (1 H, m), 1.37-1.17 (3H, m), 1.17-1.04 (1H, m), 0.81 (3H, m). LC/MS: m/z 512 [M+NH₄]⁺, m/z 493 [M+HCO₂]⁻, Rₜ 4.20 min.

### BINDING AND TRANSFECTION ASSAYS

### Binding Assay:

Compounds were tested for their ability to bind to hPPAR gamma hPPARalpha or PPARdelta using a Scintillation Proximity Assay (SPA). The PPAR ligand binding domain (LBD) was expressed in E. coli as polyHis tagged fusion proteins and purified. The LBD was then labelled with biotin and immobilised on streptavidin-modified scintillation proximity beads. The beads were then incubated with a constant amount of the appropriate radioligand (5-{4-[2-(Methyl-pyridin-2-yl-amino)-ethoxy]-benzyl}-thiazolidine-2,4-dione (J.Med.Chem. 1994, 37(23), 3977), for PPARgamma), and labelled GW 2433 (see Brown, P. J et al. Chem. Biol., 4, 909-918 (1997), for the structure and synthesis of this ligand) for PPAR alpha and PPAR delta) and variable concentrations of test compound, and after equilibration the radioactivity bound to the beads was measured by a scintillation counter. The amount of nonspecific binding, as assessed by control wells containing 50 µM of the corresponding unlabeled ligand, was subtracted from each data point. For each compound tested, plots of ligand concentration vs. CPM of radioligand bound were constructed and apparent Kl values were estimated from nonlinear least squares fit of the data assuming simple competitive binding. The details of this assay have been reported elsewhere (see, Blanchard, S. G. et. al. Development of a Scintillation Proximity Assay for Peroxisome Proliferator-Activated Receptor gamma Ligand Binding Domain. Anal. Biochem., 257, 112-119 (1998)).

### Transfection assay:

Compounds were screened for functional potency in transient transfection assays in CV-1 cells for their ability to activate the PPAR subtypes (transactivation assay). A previously established chimeric receptor system was utilized to allow comparison of the relative transcriptional activity of the receptor subtypes on the same target gene and to prevent endogenous receptor activation from complicating the interpretation of results. See, for example, Lehmann, J. M.; Moore, L. B.; Smith-Oliver, T. A.; Wilkison, W. O.; Willson, T. M.; Kliewer, S. A., An antidiabetic thiazolidinedione is a high affinity ligand for peroxisome proliferator-activated receptor gamma (PPARgamma), J. Biol. Chem., 270, 12953-6 (1995). The ligand binding domains for murine and human PPAR alpha, PPAR gamma, and PPAR delta were each fused to the yeast transcription factor GAL4 DNA binding domain. CV-1 cells were transiently transfected with expression vectors for the respective PPAR chimera along with a reporter construct containing five copies of the GAL4 DNA binding site driving expression of secreted placental alkaline phosphatase (SPAP) and beta-galactosidase. After 16 h, the medium was exchanged to DME medium supplemented with 10% delipidated fetal calf serum and the test compound at the appropriate concentration. After an additional 24h, cell extracts were prepared and assayed for alkaline phosphatase and β-galactosidase activity. Alkaline phosphatase activity was corrected for transfection efficiency using the beta-galactosidase activity as an internal standard (see, for example, Kliewer, S. A., et. al. Cell 83, 813-819 (1995)). Rosiglitazone (BRL 49653) was used as a positive control in the hPPAR gamma assay. The positive control in the hPPAR alpha assays was 2-4-[2-(3-[4-fluorophenyl]-1-heptylureido)ethyl]-phenoxy-(2-methyl propionic acid (WO 97/36579). The positive control for PPAR delta assays was 2-{2-methyl-4-[({4-methyl-2-{trifluoromethyl)phenyl]-1,3-thiazol-5-yl}methyl)sulfanyl]phenoxy}acetic acid (WO 01/00603). The positive control was (5-{4-[2-(Methyl-pyridin-2-yl-amino)-ethoxy]-benzyl}-thiazolidine-2,4-dione (J.Med.Chem. 1994, 37(23), 3977), for PPARgamma.

All of the above acid Examples showed at least 50% activation of PPARδ relative to the positive control at concentrations of 10⁻⁷ M or less.

## Claims

1. A compound of formula (i) or a pharmaceutically acceptable salt. solvate, or C₁₋₆ alkyl ester thereof, 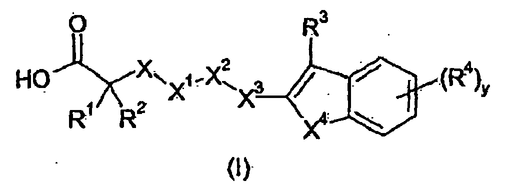 wherein:
R¹ and R² independently represent H or C₁₋₃ alkyl;
X represents O, CH₂, or a bond (i.e. is absent);
X¹ represents: 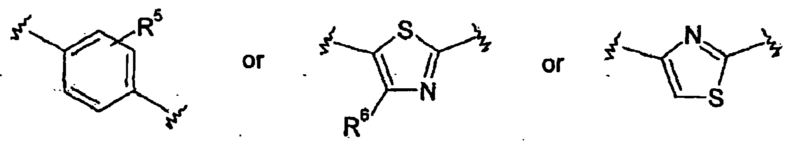
wherein R⁶ represents H, C₁₋₆ alkyl, halogen, -OC₁₋₃ alkyl, CF₃;
R⁶ represents C₁₋₃ alkyl or H;
X² is O or S;
X³ is -(CH₂)ₙ - CH(R⁷)- orCH₂-CH = CH-
wherein n is 0, 1 or 2; R⁷ represents H or C₁₋₆ alkyl, and the alkyl chain being optionally interrupted by one or more O atoms;
X⁴ is S or O;
R**³** represents H or C₁₋₆ alkyl;
R⁴ represents H, CF₃. C₁₋₆ alkyl: halogen, or -OC₁₋₃ alkyl;
y is 0, 1, 2, 3, or 4.

2. A compound of Formula (Ia) or a pharmaceutically acceptable salt, solvate or C₁₋₆ alkyl ester thereof: 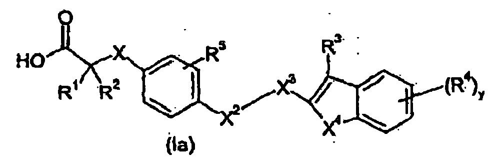 wherein X represents O, CH₂, or a bond (ie is absent), R¹, R², R³, R⁴, R⁵, X² and X³ and y are as defined in claim 1.

3. A compound of Formula (Ib) or a pharmaceutically acceptable salt, solvate or C₁₋₆ alkyl ester thereof: 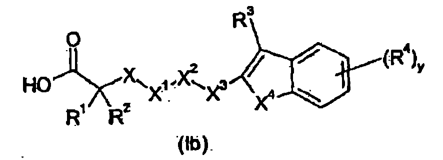 wherein X¹ represents: 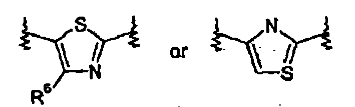 wherein X is a bond (ie) is absent), X², X³, X⁴**,** R¹, R², R³, R⁴ and R⁶ are as defined in claim 1.

4. A compound according to claims 1 - 3 wherein each R¹ and R² is independently H or methyl.

5. A compound according to claim 4 wherein R¹ and R² are both H or both methyl.

6. A compound according to claim 5 wherein R¹ and R² are both H.

7. A compound according to claim 1- 6 wherein X² is O or S.

8. A compound according to claims 1 - 7 wherein X³ is -(CH2)ₙ-CH(R⁷) wherein n is 0, 1, or 2 and R⁷ is C₁₋₄ alkyl, (methyloxy)ethyl propyloxy or H.

9. A compound according to claim 8 wherein n is 1 or 2.

10. A compound according to claim 9 wherein n is 2.

11. A compound according to claims 1 - 10 wherein R³ is CH₃ or H.

12. A compound according to claims 1 -11 wherein y is 0 or 1.

13. A compound according to claim 12 wherein y is 1.

14. A compound according to claims 1-13 wherein R⁴ is -CH₃, CF₃, OCH₃ or halogen.

15. A compound according to claim 1-14 selected from:
[(4-{[3-(5-Bromo-3-methyl-1-benzothien-2-yl)propyl]thio}-2-methylphenyl)oxy]acetic acid
[(4-{[3-(6-Bromo-3-methyl-1-benzothien-2-yl)propyl]thio}-2-methylphenyl)oxy]acetic acid
{[2-Methyl-4-({3-[3-methyl-5-(trifluoromethyl)-1-benzothien-2-yl]propyl}thio)phenyl]oxy}acetic acid
{[2-Ethyl-4-({3-[3-methyl-5-(trifluoromethyl)-1-benzothien-2-yl]propyl}thio)phenyl]oxy}acetic acid
{[4-({3-[3-Ethyl-6-(trifluoromethyl)-1-benzothien-2-yl]propyl}thio)-2-methylphenyl]oxy}acetic add
{[2-Ethyl-4-({3-[3-ethyl-6-(trifluoromethyl)-1-benzothien-2-yl]propyl}thio)phenyl]oxy}acetic acid
{[2-Methyl-4-({3-[6-(trifluoromethyl)-1-benzothien-2-yl]propyl}thio)phenyl]oxy}acetic acid
{[2-Ethyl-4-({3-[6-(trifluoromethyl)-1-benzothien-2-yl]propyl}thio)phenyl]oxy}acetic acid
{[2-(Trifluoromethyl)-4-({3-[6-(trifluoromethyl)-1-benzothien-2-yl]propyl}thio)phenyl]oxy}acetic acid
{[2-Ethyl-4-({3-[3-methyl-6-(trifluoromethyl)-1-benzothien-2-yl]propyl}thio)phenyl]oxy}acetic acid
2-Methyl-2-{[2-methyl-4-({3-[3-methyl-6-(trifluoromethyl)-1-benzothien-2-yl]propyl}thio)phenyl]oxy}propanoic acid
{[4-({3-[3-Methyl-6-(trifluoromethyl)-1-benzothien-2-yl]propyl}thio)-2-(trifluoromethyl)phenyl]oxy}acetic acid
{[2-Methyl-4-({(2E)-3-[3-methyl-5-(triftuoromethyl)-1-benzothien-2-yl]-2-propen-1-yl}thio)phenyl]oxy}actic add
2-[(4-{[3-(6-Bromo-3-methyl-1-benzothien-2-yl)propyl]oxy}-2-methylphenyl)oxy]-2-methylpropanoic acid
2-Methyl-2-{[2-methyl-4-({3-[3-methyl-5-(trifluoromethyl)-1-benzothien-2-yl]propyl}oxy)phenyl]oxy}propanoic acid
2-Methyl-2-{[2-methyl-4-({3-[3-methyl-6-(trifluoromethyl)-1-benzothien-2-yl]propyl}oxy)phenyl]oxy}propanoic acid
{[2-Methyl-4-({3-[6-(trifluoromethyl)-1-benzothien-2-yl]heptyl}thio)phenyl]oxy}acetic acid
{[2-(Trifluoromethyl)-4-({3-[6-(trifluoromethyl)-1-benzothien-2-yl]heptyl}thio)phenyl]oxy}acetic add
{[2-Methyl-4-({3-[5-(trifluoromethyl)-1-benzothien-2-yl]heptyl}thio)phenyl]oxy}acetic acid
{[2-Methyl-4-({3-[5-(trifluoromethyl)-1-benzothien-2-yl]heptyl}oxy)phenyl]oxy}acetic acid
{[2-Ethyl-4-({3-[5-(trifluoromethyl)-1-benzothien-2-yl]heptyl}oxy)phenyl]oxy}acetic acid
{[2-Methyl-4-({3-[3-methyl-6-(trifluomethyl)-1-benzothien-2-yl]hexyl}thio)phenyl]oxy}acetic acid
[4-Methyl-2-({3-[3-methyl-6-(trifluoromethyl)-1-benzothien-2-yl]hexyl}thio)-1,3-thiazol-5-yl]acetic acid
{[2-Methyl-4-({3-[3-methyl-6-(trifluoromethyl)-1-benzothien-2-yl]hexyl}oxy)phenyl]oxy}acetic acid
{[2-Ethyl-4-({3-[3-methyl-6-(trifluoromethyl)-1-benzothien-2-yl]hexyl}oxy)phenyl]oxy}acetic acid
{[2-Methyl-4-({3-[3-methyl-6-(trifluoromethyl)-1-benzothien-2-yl]heptyl}thio)phenyl]oxy}acetic acid
[4-Methyl-2-({3-[3-methyl-6-(trifluoromethyl)-1-benzothien-2-yl]heptyl}thio)-1,3-thiazol-5-yl]acetic acid
{[2-Methyl-4-({3-methyl-6-(trifluoromethyl)-1-benzothien-2-yl]heptyl}oxy)phenyl]oxy}acetic acid
{[2-Ethyl-4-({3-[3-methyl-6-(trifluoromethyl)-1-benzothien-2-yl]heptyl}oxy)phenyl]oxy}acetic acid
[4-({3-[3-Methyl-6-(trifluoromethyl)-1-benzothien-2-yl]heptyl}oxy)phenyl]acetic acid
3-[4-({3-[3-Methyl-6-(trifluoromethyl)-1-benzothien-2-yl]heptyl}oxy)phenyl]propanoic acid
[3-Chloro-4-({3-[3-methyl-6-(trifluoromethyl)-1-benzothien-2-yl]heptyl}oxy)phenyl]acetic acid
[3-(Methyloxy)-4-({3-[3-methyl-6-(trifluoromethyl)-1-benzothien-2-yl]heptyl}oxy)phenyl]acetic acid
3-[3-(Methyloxy)-4-({3-[3-methyl-6-(trifluoromethyl)-1-benzothien-2-yl]heptyl}oxy)phenyl]propanoic acid
[2-({3-[3-Methyl-6-(trifluoromethyl)-1-benzothien-2-yl]heptyl}thio)-1,3-thiazol-4-yl]acetic acid
[(4-{[3-(6-Fluoro-1-benzothien-2-yl)heptyl]oxy}-2-methylphenyl)oxy]acetic acid
[(4-{[3-(6-Fluoro-3-methyl-1-benzothien-2-yl)heptyl]oxy}-2-methylphenyl)oxy]acetic acid
{[4-({3-[3-Ethyl-5-(trifluoromethyl)-1-benzothien-2-yl]heptyl}oxy)-2-methylphenyl]oxy}acetic acid
[(4-{[3-(5-Fluoro-3-methyl-1-benzothien-2-yl)heptyl]oxy}-2-methylphenyl)oxy]acetic acid
[(4-{[3-(6-Fluoro-3-methyl-1-benzothien-2-yl]heptyl]thio}-2-methylphenyl)oxy]acetic acid
{[2-Methyl-4-({3-[3-methyl-5-(trifluoromethyl)-1-benzothien-2-yl]heptyl}thio)phenyl]oxy}acetic acid
(2-{[3-(3,6-Dimethyl-1-benzofuran-2-yl)heptyl]thio}-4-methyl-1,3-thiazol-5-yl)acetic acid
[(4-{[3-(3,6-Dimethyl-1-benzofuran-2-yl)heptyl]oxy}-2-methylphenyl)oxy]acetic acid
[(4-{[3-(3,6-Dimethyl-1-benzofuran-2-yl)heptyl]thio)-2-methylphenyl)oxy]acetic acid
[4-Methyl-2-({3-[3-methyl-6-(trifluoromethyl)-1-benzofuran-2-yl]heptyl}thio)-1,3-thiazol-5-yl]acetic acid
{[2-Methyl-4-({3-[3-methyl-6-(trifluoromethyl)-1-benzofuran-2-yl]heptyl}oxy)phenyl]oxy}acetic acid
{[2-Methyl-4-({3-[3-methyl-6-(trifluoromethyl)-1-benzofuran-2-yl]heptyl}thio)phenyl]oxy}acetic acid
[4-Methyl-2-({5-(methyloxy)-3-[3-methyl-6-(trifluoromethyl)-1-benzothien-2-yl]pentyl}thio)-1,3-thiazol-5-yl]acetic acid
{[2-Methyl-4-({5-(methyloxy)-3-[3-methyl-6-(trifluoromethyl)-1-benzothien-2-yl]pentyl}thio)phenyl]oxy}acetic acid
{[2-Methyl-4-({5-(methyloxy)-3-[3-methyl-6-(trifluoromethyl)-1-benzothien-2-yl]pentyl}oxy)phenyl]oxy}acetic acid
{[2-Ethyl-4-({5-(methyloxy)-3-[3-methyl-6-(trifluoromethyl)-1-benzothien-2-yl]pentyl}oxy)phenyl]oxy}acetic acid
[(2-Methyl-4-{[3-{3-methyl-6-(trifluoromethyl)-1-benzothien-2-yl}-3-(propyloxy)propyl]thio}phenyl)oxy]acetic acid
[(2-Methyl-4-{[3-[3-methyl-6-(trifluoromethyl)-1-benzothien-2-yl]-3-(propyloxy)propyl]oxy}phenyl)oxy]acetic acid
[2-({3-[3-Methyl-6-(methyloxy)-1-benzothien-2-yl]heptyl}thio)-1,3-thiazol-4-yl]acetic acid
{[2-Methyl-4-({3-[3-methyl-6-(methyloxy)-1-benzothien-2-yl]heptyl}oxy)phenyl]oxy}acetic acid
[4-({3-[3-Methyl-6-(methyloxy)-1-benzothien-2-yl]heptyl}oxy)-3-(methyloxy)phenyl]acetic acid

16. A compound according to any of claims 1 -15 for use in therapy.

17. A pharmaceutical composition comprising a compound according to claims 1 -16.

18. Use of a compound according to claims 1 -16 for the manufacture of a medicament for the treatment of a hPPAR disease or condition.

19. Use according to claim 18 wherein the hPPAR mediated disease or condition is dyslipidemia, syndrome X, heart failure, hypercholesteremia, cardiovascular disease, type II diabetes mellitus, type I diabetes, insulin resistance, hyperlipidemia, obesity, anorexia bulimia and anorexia nervosa.

## Patentansprüche

1. Verbindung der Formel (I) oder ein pharmazeutisch annehmbares Salz, Solvat oder C₁₋₆-Alkylester davon, 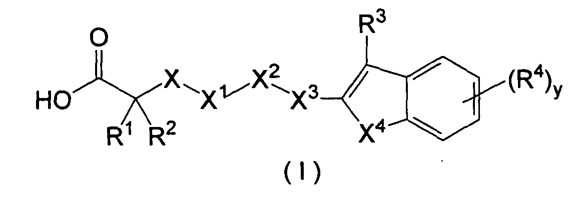 worin:
R¹ und R² unabhängig H oder C₁₋₃-Alkyl darstellen;
X O, CH₂ oder eine Bindung (d.h. fehlt) darstellt;
X¹ folgendes darstellt: 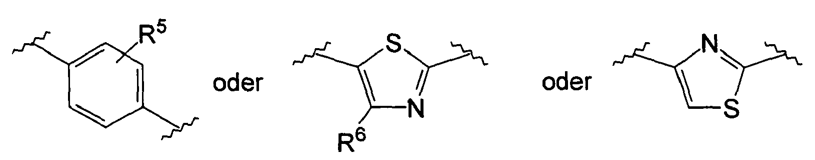
worin R⁵ H, C₁₋₆-Alkyl, Halogen, -OC₁₋₃-Alkyl, CF₃ darstellt;
R⁶ C₁₋₃-Alkyl oder H darstellt;
X² O oder S ist;
X³ -(CH₂)ₙ-CH(R⁷)- oder CH₂-CH=CH- ist,
worin n 0, 1 oder 2 ist; R⁷ H oder C₁₋₆-Alkyl darstellt; und die Alkyl-Kette gegebenenfalls durch ein oder mehre O-Atome unterbrochen ist;
X⁴ S oder O ist;
R³ H oder C₁₋₆-Alkyl darstellt;
R⁴ H, CF₃, C₁₋₆-Alkyl, Halogen oder -OC₁₋₃-Alkyl darstellt;
y 0, 1, 2, 3 oder 4 ist.

2. Verbindung der Formel (Ia) oder ein pharmazeutisch annehmbares Salz, Solvat oder C₁₋₆-Alkylester davon, 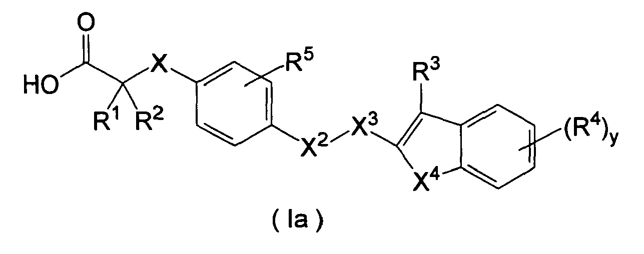 worin X O, CH₂ oder eine Bindung (d.h. fehlt) darstellt, R¹, R², R³, R⁴, R⁵, X² und X³ und y wie in Anspruch 1 definiert sind.

3. Verbindung der Formel (Ib) oder ein pharmazeutisch annehmbares Salz, Solvat oder C₁₋₆-Alkylester davon, 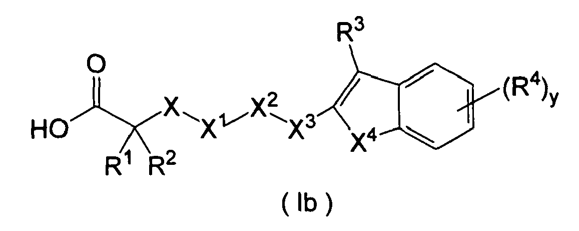 worin X¹ folgendes darstellt: 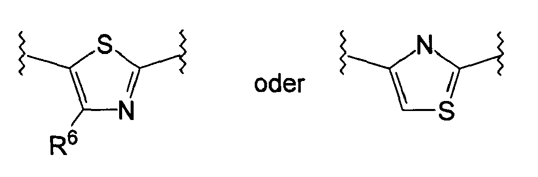 worin X eine Bindung ist (d.h. fehlt), X², X³, X⁴, R¹, R², R³, R⁴ und R⁶ wie in Anspruch 1 definiert sind.

4. Verbindung gemäß den Ansprüchen 1 bis 3, worin jedes R¹ und R² unabhängig H oder Methyl ist.

5. Verbindung gemäß Anspruch 4, worin R¹ und R² beide H oder beide Methyl sind.

6. Verbindung gemäß Anspruch 5, worin R¹ und R² beide H sind.

7. Verbindung gemäß den Ansprüchen 1 bis 6, worin X² 0 oder S ist.

8. Verbindung gemäß den Ansprüchen 1 bis 7, worin X³ -(CH₂)ₙ-CH(R⁷) ist, worin n 0, 1 oder 2 ist und R⁷ C₁₋₄-Alkyl, (Methyloxy)ethyl, Propyloxy oder H ist.

9. Verbindung gemäß Anspruch 8, worin n 1 oder 2 ist.

10. Verbindung gemäß Anspruch 9, worin n 2 ist.

11. Verbindung gemäß den Ansprüchen 1 bis 10, worin R³ CH₃ oder H ist.

12. Verbindung gemäß den Ansprüchen 1 bis 11, worin y 0 oder 1 ist.

13. Verbindung gemäß Anspruch 12, worin y 1 ist.

14. Verbindung gemäß den Ansprüchen 1 bis 13, worin R⁴ CH₃, CF₃, OCH₃ oder Halogen ist.

15. Verbindung gemäß den Ansprüchen 1 bis 14, ausgewählt aus:
[(4-{[3-(5-Brom-3-methyl-1-benzothien-2-yl)propyl]thio}-2-methylphenyl)oxy]essigsäure,
[(4-{[3-(6-Brom-3-methyl-1-benzothien-2-yl)propyl]thio}-2-methylphenyl)oxy]essigsäure,
{[2-Methyl-4-({3-[3-methyl-5-(trifluormethyl)-1-benzothien-2-yl]propyl}thio)phenyl]oxy}essigsäure,
{[2-Ethyl-4-({3-[3-methyl-5-(trifluormethyl)-1-benzothien-2-yl]propyl}thio)phenyl]oxy}essigsäure,
{[4-({3-[3-Ethyl-6-(trifluormethyl)-1-benzothien-2-yl]propyl}thio)-2-methylphenyl]oxy}essigsäure,
{[2-Ethyl-4-({3-[3-ethyl-6-(trifluormethyl)-1-benzothien-2-yl]propyl}thio)phenyl]oxy}essigsäure,
{[2-Methyl-4-({3-[6-(trifluormethyl)-1-benzothien-2-yl]propyl}thio)phenyl]oxy}essigsäure,
{[2-Ethyl-4-({3-[6-(trifluormethyl)-1-benzothien-2-yl]propyl}thio)phenyl]oxy}essigsäure,
{[2-(Trifluormethyl)-4-({3-[6-(trifluormethyl)-1-benzothien-2-yl]propyl}thio)phenyl]oxy}essigsäure,
{[2-Ethyl-4-({3-[3-methyl-6-(trifluormethyl)-1-benzothien-2-yl]propyl}thio)phenyl]oxy}essigsäure,
2-Methyl-2-{[2-methyl-4-({3-[3-methyl-6-(trifluormethyl)-1-benzothien-2-yl]propyl}thio)phenyl]oxy}propansäure,
{[4-({3-[3-Methyl-6-(trifluormethyl)-1-benzothien-2-yl]propyl}thio)-2-(trifluormethyl)-phenyl]oxy}essigsäure,
{[2-Methyl-4-({(2E)-3-[3-methyl-5-(trifluormethyl)-1-benzothien-2-yl]-2-propen-1-yl}thio)phenyl]-oxy}essigsäure,
2-[(4-{[3-(6-Brom-3-methyl-1-benzothien-2-yl)propyl]oxy}-2-methylphenyl)oxy]-2-methylpropansäure,
2-Methyl-2-{[2-methyl-4-({3-[3-methyl-5-(trifluormethyl)-1-benzothien-2-yl]propyl}oxy)phenyl]oxy}propansäure,
2-Methyl-2-{[2-methyl-4-({3-[3-methyl-6-(trifluormethyl)-1-benzothien-2-yl]propyl}oxy)phenyl]oxy}propansäure,
{[2-Methyl-4-({3-[6-(trifluormethyl)-1-benzothien-2-yl]heptyl}thio)phenyl]oxy}essigsäure,
{[2-(Trifluormethyl)-4-({3-[6-(trifluormethyl)-1-benzothien-2-yl]heptyl}thio)phenyl]oxy}essigsäure,
{[2-Methyl-4-({3-[5-(trifluormethyl)-1-benzothien-2-yl]heptyl}thio)phenyl]oxy}essigsäure,
{[2-Methyl-4-({3-[5-(trifluormethyl)-1-benzothien-2-yl]heptyl}oxy)phenyl]oxy}essigsäure,
{[2-Ethyl-4-({3-[5-(trifluormethyl)-1-benzothien-2-yl]heptyl}oxy)phenyl]oxy}essigsäure,
{[2-Methyl-4-({3-[3-methyl-6-(trifluormethyl)-1-benzothien-2-yl]hexyl}thio)phenyl]oxy}lessigsäure,
[4-Methyl-2-({3-[3-methyl-6-(trifluormethyl)-1-benzothien-2-yl]hexyl}thio)-1,3-thiazol-5-yl]essigsäure,
{[2-Methyl-4-({3-[3-methyl-6-(trifluormethyl)-1-benzothien-2-yl]hexyl}oxy)phenyl]oxy}essigsäure,
{[2-Ethyl-4-({3-[3-methyl-6-(trifluormethyl)-1-benzothien-2-yl]hexyl}oxy)phenyl]oxy}essigsäure,
{[2-Methyl-4-({3-[3-methyl-6-(trifluormethyl)-1-benzothien-2-yl]heptyl}thio)phenyl]oxy}essigsäure,
[4-Methyl-2-({3-[3-methyl-6-(trifluormethyl)-1-benzothien-2-yl]heptyl}thio)-1,3-thiazol-5-yl]essigsäure,
{[2-Methyl-4-({3-[3-methyl-6-(trifluormethyl)-1-benzothien-2-yl]heptyl}oxy)phenyl]oxy}essigsäure,
{[2-Ethyl-4-({3-[3-methyl-6-(trifluormethyl)-1-benzothien-2-yl]heptyl}oxy)phenyl]oxy}essigsäure,
[4-({3-[3-Methyl-6-(trifluormethyl)-1-benzothien-2-yl]heptyl}oxy)phenyl]essigsäure,
3-[4-({3-[3-Methyl-6-(trifluormethyl)-1-benzothien-2-yl]heptyl}oxy)phenyl]propansäure,
[3-Chlor-4-({3-[3-methyl-6-(trifluormethyl)-1-benzothien-2-yl]heptyl}oxy)phenyl]essigsäure,
[3-(Methyloxy)-4-({3-[3-methyl-6-(trifluormethyl)-1-benzothien-2-yl]heptyl}oxy)phenyl]essigsäure,
3-[3-(Methyloxy)-4-({3-[3-methyl-6-(trifluormethyl)-1-benzothien-2-yl]heptyl}oxy)phenyl]propansäure,
[2-({3-[3-Methyl-6-(trifluormethyl)-1-benzothien-2-yl]heptyl}thio)-1,3-thiazol-4-yl]essigsäure,
[(4-{[3-(6-Fluor-1-benzothien-2-yl)heptyl]oxy}-2-methylphenyl)oxy]essigsäure,
[(4-{[3-(6-Fluor-3-methyl-1-benzothien-2-yl)heptyl]oxy}-2-methylphenyl)oxy]essigsäure,
{[4-({3-[3-Ethyl-5-(trifluormethyl)-1-benzothien-2-yl]heptyl}oxy)-2-methylphenyl]oxy}essigsäure,
[(4-{[3-(5-Fluor-3-methyl-1-benzothien-2-yl)heptyl]oxy}-2-methylphenyl)oxy]essigsäure,
[(4-{[3-(6-Fluor-3-methyl-1-benzothien-2-yl)heptyl]thio}-2-methylphenyl)oxy]essigsäure,
{[2-Methyl-4-({3-[3-methyl-5-(trifluormethyl)-1-benzothien-2-yl]heptyl}thio)phenyl]oxy}essigsäure,
(2-{[3-(3,6-Dimethyl-1-benzofuran-2-yl)heptyl]thio}-4-methyl-1,3-thiazol-5-yl)essigsäure,
[(4-{[3-(3,6-Dimethyl-1-benzofuran-2-yl)heptyl]oxy}-2-methylphenyl)oxy]essigsäure,
[(4-{[3-(3,6-Dimethyl-1-benzofuran-2-yl)heptyl]thio}-2-methylphenyl)oxy]essigsäure,
[4-Methyl-2-({3-[3-methyl-6-(trifluormethyl)-1-benzofuran-2-yl]heptyl}thio)-1,3-thiazol-5-yl]essigsäure,
{[2-Methyl-4-({3-[3-methyl-6-(trifluormethyl)-1-benzofuran-2-yl]heptyl}oxy)phenyl]oxy}essigsäure,
{[2-Methyl-4-({3-[3-methyl-6-(trifluormethyl)-1-benzofuran-2-yl]heptyl}thio)phenyl]oxy}essigsäure,
[4-Methyl-2-({5-(methyloxy)-3-[3-methyl-6-(trifluormethyl)-1-benzothien-2-yl]pentyl}thio)-1,3-thiazol-5-yl]essigsäure,
{[2-Methyl-4-({5-(methyloxy)-3-[3-methyl-6-(trifluormethyl)-1-benzothien-2-yl]pentyl}thio)phenyl]oxy}essigsäure,
{[2-Methyl-4-({5-(methyloxy)-3-[3-methyl-6-(trifluormethyl)-1-benzothien-2-yl]pentyl}oxy)phenyl]oxy}essigsäure,
{[2-Ethyl-4-({5-(methyloxy)-3-[3-methyl-6-(trifluormethyl)-1-benzothien-2-yl]pentyl}oxy)phenyl]oxy}essigsäure,
[(2-Methyl-4-{[3-[3-methyl-6-(trifluormethyl)-1-benzothien-2-yl]-3-(propyloxy)propyl]thio}-phenyl)oxy]essigsäure,
[(2-Methyl-4-{[3-[3-methyl-6-(trifluormethyl)-1-benzothien-2-yl]-3-(propyloxy)propyl]oxy}-phenyl)oxy]essigsäure,
[2-({3-[3-Methyl-6-(methyloxy)-1-benzothien-2-yl]heptyl}thio)-1,3-thiazol-4-yl]essigsäure,
{[2-Methyl-4-({3-[3-methyl-6-(methyloxy)-1-benzothien-2-yl]heptyl}oxy)phenyl]oxy}essigsäure,
[4-({3-[3-Methyl-6-(methyloxy)-1-benzothien-2-yl]heptyl}oxy)-3-(methyloxy)phenyl]essigsäure.

16. Verbindung gemäß einem der Ansprüche 1 bis 15 zur Verwendung in der Therapie.

17. Pharmazeutische Zusammensetzung, die eine Verbindung gemäß den Ansprüchen 1 bis 16 umfaßt.

18. Verwendung einer Verbindung gemäß den Ansprüchen 1 bis 16 in der Herstellung eines Medikaments zur Behandlung einer hPPAR-Erkrankung oder -Störung.

19. Verwendung gemäß Anspruch 18, worin die hPPAR-vermittelte Erkrankung oder Störung Dyslipidämie, Syndrom X, Herzversagen, Hypercholesterinämie, kardiovaskuläre Krankheit, Typ-II-Diabetes mellitus, Typ-I-Diabetes, Insulinresistenz, Hyperlipidämie, Fettleibigkeit, Anorexia bulimia und Anorexia nervosa ist.

## Revendications

1. Composé de formule (I) ou sel pharmaceutiquement acceptable, solvate ou ester d'alkyle en C₁-C₆ de celui-ci, 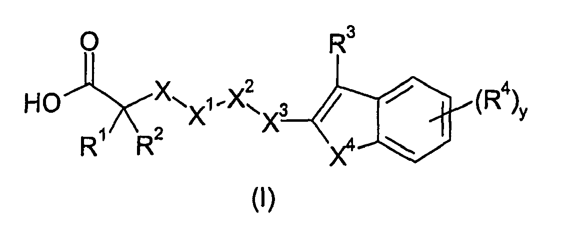 dans lequel
R¹ et R² représentent indépendamment H ou un groupe alkyle en C₁-C₃ ;
X représente 0, CH₂ ou une liaison (c'est-à-dire qu'il est absent) ;
X¹ représente : 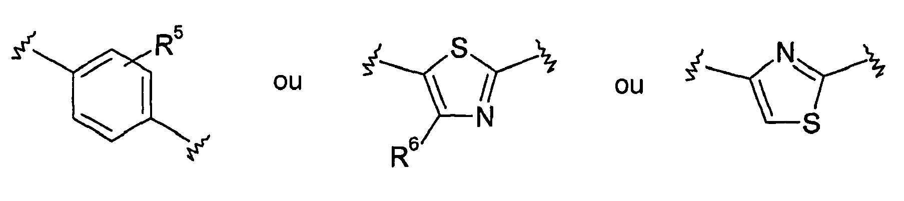
où R⁵ représente H, un groupe alkyle en C₁-C₆, un atome d'halogène, un groupe -O-alkyle en C₁-C₃ ou CF₃ ;
R⁶ représente H ou un groupe alkyle en C₁-C₃ ;
X² représente O ou S ;
X³ représente -(CH₂)ₙ-CH(R⁷)- ou -CH₂-CH=CH-
où n vaut 0, 1 ou 2 ; R⁷ représente H ou un groupe alkyle en C₁-C₆, et la chaîne alkyle est éventuellement interrompue par un ou plusieurs atomes O ;
x⁴ représente S ou O ;
R³ représente H ou un groupe alkyle en C₁-C₆ ;
R⁴ représente H, CF₃, un groupe alkyle en C₁-C₆, un atome d'halogène ou un groupe -O-alkyle en C₁-C₃ ;
y vaut 0, 1, 2, 3 ou 4.

2. Composé de formule (Ia) ou sel pharmaceutiquement acceptable, solvate ou ester d'alkyle en C₁-C₆ de celui-ci, 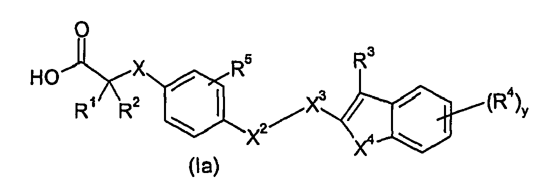 dans lequel
X représente O, CH₂ ou une liaison (c'est-à-dire qu'il est absent) ;
R¹, R², R³, R⁴, R⁵, X², X³ et y sont tels que définis dans la revendication 1.

3. Composé de formule (Ib) ou sel pharmaceutiquement acceptable, solvate ou ester d'alkyle en C₁-C₆ de celui-ci, 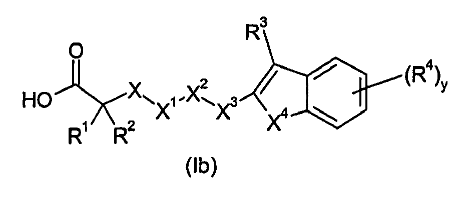 dans lequel X¹ représente : 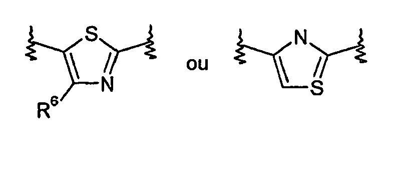
x représente une liaison (c'est-à-dire qu'il est absent), X², X³, X⁴, R¹, R², R³, R⁴ et R⁶ sont tels que définis dans la revendication 1.

4. Composé selon l'une quelconque des revendications 1 à 3, dans lequel chaque R¹ et R² sont indépendamment H ou un groupe méthyle.

5. Composé selon la revendication 4, dans lequel R¹ et R² sont tous les deux H ou tous les deux un groupe méthyle.

6. Composé selon la revendication 5, dans lequel R¹ et R² sont tous les deux H.

7. Composé selon l'une quelconque des revendications 1 à 6, dans lequel X² est O ou S.

8. Composé selon l'une quelconque des revendications 1 à 7, dans lequel X³ représente -(CH₂)ₙ-CH(R⁷) où n vaut 0, 1 ou 2, et R⁷ représente un groupe alkyle en C₁-C₄, un groupe (méthyloxy)éthylpropyloxy ou H.

9. Composé selon la revendication 8, dans lequel n vaut 1 ou 2.

10. Composé selon la revendication 9, dans lequel n vaut 2.

11. Composé selon l'une quelconque des revendications 1 à 10, dans lequel R³ représente CH₃ ou H.

12. Composé selon l'une quelconque des revendications 1 à 11, dans lequel y vaut 0 ou 1.

13. Composé selon la revendication 12, dans lequel y vaut 1.

14. Composé selon l'une quelconque des revendications 1 à 13, dans lequel R⁴ représente CH₃, CF₃, OCH₃ ou un atome d'halogène.

15. Composé selon l'une quelconque des revendications 1 à 14, choisi parmi
l'acide [(4-{[3-(5-bromo-3-méthyl-1-benzothién-2-yl)-propyl]thio}-2-méthylphényl)oxy]acétique,
l'acide [(4-{[3-(6-bromo-3-méthyl-1-benzothién-2-yl)-propyl]thio}-2-méthylphényl)oxy]acétique,
l'acide {[2-méthyl-4-({3-[3-méthyl-5-(trifluorométhyl)-1-benzothién-2-yl]propyl}thio)phényl]oxy}acétique,
l'acide {[2-éthyl-4-({3-[3-méthyl-5-(trifluorométhyl)-1-benzothién-2-yl]propyl}thio)phényl]oxy}acétique,
l'acide {[4-({3-[3-éthyl-6-(trifluorométhyl)-1-benzo-thién-2-yl]propyl}thio)-2-méthylphényl]oxy}acétique,
l'acide {[2-éthyl-4-({3-[3-éthyl-6-(trifluorométhyl)-1-benzothién-2-yl]propyl}thio)phényl]oxy}acétique,
l'acide {[2-méthyl-4-({3-[6-(trifluorométhyl)-1-benzo-thién-2-yl]propyl}thio)phényl]oxy}acétique,
l'acide {[2-éthyl-4-({3-[6-(trifluorométhyl)-1-benzo-thién-2-yl]propyl}thio)phényl]oxy}acétique,
l'acide {[2-(trifluorométhyl)-4-({3-[6-(trifluorométhyl)-1-benzothién-2-yl]propyl}thio)phényl]oxylacétique,
l'acide {[2-éthyl-4-({3-[3-méthyl-6-(trifluorométhyl)-1-benzothién-2-yl]propyl}thio)phényl]oxy}acétique,
l'acide 2-méthyl-2-{[2-méthyl-4-({3-[3-méthyl-6-(trifluorométhyl)-1-benzothién-2-yl]propyl}thio)phényl]oxy}-propanoïque,
l'acide {[4-({3-[3-méthyl-6-(trifluorométhyl)-1-benzo-thién-2-yl]propyl}thio)-2-(trifluorométhyl)phényl]oxy}-acétique,
l'acide {[2-méthyl-4-({(2E)-3-[3-méthyl-5-(trifluoro-méthyl)-1-benzothién-2-yl]-2-propén-1-yl}thio)phényl]oxy}-acétique,
l'acide 2-[(4-{[3-(6-bromo-3-méthyl-1-benzothién-2-yl)-propyl]oxy}-2-méthylphényl)oxy]-2-méthylpropanoïque,
l'acide 2-méthyl-2-{[2-méthyl-4-({3-[3-méthyl-5-trifluorométhyl)-1-benzothién-2-yl]propyl}oxy)phényl]oxy}-propanoïque,
l'acide 2-méthyl-2-{[2-méthyl-4-({3-[3-méthyl-6-(trifluorométhyl)-1-benzothién-2-yl]propyl}oxy)phényl]oxy}-propanoïque,
l'acide {[2-méthyl-4-({3-[6-(trifluorométhyl)-1-benzo-thién-2-yl]heptyl}thio)phényl]oxy}acétique,
l'acide {[2-(trifluorométhyl)-4-({3-[6-(trifluorométhyl)-1-benzothién-2-yl]heptyl}thio)phényl]oxy}acétique,
l'acide {[2-méthyl-4-({3-[5-(trifluorométhyl)-1-benzothién-2-yl]heptyl}thio)phényl]oxy}acétique,
l'acide {[2-méthyl-4-({3-[5-(trifluorométhyl)-1-benzothién-2-yl]heptyl}oxy)phényl]oxy}acétique,
l'acide {[2-éthyl-4-({3-[5-(trifluorométhyl)-1-benzothién-2-yl]heptyl}oxy)phényl]oxy}acétique,
l'acide {[2-méthyl-4-({3-[3-méthyl-6-(trifluorométhyl)-1-benzothién-2-yl]hexyl}thio)phényl]oxy}acétique,
l'acide [4-méthyl-2-({3-[3-méthyl-6-(trifluorométhyl)-1-benzothién-2-yl]hexyl}thio)-1,3-thiazol-5-yl]acétique,
l'acide {[2-méthyl-4-({3-[3-méthyl-6-(trifluorométhyl)-1-benzothién-2-yl]hexyl}oxy)phényl]oxy}acétique,
l'acide {[2-éthyl-4-({3-[3-méthyl-6-(trifluorométhyl)-1-benzothién-2-yl]hexyl}oxy)phényl]oxy}acétique,
l'acide {[2-méthyl-4-({3-[3-méthyl-6-(trifluorométhyl)-1-benzothién-2-yl]heptyl}thio)phényl]oxy}acétique,
l'acide [4-méthyl-2-({3-[3-méthyl-6-(trifluorométhyl)-1-benzothién-2-yl]heptyl}thio)-1,3-thiazol-5-yl]acétique,
l'acide {[2-méthyl-4-({3-[3-méthyl-6-(trifluorométhyl)-1-benzothién-2-yl]heptyl}oxy)phényl]oxy}acétique,
l'acide {[2-éthyl-4-({3-[3-méthyl-6-(trifluorométhyl)-1-benzothién-2-yl]heptyl}oxy)phényl]oxy}acétique,
l'acide [4-({3-[3-méthyl-6-(trifluorométhyl)-1-benzo-thién-2-yl]heptyl}oxy)phényl]acétique,
l'acide 3-{4-({3-[3-méthyl-6-(trifluorométhyl)-1-benzo-thién-2-yl]heptyl}oxy)phényl]propanoïque,
l'acide [3-chloro-4-({3-[3-méthyl-6-(trifluorométhyl)-1-benzothién-2-yl]heptyl}oxy)phényl]acétique,
l'acide [3-(méthyloxy)-4-({3-[3-méthyl-6-(trifluoro-méthyl)-1-benzothién-2-yl]heptyl}oxy)phényl]acétique,
l'acide 3-[3-(méthyloxy)-4-({3-[3-méthyl-6-(trifluoro-méthyl)-1-benzothién-2-yl]heptyl}oxy)phényl]propanoïque,
l'acide [2-({3-[3-méthyl-6-(trifluorométhyl)-1-benzo-thién-2-yl]heptyl}thio)-1,3-thiazol-4-yl]acétique,
l'acide [(4-{[3-(6-fluoro-1-benzothién-2-yl)heptyl]oxy}-2-méthylphényl)oxy]acétique,
l'acide [(4-{[3-(6-fluoro-3-méthyl-1-benzothién-2-yl)-heptyl]oxy}-2-méthylphényl)oxy]acétique,
l'acide {[4-({3-[3-éthyl-5-(trifluorométhyl)-1-benzo-thién-2-yl]heptyl}oxy)-2-méthylphényl]oxy}acétique,
l'acide [(4-{[3-(6-fluoro-3-méthyl-1-benzothién-2-yl)-heptyl]oxy}-2-méthylphényl)oxy]acétique,
l'acide [(4-{[3-(6-fluoro-3-méthyl-1-benzothién-2-yl)-heptyl]thio}-2-méthylphényl)oxy]acétique,
l'acide {[2-méthyl-4-({3-[3-méthyl-5-(trifluorométhyl)-1-benzothién-2-yl]heptyl}thio)phényl]oxy}acétique,
l'acide (2-{[3-(3,6-diméthyl-1-benzofuran-2-yl)heptyl]-thio}-4-méthyl-1,3-thiazol-5-yl)acétique,
l'acide [(4-{[3-(3,6-diméthyl-1-benzofuran-2-yl)heptyl]-oxy}-2-méthylphényl)oxy]acétique,
l'acide [(4-{[3-(3,6-diméthyl-1-benzofuran-2-yl)heptyl]-thio}-2-méthylphényl)oxy]acétique,
l'acide [4-méthyl-2-({3-[3-méthyl-6-(trifluorométhyl)-1-benzofuran-2-yl]heptyl}thio)-1,3-thiazol-5-yl]acétique,
l'acide {[2-méthyl-4-({3-[3-méthyl-6-(trifluorométhyl)-1-benzofuran-2-yl]heptyl}oxy)phényl]oxy}acétique,
l'acide {[2-méthyl-4-({3-[3-méthyl-6-(trifluorométhyl)-1-benzofuran-2-yl]heptyl}thio)phényl]oxy}acétique,
l'acide [4-méthyl-2-({5-(méthyloxy)-3-[3-méthyl-6-(trifluorométhyl)-1-benzothién-2-yl]pentyl}thio)-1,3-thiazol-5-yl]acétique,
l'acide {[2-méthyl-4-({5-(méthyloxy)-3-[3-méthyl-6-(trifluorométhyl)-1-benzothién-2-yl]pentyl}thio)phényl]oxy}-acétique,
l'acide {[2-méthyl-4-({5-(méthyloxy)-3-[3-méthyl-6-(trifluorométhyl)-1-benzothién-2-yl]pentyl}oxy)phényl]oxy}-acétique,
l'acide {[2-éthyl-4-({5-(méthyloxy)-3-[3-méthyl-6-(trifluorométhyl)-1-benzothién-2-yl]pentyl}oxy)phényl]oxy}-acétique,
l'acide [(2-méthyl-4-{[3-[3-méthyl-6-(trifluorométhyl)-1-benzothién-2-yl]-3-(propyloxy)propyl]thio}phényl)oxy]acétique,
l'acide [(2-méthyl-4-{[3-[3-méthyl-6-(trifluorométhyl)-1-benzothién-2-yl]-3-(propyloxy)propyl]oxy}phényl)oxy]acétique,
l'acide [2-({3-[3-méthyl-6-(méthyloxy)-1-benzothién-2-yl]heptyl}thio)-1,3-thiazol-4-yl]acétique,
l'acide {[2-méthyl-4-({3-[3-méthyl-6-(méthyloxy)-1-benzothién-2-yl]heptyl}oxy)phényl]oxy}acétique,
l'acide [4-({3-[3-méthyl-6-(méthyloxy)-1-benzothién-2-yl]heptyl)oxy)-3-(méthyloxy)phényl]acétique.

16. Composé selon l'une quelconque des revendications 1 à 15, pour une utilisation en thérapie.

17. Composition pharmaceutique comprenant un composé selon l'une quelconque des revendications 1 à 16.

18. Utilisation d'un composé selon l'une quelconque des revendications 1 à 16 pour la fabrication d'un médicament destiné au traitement d'une maladie ou d'une pathologie médiée par le hPPAR.

19. Utilisation selon la revendication 18, dans laquelle la maladie ou la pathologie médiée par le hPPAR est la dyslipidémie, le syndrome X, l'insuffisance cardiaque, l'hypercholestérolémie, une maladie cardiovasculaire, le diabète sucré de type II, le diabète de type 1, l'insulino-résistance, l'hyperlipidémie, l'obésité, la boulimie anorexique et la névrose anorexique.
